# EUROPÄISCHE PATENTSCHRIFT

(11) **EP 1 456 212 B1**
(45) Veröffentlichungstag und Bekanntmachung des Hinweises auf die Patenterteilung: **06.12.2006**
(21) Anmeldenummer: 02804904.7
(22) Anmeldetag: 16.12.2002
(51) Int. Cl.: C07D 489/08, A61K 31/485

(54) **6-AMINOMORPHINANDERIVATE, HERSTELLUNGSVERFAHREN DAFÜR UND DEREN VERWENDUNG**
6-AMINOMORPHINANE DERIVATIVES, METHOD FOR THE PRODUCTION AND USE THEREOF
DERIVES DE 6-AMINOMORPHINANE, LEUR PROCEDE DE PRODUCTION ET LEUR UTILISATION

(30) Priorität: 17.12.2001 DE 10161963
(43) Veröffentlichungstag der Anmeldung: 15.09.2004
(73) Patentinhaber: ALCASYNN PHARMACEUTICALS GMBH, 6020 Innsbruck (AT)
(72) Erfinder: SCHÜTZ, Johannes, 6020 Innsbruck (AT); SCHMIDHAMMER, Helmut, 6020 Innsbruck (AT)
(74) Vertreter: Grünecker, Kinkeldey, Stockmair & Schwanhäusser Anwaltssozietät
(86) Internationale Anmeldenummer: PCT/EP2002/014343
(87) Internationale Veröffentlichungsnummer: WO 2003/051888

(56) Entgegenhaltungen:
- EP-A- 0 661 283
- EP-A- 0 846 694
- WO-A-99/38869
- BOTROS S. ET ALL: "Opioid Agonist and Antagonist Activities of Peripherally Selective Derivatives of Naltrexamine and Oxymorphamine" J.MED.CHEM., Bd. 32, Nr. 9, 1989, Seiten 2068-2071, XP002237565
- PORTOGHESE P. S. ET ALL: "A Novel Opioid Receptor Site Directed Alkylating Agent with Irreversible Narcotic Antagonistic and Reversible Agonistic Activities" J.MED.CHEM., Bd. 23, Nr. 3, 1980, Seiten 233-234, XP002237566
- DATABASE CA [Online] CHEMICAL ABSTRACTS SERVICE, COLUMBUS, OHIO, US; IWAI, ISSEI ET AL: "6-Aminodihydrocorsides" retrieved from STN Database accession no. 66:18786 XP002237567 & JP 41 018823 B (SANKYO CO., LTD.) 31. Oktober 1964 (1964-10-31)

## Beschreibung

Die vorliegende Erfindung betrifft eine Klasse von 6-Aminomorphinanverbindungen, die als hochaktive Analgetika verwendet werden können. Die vorliegende Erfindung bezieht sich auch auf deren pharmazeutisch akzeptierbaren Salze und leicht zugänglichen Derivate (z.B. Ester oder Amide der Aminosäurederivate), auf einen Prozess zu deren Herstellung und deren Verwendung in der Herstellung pharmazeutischer Spezialitäten.

Die Existenz von Opioid-Rezeptoren als Rezeptoren des Zentralnervensystems (ZNS), welche analgetische Wirkung vermitteln, ist eindeutig erwiesen. Diese Rezeptoren werden in drei Subtypen, µ, κ und δ eingeteilt. Aktivierung dieser Rezeptoren durch Opioide hat einen analgetischen Effekt zur Folge. Die Aktivierung der µ-Rezeptoren bewirkt den höchsten analgetischen Effekt, wobei besonders Morphinane mit einer Sauerstofffunktion in Position 6 (Morphin, Oxymorphon, Hydromorphon etc.) als effektive Analgetika eingesetzt werden. In der Vergangenheit wurde viel Arbeit in Struktur-Aktivitäts-Beziehungsstudien dieser Substanzklasse investiert.

Im Journal of Medicinal Chemistry 1984, 27, S. 1575-1579 sind verschiedene 14-Methoxymorphinan-6-one mit verschiedenen Substituenten in Position 3 beschrieben. Diese Derivate zeigen höhere analgetische Aktivität als deren 14-Hydroxyanaloga.

Eine detaillierte Studie von 5-Methyloxymorphon (= 14-Hydroxy-5-methyldihydromorphinon) ist in Helvetica Chimica Acta (1988, 71, S. 1801-1804) beschrieben, die zu dem Ergebnis gelangt, daß die Einführung einer 5-Methylgruppe die opioidagonistischen Eigenschaften von Oxymorphon verringert.

Eine weitere Studie an 14-Alkoxymorphinan-6-onen ist in Helvetica Chimica Acta 1989, 72, S. 1233-1239 beschrieben, in welcher der Einfluss verschiedener Substituenten in Position 3 und des Aminstickstoffs evaluiert wurden.

Die EP 0 661 283 A1 offenbart Morphinanderivate, die als Medikament im Bereich Gehirnzellenschutz eingesetzt werden können.

S. Botros et al. offenbaren in J. Med. Chem. 1989, 32, 2068-2071 Opioid-Agonisten und Antagonisten mit einer Morphingrundstruktur.

Die EP 0 846 694A1 offenbart Morphinanderivate und deren pharmazeutische Anwendung.

P.S. Portoghese et al. offenbaren in J. Med. Chem. 1980, 23, 234-237 neue Alkylierungsmittel für Opioidrezeptoren.

JP-41-01882313 offenbart Morphinanderivate.

Die Deutsche Offenlegungsschrift DE 34 12 727 beschreibt 14-Alkoxy-N-methylmorphinan-6-one (14-O-Alkyloxymorphone) mit höherer Aktivität als deren 14-Hydroxy-Analoga.

Kürzlich wurde auch die Existenz von Opioid-Rezeptoren in der Peripherie nachgewiesen (z.B. in Knochen, Gelenken, Knorpeln, Muskeln etc.). Es konnte gezeigt werden, dass über diese peripheren Opioidrezeptoren auch Analgesie vermittelt wird (C. Stein, New Engl. J. Med. 1995, 332, S. 1685-1690). Dazu ist nur eine geringe Dosis eines Opioids (z. B. Morphin), das direkt in das verletzte Gewebe mittels Injektion appliziert wird, notwendig. Diese geringe Dosis hat keine durch das Zentralnervensystem vermittelten Nebenwirkungen zur Folge. Der analgetische Effekt wurde insbesonders bei der Behandlung von Entzündungen und von neuropathischen Schmerzen beobachtet (R. Likar et al., Brit. J. Anaesth. 1999, 83, S. 241-244; V. Kayser et al., Neurosci. 1995, 64, 537-545). Die Art der Applikation (Injektion) stellt einen beträchtlichen Nachteil dieser Behandlung dar. Wiederholte Injektionen in das betroffene Gewebe oder Gelenk bringen Risken wie z.B. Blutungen, Infektionen oder Knorpelschäden mit sich. Analgetisch wirksame Substanzen, welche nur limitierten Zugang zum ZNS haben (auf Grund der Tatsache, dass sie die Blut-Hirn-Schranke nicht oder nur in sehr geringem Ausmaß passieren können) und welche systemisch oder oral verabreicht werden können, sind von hohem Interesse.

Aufgabe der vorliegenden Erfindung war es, neue hochaktive Analgetika herzustellen, die vorzugsweise beschränkten Zugang zum ZNS besitzen und welche bevorzugt in der Peripherie und nicht zentral wirken und die zudem weiter bevorzugt systemisch oder oral verabreicht werden können. Besonders erfolgversprechend wären in diesem Zusammenhang Substanzen, die einen ausschließlich peripheren analgetischen Effekt zeigen, ohne die Nebenwirkungen, die bei zentraler Wirkungsweise auftreten.

Die vorliegende Erfindung löst die oben gestellte Aufgabe durch den Gegenstand der unabhängigen Ansprüche. Bevorzugte Ausführungsformen sind in den Unteransprüchen angegeben.

Die vorliegende Erfindung liefert hochaktive Verbindungen der Formel (I), 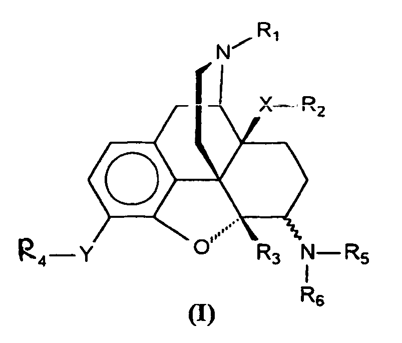 in der die Substituenten die folgende Bedeutung haben:
R₁ : Wasserstoff C₁-C₆-Alkyl; C₂-C₆-Alkenyl; C₂-C₆-Alkinyl; C₁-C₆-Monohydroxyalkyl; C₂-C₆-Dihydroxyalkyl; C₃-C₆-Trihydroxyalkyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
der mit R₁ verbundene Stickstoff kann zusätzlich auch quarternisiert sein durch zwei Substituenten R₁, die gleich oder verschieden sein können und die wie zuvor dargestellt definiert sind und wobei der zweite, quaternisierende Substituent zusätzlich die Bedeutung Hydroxyl, Oxyl (N-Oxid) sowie Alkoxyl haben kann;
R₂ ist Wasserstoff; C₁-C₆-Alkyl; C₂-C₆-Alkenyl, C₂-C₆-Alkinyl; C₁-C₆-Monohydroxyalkyl; C₂-C₆-Dihydroxyalkyl; C₃-C₆-Trihydroxyalkyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆ ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₂-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist.
R₃: Wasserstoff, C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H; CH₂OH.
R₄, vorbehaltlich der folgenden Definition von Y: Wasserstoff; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist, C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₂-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist; Iminomethyl, Formamidinyl, C₁-C₆-N-Alkyl- und N,N'-Dialkylformamidinyl; C₂-C₆-N-Alkenyl- und N,N'-Dialkenylformamidinyl; C₂-C₆-N-Alkinyl- und N,N'-Dialkinylformamidinyl; C₄-C₁₆-N-Cycloalkylalkyl- und N,N'-Dicycloalkylalkylformamidinyl, worin Cycloalkyl C₃-C₁₀-Cycloalkyl ist und Alkyl C₁-C₆-Alkyl ist; C₅-C₁₆-N-Cycloalkylalkenyl- und N,N'-Dicycloalkylalkenylformamidinyl, worin Cycloalkyl C₃-C₁₀-Cycloalkyl ist und Alkenyl C₂-C₆-Alkenyl ist; C₅-C₁₆-N-Cycloalkylalkinyl- und N,N'-Dicycloalkylalkinylformamidinyl, worin Cycloalkyl C₃-C₁₀-Cycloalkyl ist und Alkinyl C₂-C₆-Alkinyl ist; C₇-C₁₆-N-Arylalkyl- und N,N'-Diarylalkylformamidinyl, worin Aryl C₆-C₁₀-Aryl ist und Alkyl C₁-C₆-Alkyl ist.
R₅ und R₆, die gleich oder verschieden sein können: Wasserstoff; weiter können R₅ und R₆, die gleich oder verschieden sein können, CH(A)CO₂B darstellen, worin A Wasserstoff; Hydroxyl; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; Amino; C₁-C₆-Alkylamino; Guanidino; C₁-C₆-Alkyl-CO₂B ist; und worin B Wasserstoff; C₁-C₃₀-, bevorzugt C₁-C₆-Alkyl; C₂-C₃₀-, bevorzugt C₂-C₆-Alkenyl; C₂-C₃₀-, bevorzugt C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl ist; Phenyl; substituiertes Phenyl; CH₂OCO-C₁-C₆-Alkyl; CH(C₁-C₆-Alkyl)OCO-C₁-C₆-Alkyl; CH₂OCOO-C₁-C₆-Alkyl; CH(C₁-C₆-Alkyl)OCOO-C₁-C₆-Alkyl; CH₂CON(C₁-C₆-Alkyl)₂; CH(C₁-C₆-Alkyl)CON(C₁-C₆-Alkyl)₂; Phthalidyl, (5-Methyl-2-oxo-1,3-dioxol-4-yl)methyl; weiter können R₅ und R₆, die gleich oder verschieden sein können, Iminomethyl, Formamidinyl, C₁-C₆-N-Alkyl- und N,N'-Dialkylformamidinyl; C₂-C₆-N-Alkenyl- und N,N'-Dialkenylformamidinyl; C₂-C₆-N-Alkinyl- und N,N'-Dialkinylformamidinyl; C₄-C₁₆-N-Cycloalkylalkyl- und N,N'-Dicycloalkylalkylformamidinyl, worin Cycloalkyl C₃-C₁₀-Cycloalkyl ist und Alkyl C₁-C₆-Alkyl ist; C₅-C₁₆-N-Cycloalkylalkenyl- und N,N'-Dicycloalkylalkenylformamidinyl, worin Cycloalkyl C₃-C₁₀-Cycloalkyl ist und Alkenyl C₂-C₆-Alkenyl ist; C₅-C₁₆-N-Cycloalkylalkinyl- und N,N'-Dicycloalkylalkinylformamidinyl, worin Cycloalkyl C₃-C₁₀-Cycloalkyl ist und Alkinyl C₂-C₆-Alkinyl ist; C₇-C₁₆-N-Arylalkyl- und N,N'-Diarylalkylformamidinyl, worin Aryl C₆-C₁₀-Aryl ist und Alkyl C₁-C₆-Alkyl ist; C₈-C₁₆-N-Arylalkenyl- und N,N'-Diarylalkenylformamidinyl, worin Aryl C₆-C₁₀-Aryl und Alkenyl C₂-C₆-Alkenyl ist; C₈-C₁₆-N-Arylalkinyl- und N,N'-Diarylalkinylformamidinyl, worin Aryl C₆-C₁₀-Aryl ist und Alkinyl C₂-C₆-Alkinyl; C₂-C₇-N-Alkyloxycarbonyl- und N,N'-Bis(alkyloxycarbonyl)formamidinyl; C₃-C₈-N-Alkenyloxycarbonyl- und N,N'-Bis(alkenyloxycarbonyl)formamidinyl; C₃-C₈-N-Alkinyloxycarbonyl- und N,N'-Bis(alkinyloxycarbonyl)formamidinyl; C₈-C₁₇-N-Arylalkyloxycarbonyl- und N,N'-Bis(arylalkyloxycarbonyl)formamidinyl, worin Aryl C₆-C₁₀-Aryl ist und Alkyloxy C₁-C₆-Alkyloxy; C₉-C₁₇-N-Arylalkenyloxycarbonyl- und N,N'-Bis(arylalkenyloxycarbonyl)formamidinyl, worin Aryl C₆-C₁₀-Aryl ist und Alkenyloxy C₂-C₆-Alkenyloxy ist; C₉-C₁₇-N-Arylalkinyloxycarbonyl- und N,N'-Bis(arylalkinyloxycarbonyl)formamidinyl, worin Aryl C₆-C₁₀-Aryl ist und Alkinyloxy C₂-C₆-Alkinyloxy ist; C₂-C₇-N-Alkanoyl- und N,N'-Dialkanoylformamidinyl; C₃-C₈-N-Alkenoyl- und N,N'-Dialkenoylformamidinyl; C₃-C₈-N-Alkinoyl- und N,N'-Dialkinoylformamidinyl; C₈-C₁₆-N-Arylalkanoyl- und N,N'-Diarylalkanoylformamidinyl, worin Aryl C₆-C₁₀-Aryl ist und Alkanoyl C₂-C₆-Alkanoyl; C₉-C₁₆-N-Arylalkenoyl- und N,N'-Diarylalkenoylformamidinyl, worin Aryl C₆-C₁₀-Aryl ist und Alkenoyl C₃-C₆-Alkenoyl ist; C₉-C₁₆-N-Arylalkinoyl- und N,N'-Diarylalkinoylformamidinyl, worin Aryl C₆-C₁₀-Aryl ist und Alkinoyl C₃-C₆-Alkinoyl ist, darstellen; weiter können R₅ und R₆, die gleich oder verschieden sein können, 4,5-Dihydro-1*H*-imidazol-2-yl, 1,4,5,6-Tetrahydropyrimidin-2-yl, 4,5,6,7-Tetrahydro-1H-[1,3]diazepin-2-yl sein.
X ist Sauerstoff,
Y ist Sauerstoff oder die Gruppierung (Y-R₄) ist H.

Die vorliegende Erfindung umfasst auch pharmazeutisch akzeptable Säureadditionssalze und leicht zugängliche Derivate (z.B. Ester oder Amide der Aminosäurederivate) der Verbindungen der Formel (I)

In der vorliegenden Erfindung umfassen die Begriffe Alkyl, Alkenyl und Alkinyl sowohl verzweigte als auch unverzweigte Alkyl-, Alkenyl- und Alkinylgruppen sowie mono-, di- und trihydroxysubstituierte verzweigte und unverzweigte Alkyl-, Alkenyl- und Alkinylgruppen. Aryl kann unsubstituiert oder mono-, di- oder trisubstituiert sein, wobei die Substituenden unabhängig gewählt werden können unter Hydroxy, Halogen, Nitro, Cyano, Thiocyanato, Trifluormethyl, C₁-C₃-Alkyl, C₁-C₃-Alkoxy, CO₂H, CONH₂, CO₂(C₁-C₃-Alkyl), CONH(C₁-C₃-Alkyl), CON(C₁-C₃-Alkyl)₂, CO(C₁-C₃-Alkyl); amino; (C₁-C₃-Monoalkyl)amino, (C₁-C₃-Dialkyl)amino, C₅-C₆-Cycloalkylamino; (C₁-C₃-Alkanoyl)amido, SH, SO₃H, SO₃(C₁-C₃-Alkyl), SO₂(C₁-C₃-Alkyl), SO(C₁-C₃-Alkyl), C₁-C₃-Alkylthio or C₁-C₃-Alkanoylthio. Die oben angeführten Definitionen für Alkyl, Alkenyl, Alkinyl und Aryl sind für alle Substituenten der vorliegenden Anmeldung gültig.

Die Verbindungen der vorliegenden Erfindung beinhalten pharmazeutisch und pharmakologisch akzeptierbare Salze der Verbindungen der Formel (I). Entsprechend der vorliegenden Erfindung sind sowohl anorganische als auch organische Salze geeignet. Beispiele für geeignete anorganische Salze für die vorliegende Erfindung sind Hydrochloride, Hydrobromide, Sulfate, Phosphate und Tetrafluoroborate. Mögliche organische Salze sind zum Beispiel Acetate, Tartrate, Lactate, Benzoate, Stearate, Pamoate, Methansulfonate, Salicylate, Fumarate, Maleinate, Succinate, Aspartate, Citrate, Oxalate, Trifluoracetate und Orotate.

Bevorzugt sind die Säureadditionssalze als konventionelle pharmazeutisch akzeptable Additionssalze, insbesondere bevorzugt die Hydrochloride, die Hydrobromide, die Tetrafluoroborate und die Trifluoracetate. Bevorzugt sind X und Y Sauerstoff. Bevorzugt ist R₁ Alkyl wie vorstehend definiert, insbesondere Methyl oder Ethyl, wobei Methyl bevorzugt ist, oder Cycloalkylalkyl, bevorzugt Cyclopropylmethyl. R₂ ist bevorzugt nicht eine Gruppe, die mit X eine Estereinheit formt. Die anderen Definitionen für R₂ wie sie in Anspruch 1 definiert sind, sind dagegen bevorzugt, wobei insbesondere Alkyl, wie vorstehend definiert bevorzugt ist, insbesondere bevorzugt Methyl, Ethyl und Propyl, gegebenenfalls substituiert, z.B. mit einer Phenylgruppe, beispielsweise um eine 3-Phenylpropylgruppe zu ergeben (d.h., anders gesagt, bevorzugt für R₂ ist auch eine Arylalkylgruppe, insbesondere 3-Phenylpropyl). R₁ und R₂ sind insbesondere bevorzugt beide gleichzeitig Alkyl, insbesondere entweder beide gleichzeitig Methyl oder Methyl (R₁) und Ethyl (R₂). Eine weitere bevorzugte Kombination von R₁ und R₂ ist Cycloalkylalkyl, insbesondere Cyclopropylmethyl für R₁ und Arylalkyl, bevorzugt Phenylpropyl für R₂ R₃ und R₄ sind jeweils bevorzugt Wasserstoff oder Alkyl, wobei als Alkylgruppe Methyl besonders bevorzugt ist. R₄ ist daneben noch bevorzugt C(N-Boc)(NH-Boc). R₅ und R₆ sind bevorzugt so ausgewählt, dass der eine H und der andere von H verschieden ist, wobei dieser von H verschiedene Rest bevorzugt nicht halogeniert ist. R₅ und R₆ werden bevorzugt ausgewählt, unabhängig voneinander, unter Wasserstoff, CH₂COOC(CH₃)₃, CH₂COOH, CH(CH₃)COOC(CH₃), CH(CH₃)COOH, CH(CH₂Ph)COOC(CH₃)₃, CH(CH₂Ph)COOH, C(N-Boc)NH-BOC und C(NH)NH₂, wobei R₆ bevorzugt H ist und R₅ bevorzugt eine der obengenannten Gruppen oder H ist. Auch bevorzugt sind R₅ und R₆ beide H.

In einer speziell bevorzugten Darstellung sind X und Y Sauerstoff. Bevorzugt sind dann R₁ Methyl und Cyclopropylmethyl und R₂ Alkyl und Arylalkyl, insbesondere Methyl und 3-Phenylpropyl, und R₃, R₄ und R₆ Wasserstoff Bevorzugt wird R₅ dann ausgewählt aus tert.-Butoxycarbonylmethyl, Hydroxycarbonylmethyl, 2-(tert.-Butoxycarbonylethyl), 2-(Hydroxycarbonylethyl), 2-(tert.-Butoxycarbonyl-1-phenylethyl), 2-(Hydroxycarbonyl-2-phenylethyl), Wasserstoff, Benzyl (in diesem Fall ist auch R₆ eine Benzylgruppe), N,N'-Bis-(tert.-Butoxycarbonyl)formamidinyl und Formamidinyl.

In einer besonders bevorzugten Darstellung der vorliegenden Erfindung wird die Verbindung der Formel I ausgewählt aus:
(4,5α-Epoxy-3-hydroxy-14β-methoxy-17-methylmorphinan-6α-ylamino)-essigsäure-tert.-butylester
(4,5α-Epoxy-3-hydroxy-14β-methoxy-17-methylmorphinan-6β-ylamino)-essigsäure-tert.-butylester
(4,5α-Epoxy-3-hydroxy-14β-methoxy-17-methylmorphinan-6α-ylamino)-essigsäure
(4,5α-Epoxy-3-hydroxy-14β-methoxy-17-methylmorphinan-6β-ylamino)-essigsäure
(2'S)-2'-(4,5αEpoxy-3-hydroxy-14β-methoxy-17-methylmorphinan-6α-ylamino)-propionsäure-tert.-butylester
(2'S)-2'-(4,5α-Epoxy-3-hydroxy-14β-methoxy-17-methylmorphinan-6β-ylamino)-propionsäure-tert.-butylester
(2'S)-2'-(4,5α-Epoxy-3-hydroxy-14β-methoxy-17-methylmorphinan-6α-ylamino)-propionsäure
(2'S)-2'-(4,5α-Epoxy-3-hydroxy-14β-methoxy-17-methylmorphinan-6β-ylamino)-propionsäure
(2'S)-2'-(4,5α-Epoxy-3-hydroxy-14β-methoxy-17-methylmorphinan-6α-ylamino)-3'-phenylpropionsäure-tert.-butylester
(2'S)-2'-(4,5α-Epoxy-3-hydroxy-14β-methoxy-17-methylmorphinan-6β-ylamino)-3'-phenylpropionsäure-tert.-butylester
(2'S)-2'-(4,5α-Epoxy-3-hydroxy-14β-methoxy-17-methylmorphinan-6α-ylamino)-3'-phenylpropionsäure
(2'S)-2'-(4,5α-Epoxy-3-hydroxy-14β-methoxy-17-methylmorphinan-6β-ylamino)-3'-phenylpropionsäure
6α-Amino-4,5α-epoxy-14β-methoxy-17-methylmorphinan-3-ol
6β-Dibenzylamino-4,5α-epoxy-14β-methoxy-17-methylmorphinan-3-ol
6β-Amino-4,5α-epoxy-14β-methoxy-17-methylmorphinan-3-ol
4,5α-Epoxy-6β-[N,N'-bis-(tert.-butoxycarbonyl)guanidinyl]-14β-methoxy-17-methylmorphinan-3-ol
4,5α-Epoxy-6β-guanidinyl-14β-methoxy-17-methylmorphinan-3-ol
4,5α-Epoxy-6α-[N,N'-bis-(tert.-butoxycarbonyl)guanidinyl]-14β-methoxy-17-methylmorphinan-3-ol
4,5α-Epoxy-6α-guanidinyl-14β-methoxy-17-methylmorphinan-3-ol
1,3-Bis-(tert.-butoxycarbonyl)-2-{4,5α-Epoxy-6β-[N,N'-bis-(tert.-butoxycarbonyl)guanidinyl]-14β-methoxy-17-methylmorphinan-3-yl}-isoharnstoff
1, 3-Bis-(tert.-butoxycarbonyl)-2-{4,5α-Epoxy-6α-[N,N'-bis-(tert.-butoxycarbonyl)guanidinyl]-14β-methoxy-17-methylmorphinan-3-yl}-isoharnstoff
(4,5α-Epoxy-3-hydroxy-14β-methoxy-17-methylmorphinan-6α-ylamino)-essigsäureethylester Diydrochlorid
(4,5α-Epoxy-3-hydroxy-14β-methoxy-17-methylmorphinan-6β-ylamino)-essigsäureethylester Dihydrochlorid
(4,5α-Epoxy-3-hydroxy-14β-ethoxy-17-methylmorphinan-6α-ylamino)-essigsäure-tert.-butylester
(4,5α-Epoxy-3-hydroxy-14β-ethoxy-17-methylmorphinan-6β-ylamino)-essigsäure-tert.-butylester
(4,5α-Epoxy-3-hydroxy-14β-ethoxy-17-methylmorphinan-6α-ylamino)-essigsäure Bis(tetrafluoroborat)
(4,5α-Epoxy-3-hydroxy-14β-ethoxy-17-methylmorphinan-6β-ylamino)-essigsäure Bis(tetrafluoroborat)
(2'S)-2'-(17-Cyclopropylmethyl-4,5α-epoxy-3,14β-dihydroxymorphinan-6β-ylamino)-3-phenylpropionsäure-tert.-butylester
(2'S)-2'-(17-Cyclopropylmethyl-4,5α-epoxy-3,14β-dihydroxymorphinan-6β-ylamino)-3-phenylpropionsäure Bis(tetrafluoroborat)
{17-Cyclopropylmethyl-4,5α-epoxy-3-hydroxy-14β-[(3-phenylpropyl)oxy]-morphinan-6α-ylamino}-essigsäure-tert.-butylester
{17-Cyclopropylmethyl-4,5α-epoxy-3-hydroxy-14β-[(3-phenylpropyl)oxy]-morphinan-6α-ylamino}-essigsäure-tert.-butylester
(2'S)-2'-(17-Cyclopropylmethyl-4,5α-epoxy-3-hydroxy-14β-[(3-phenylpropyl)oxy]-morphinan-6α-ylamino)-3-phenylpropionsäure-tert.-butylester
{17-Cyclopropylmethyl-4,5α-epoxy-3-hydroxy-14β-[(3-phenylpropyl)oxy]-morphinan-6β-ylamino}-essigsäure Dihydrochlorid

Es wurde nun gefunden, dass die Verbindungen der gegenständlichen Erfindung vom 6-Aminomorphinan-Typus wirksame Opioid-Rezeptorliganden repräsentieren und ein hohes therapeutisches Einsatzpotential aufweisen, als Analgetika, als Immunomodulatoren mit immunstimulierender oder immunsuppressiver Wirkung, als Krebstherapeutika, Entzündungshemmer, als Antirheumatika, Diuretika, Anorektika, als Mittel gegen Diarrhoe, als Anästhetika oder als neuroprotektive Wirkstoffe.

Die in den Ansprüchen angeführten Verbindungen sind daher potentiell verwendbar zur Behandlung von Schmerzen, funktionellen Intestinalerkrankungen, wie Abdominalschmerzen, Darmverschluss (Ileus) oder Obstipation, zur Behandlung von Säugetieren, speziell Menschen, zur Behandlung des Raynaud-Syndroms, zur Behandlung von Leiden bedingt durch Gefäßverengung, zur Behandlung von Dismenorrhoe, Angina pectoris, Herzinfarkt, Emphysem, Bronchialspasmen, chronisch obstruktiver Bronchitis, rheumatischer Beschwerden, Nephrosen, Nephritis in Verbindung mit rheumatischen Erkrankungen, zur Behandlung von Tumoren, Phaeochromozytom, der Addison-Krankheit, Leberzirrhose, chronischer Entzündungen des Dünn- und Dickdarms (z.B. Reizdarmsyndrom - Colon irritabile, Colitis ulcerosa, Morbus Crohn), zur Suchtentwöhnung von beispielsweise Opiaten, Kokain oder Alkohol, oder zur Behandlung von psychischen Erkrankungen wie Dysphorie oder Schizophrenie.

Die Verbindungen der vorliegenden Erfindung eignen sich zur Verwendung für die Herstellung eines Arzneimittels für die Behandlung von Schmerzen, umfassend akute und chronische Schmerzen, am Bewegungsapparat wie Nackenschmerzen, Rückenschmerzen, Hüftschmerzen, Knieschmerzen, Schulterschmerzen oder Myofaszialschmerzen, von komplexen regionalen Schmerzsyndromen, von Phantomschmerzen, von Gesichtsschmerzen, von Rheumaschmerzen, von Krebsschmerzen, von Schmerzen nach Verbrennungen, von Schmerzen nach Unfällen, von Schmerzen bei chronischen Entzündungen, von Viszeralschmerzen, von Kopfschmerzen wie z.B. Spannungskopfschmerz, zervikogener Kopfschmerz oder Migräne, Schmerzen nach zentralen Läsionen wie z.B. bei Querschnittslähmungen oder Thalamusläsionen, von Neuralgieschmerzen wie Schmerzen bei der Zosterneuralgie oder der postzosterischen Neuralgie, von Ischämieschmerzen wie bei Angina pectoris oder peripheren arteriellen Verschlusskrankheiten, von postoperativen Schmerzen, von neuropathischen Schmerzen wie Schmerzen bei diabetischer Neuropathie, Schmerzen nach Virusinfektionen oder Schmerzen nach Nervenläsionen.

Die erfindungsgemäßen pharmazeutischen Zusammensetzungen, welche eine Verbindung der vorliegenden Erfindung und/oder ein pharmazeutisch akzeptierbares Salz davon als aktiven Inhaltsstoff zusammen mit einem pharmazeutisch akzeptierbaren Trägerstoff enthalten, eignen sich zur Behandlung der in der Beschreibung erwähnten Indikationen.

Die erfindungsgemäße Verwendung umfasst die Verwendung als analgetischer, immunmodulierender, Antitumor-, antiproliferativer, entzündungshemmender, antirheumatischer, diuretischer, anorektischer, antidiarrhoeischer, anästhetischer, neuroprotektiver Wirkstoff und als Wirkstoff zur Prävention und Behandlung von Darmverschluss (Ileus).

Bevorzugte Verwendungen erfolgen zur Herstellung eines Medikaments zur Behandlung von Schmerzen, funktionellen Intestinalerkrankungen, des Raynaud-Syndroms, zur Behandlung von Leiden bedingt durch Gefäßverengung, Angina pectoris, Herzinfarkt, Emphysem, Bronchialspasmen, chronisch obstruktiver Bronchitis, rheumatischer Beschwerden (einschließlich rheumatoider Arthritis, Arthrose, Osteoarthritis, Spondylosen, Lumbago, Lupus erythematodes, Spondylarthropathien), Nephrosen, Nephritis in Verbindung mit rheumatischen Erkrankungen, zur Behandlung von Tumoren, Krebs, Phaeochromozytom, der Addison-Krankheit, Leberzirrhose, chronischer Entzündungen des Dünn- und Dickdarms (z.B. Reizdarmsyndrom - Colon irritabile, Colitis ulcerosa, Morbus Crohn), zur Behandlung von Drogenmissbrauch, psychischen Erkrankungen oder erektiler Dysfunktion und/oder zur Unterdrückung der Abstossung von Transplantaten nach Transplantationen bei Säugetieren, speziell bei Menschen.

Überraschend wurde auch gefunden, dass die Verbindungen der gegenständlichen Erfindung die Blut-Hirn-Schranke nicht oder nur in geringem Ausmaß zu überwinden vermögen, und ihnen daher eine spezielle Bedeutung hinsichtlich ihrer Verwendung als peripher wirksame Therapeutika zukommt, beispielsweise als Medikamente zur Schmerzbehandlung, zur Rheumatherapie, zur Unterdrückung von Organabstoßung nach Transplantationen bei Säugetieren, speziell Menschen, als auch zur Behandlung von Erektionsstörungen. Der limitierte Zugang zum Zentralnervensystem geht einher mit einer sehr verringerten Nebenwirkungsrate, was zentrale Nebenwirkungen wie z.B. Übelkeit, Erbrechen, Sedation, Benommenheit, Verwirrtheit, Atemdepression und Sucht betrifft.

Außerdem wurde überraschenderweise gefunden, dass die Verbindungen der gegenständlichen Erfindung eine sehr lange analgetische Wirkungsdauer besitzen. Das ermöglicht eine geringere Dosierung und eine weniger häufige Verabreichung des Medikaments, was eine geringere Nebenwirkungsrate und Toxizität sowie eine höhere Bereitschaft der Patienten zur Einnahme des Medikaments zur Folge hat.

### Herstellung der Verbindungen

Die Verbindungen gemäß der vorliegenden Erfindung, welche durch die Formel (I) repräsentiert werden, können mithilfe der folgenden Methoden gewonnen werden:
Ausgehend von Thebain der Formel (II), 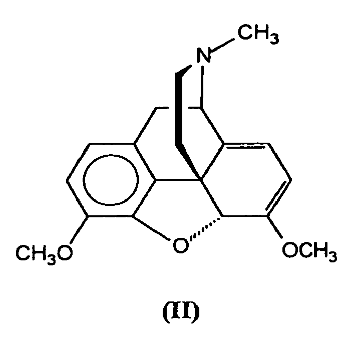 wird diese Verbindung mit Dialkylsulfaten, Fluorsulfonsäurealkylestern, Alkylsulfonsäurealkylestern, Arylsulfonsäurealkylestern, Alkylhalogeniden, Aralkylhalogeniden, Alkylsulfonsäurearalkylestern, Arylsulfonsäurearalkylestern, Arylalkenylhalogeniden, Chlorameisensäureestern oder Ähnlichem umgesetzt in Lösungsmitteln wie Tetrahydrofuran, 1,2-Dimethoxyethan, Diethylether oder Ähnlichem in Gegenwart einer starken Base wie n-Butyllithium, Lithiumdiethylamid, Lithiumdiisopropylamid oder Ähnlichem bei niedrigen Temperaturen (-20° C bis -80° C) (s. Boden et al., J. Org. Chem., Vol. 47, S. 1347-1349, 1982; Schmidhammer et al., Helv. Chim. Acta, Vol. 71, S. 642-647, 1988; Gates et al., J. Org. Chem., Vol. 54, S. 972-975, 1984) um die Verbindungen der Formel (III) erhalten, worin R₃ C₁-C₆-Alkyl ist; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H.

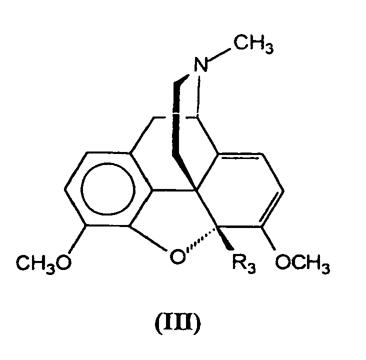

Die Verbindungen der Formel (III) oder Thebain (Formel (II)) können in die entsprechenden 14-Hydroxycodeinone der Formel (IV) umgewandelt werden, 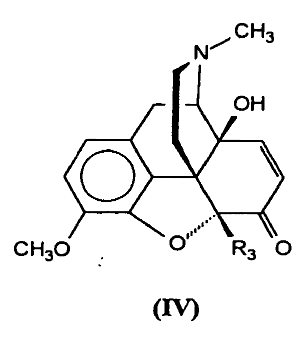 worin R₃ Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂ (C₁-C₆-Alkyl) darstellt. Diese Reaktion wird mit Perameisensäure (s. H. Schmidhammer et al., Helv. Chim. Acta, Vol. 71, 1801-1804, 1988), m-Chlorperbenzoesäure oder Ähnlichem durchgeführt, bei Temperaturen zwischen 0°C und 60°C. Die bevorzugte Methode ist die Reaktion mit Perameisensäure bei 0°C bis 40°C.

Diese 14-Hydroxycodeinone der Formel (IV) werden in der Folge mit Dialkylsulfaten, Alkylhalogeniden, Alkenylhalogeniden, Alkinylhalogeniden, Arylalkylhalogeniden, Arylalkenylhalogeniden, Arylalkinylhalogeniden oder Chloroformiaten umgesetzt in Lösungsmitteln wie N,N-Dimethylformamid (DMF) oder Tetrahydrofuran (THF) in Gegenwart einer starken Base wie Natriumhydrid, Kaliumhydrid oder Natriumamid um die Verbindungen der Formel (V) zu erhalten, 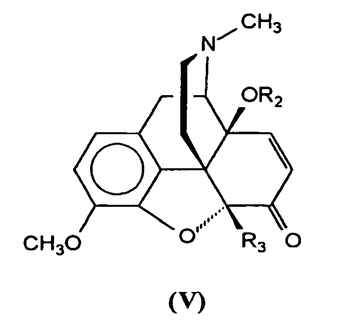 worin R₃ wie oben definiert ist; und R₂ C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₁-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist, darstellt.

Diese Verbindungen werden anschließend mittels katalytischer Hydrierung über einem Katalysator wie Pd/C, PdO, Pd/Al₂O₃, Pt/C, PtO₂, Pt/Al₂O₃, oder Ähnlichem in Lösungsmitteln wie Alkoholen, Alkohol/Wasser-Gemischen, Eisessig oder Ähnlichem reduziert zu Verbindungen der Formel (VI), 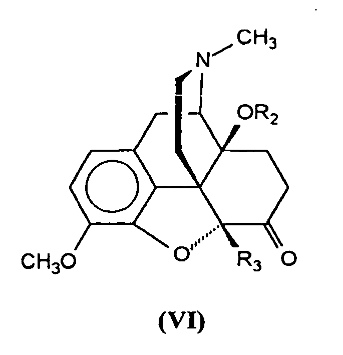 worin R₂ und R₃ wie oben definiert sind.

Die anschließende N-Demethylierung wird mit Chloroformiaten oder Bromcyan in Lösungsmitteln wie 1,2-Dichlormethan, Chloroform oder Ähnlichen durchgeführt, und man erhält Verbindungen der Formel (VII), 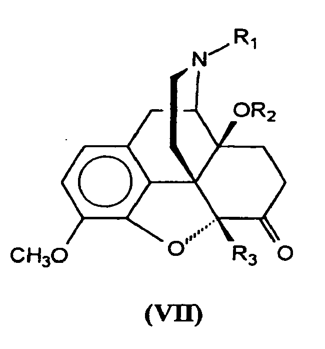 worin R₁ CO₂CH(Cl)CH₃, CO₂CH=CH₂, CO₂CH₂CCl₃, CO₂CH₂CH₃, CO₂Ph, CN oder Ähnliches darstellt; und R₂ und R₃ wie oben definiert sind.

Die Carbamate der Formel (VII) werden entweder durch Rückflußerhitzen in Alkoholen (im Falle von 1-Chlorethylcarbamaten) oder durch Zugabe von Hydrogenhalogeniden bzw. Halogenen und anschließendes Rückflußerhitzen in Alkoholen (im Falle von Vinylcarbamaten) gespalten, die Cyanamide der Formel (VII) durch saure oder alkalische Hydrolyse, wodurch N-Nor-Verbindungen der Formel (VIII) erhalten werden, 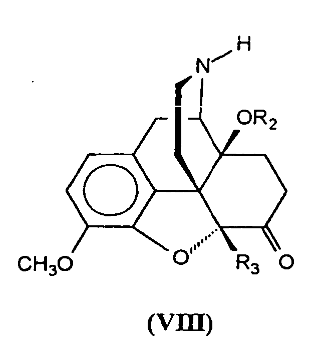 in denen R₂ und R₃ wie oben definiert sind.

Die N-Alkylierung der Verbindungen der Formel (VIII) erreicht man mit Alkylhalogeniden, Dialkylsulfaten, Alkenylhalogeniden, Alkinylhalogeniden, Cycloalkylalkylhalogeniden, Cycloalkenylalkylhalogeniden, Arylalkylhalogeniden, Arylalkenylhalogeniden, Arylalkinylhalogeniden oder Ähnlichem in Lösungsmitteln wie Dichlormethan, Chloroform oder N,N-Dimethylformamid in Gegenwart einer Base wie Natriumhydrogencarbonat, Kaliumcarbonat, Triethylamin oder Ähnlichem und erhält somit Verbindungen der Formel (IX), 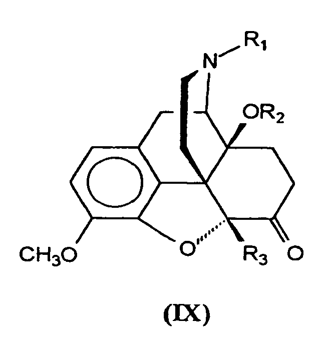 worin R₂ und R₃ wie oben definiert sind; und R₁ Wasserstoff; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl darstellt.

Etherspaltung dieser Verbindungen der Formel (IX) mit Bortribromid (in einem Lösungsmittel wie Dichlormethan oder Chloroform) bei 0°C, 48% Bromwasserstoffsäure (Rückflusserhitzen), mit Natriumalkanthiolaten (in einem Lösungsmittel wie N,N-Dimethylformamid) oder mit anderen allgemein bekannten Etherspaltungsreagenzien, gibt phenolische Verbindungen der Formel (X), 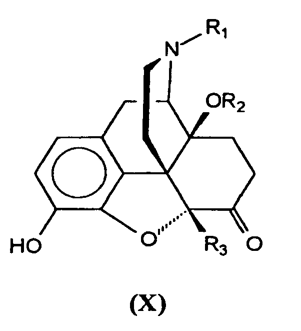 in denen R₁, R₂ und R₃ wie oben definiert sind.

Die 3-O-Alkylierung der Verbindungen der Formel (X) erreicht man mit Alkylhalogeniden, Dialkylsulfaten, Alkenylhalogeniden, Alkinylhalogeniden, Cycloalkylalkylhalogeniden, Cycloalkylalkenylhalogeniden, Arylalkylhalogeniden, Arylalkenylhalogeniden, Arylalkinylhalogeniden oder Ähnlichem in Lösungsmitteln wie Dichlormethan, Chloroform, Aceton oder N,N-Dimethylformamid in Gegenwart einer Base wie Natriumhydrogencarbonat, Kaliumcarbonat, Triethylamin oder Ähnlichem; 3-O-Acylierung der Verbindungen der Formel (X) erreicht man mit Carbonsäurehalogeniden, Carbonsäureanhydriden oder Ähnlichem in Lösungsmitteln wie Dichlormethan, Chloroform, Aceton, N,N-Dimethylformamid, Pyridin oder Ähnlichem und erhält somit Verbindungen der Formel (XI); 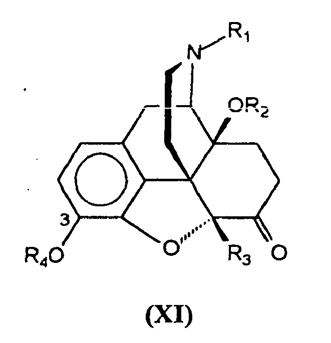 worin R₁, R₂ und R₃ wie oben definiert sind; und R₄ C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Akinyl darstellt; C₁-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist.

Die Verbindungen der Formel (XI) werden mit Ammoniumacetat, primären und sekundären Aminen, Hydroxylamin Hydrochlorid, Aminosäuren, Aminosäureestem oder Ähnlichem in Lösungsmitteln wie Alkoholen, N,N-Dimethylformamid oder Toluol umgesetzt und man erhält Imine der Formel (XII) und Iminiumsalze der Formel (XIII), 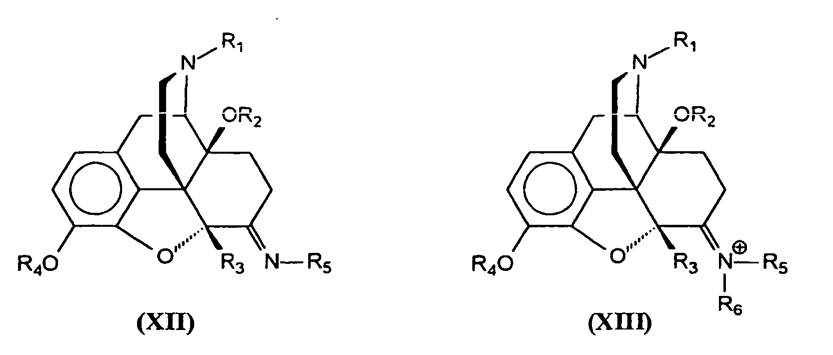 in denen R₁, R₂, R₃ und R₄ wie oben definiert sind; und R₅ und R₆, die gleich oder verschieden sein können, Wasserstoff; CH(A)CO₂B, worin A Wasserstoff; Hydroxyl; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; Amino; C1-C6-Alkylamino; Guanidino; C₁-C₆-Alkylguanidino; C₁-C₆-Alkyl-CO₂B darstellt; und B Wasserstoff; C₁-C₆-Alkyl, C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl ist, darstellen.

Die Reduktion der Imine und Iminiumsalze erfolgt mit komplexen Metallhydriden wie Lithiumaluminiumhydrid, Lithiumborhydrid, Natriumborhydrid und Natriumcyanoborhydrid oder Ähnlichem in Alkoholen, mit Borantetrahydrofuran oder Ähnlichem in Tetrahydrofuran (THF), mit Cyclohexen oder Cyclohexadien oder Ähnlichem in Gegenwart eines Hydrierungskatalysators wie Pd/C, mit Wasserstoff in Gegenwart eines Hydrierungskatalysators wie Pd/C, PdO, Pd/Al₂O₃, Pt/C, Pt/C (sulfidiert), PtO₂, Pt/Al₂O₃, Rh/C, Rh/Al₂O₃ oder Ähnlichem in Lösungsmitteln wie Alkoholen, Eisessig oder Ähnlichem und man erhält die entsprechenden Amine der Formel (XIV), 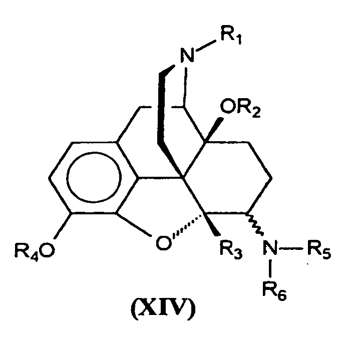 in denen R₁, R₂, R₃ und R₄ wie oben definiert sind; und R₅ und R₆, die gleich oder verschieden sein können, Wasserstoff; CH(A)CO₂B, worin A Wasserstoff; Hydroxyl; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; Amino; C1-C6-Alkylamino; Guanidino; C₁-C₆-Alkylguanidino; C₁-C₆-Alkyl-CO₂B darstellt; und B Wasserstoff, C₁-C₆-Alkyl; C₂-C₆-Alkenyl; C₂-C₆-Alkinyl, C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl ist, darstellen. Diese Verbindungen entsprechen den erfindungsgemäßen Verbindungen der Formel (I), 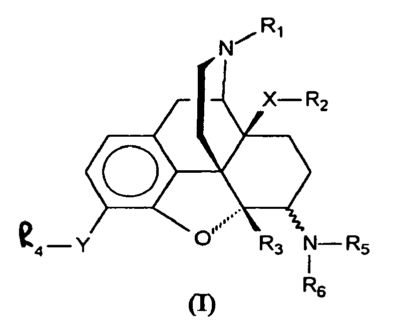 worin R₁, R₂, R₃, R₄, R₅ und R₆ wie oben definiert sind und X und Y Sauerstoff sind.

Wenn R₅ und R₆ in den erfindungsgemäßen Verbindungen der Formel (I) Wasserstoff sind und X und Y Sauerstoff, kann man diese 6-Aminoverbindungen der Formel (XV), 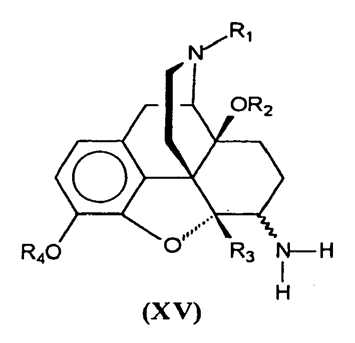 in denen R₁, R₂, R₃ und R₄ wie oben definiert sind, mit Guanidinylierungsmitteln wie N,N'-Bis-(tert.-butoxycarbonyl)-S-methylisothioharnstoff in Gegenwart von Salzen wie Quecksilber(II)chlorid, Silbernitrat oder Ähnlichen sowie von Basen wie Triethylamin, N-Ethyldiisopropylamin oder Ähnlichen in Lösungsmitteln wie N,N-Dimethylformamid oder Ähnlichen umsetzen und man erhält je nach der eingesetzten Menge an Guanidinylierungsmittel entweder Verbindungen der Formel (XVI) oder Verbindungen der Formel (XVIa), 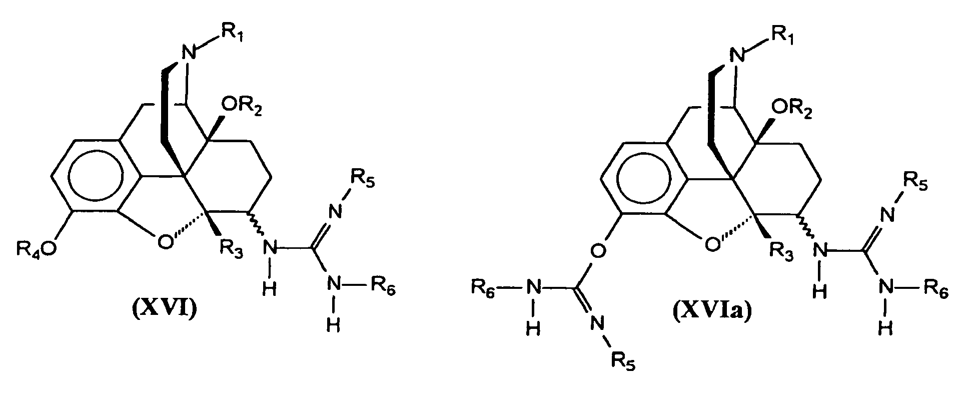 in denen R₁, R₂, R₃ und R₄ wie oben definiert sind; und R₅ und R₆, die gleich oder verschieden sein können, Wasserstoff; eine Schutzgruppe wie z.B. tert.-Butoxycarbonyl (Boc) oder Benzyloxycarbonyl (Z); C₂-C₇-Alkyloxycarbonyl; C₃-C₈-Alkenyloxycarbonyl; C₃-C₈-Alkinyloxycarbonyl; C₈-C₁₇-Arylalkyloxycarbonyl, worin Aryl C₆-C₁₀-Aryl ist und Alkyloxy C₁-C₆-Alkyloxy; C₉-C₁₇-Arylalkenyloxycarbonyl, worin Aryl C₆-C₁₀-Aryl ist und Alkenyloxy C₂-C₆-Alkenyloxy ist; C₉-C₁₇-Arylalkinyloxycarbonyl, worin Aryl C₆-C₁₀-Aryl ist und Alkinyloxy C₂-C₆-Alkinyloxy ist; C₂-C₇-Alkanoyl; C₈-C₁₇-Aralkanoyl, worin Aryl C₆-C₁₀-Aryl ist und Alkyl C₂-C₇-Alkyl ist; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl darstellen.

Die anschließende Abspaltung der Schutzgruppen (R₅, R₆) mit Säuren wie Halogenwasserstoffsäuren, Trifluoressigsäure, Tetrafluoroborsäure oder Ähnlichen in Lösungsmitteln wie Dichlormethan, Diethylether, Alkoholen, Alkohol/Wasser-Gemischen oder Ähnlichen ergibt die 6-Guanidinylverbindungen der Formel (XVII), 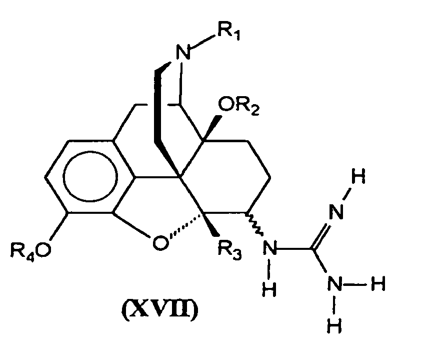 in denen R₁, R₂, R₃ und R₄ wie oben definiert sind. Diese Verbindungen entsprechen den erfindungsgemäßen Verbindungen der Formel (I), in denen R₁, R₂, R₃ und R₄ wie oben definiert sind und R₅ Formamidinyl, R₆ Wasserstoff, X und Y Sauerstoff sind.

Wenn R₅ und R₆ in den erfindungsgemäßen Verbindungen der Formel (I) Wasserstoff sind und X und Y Sauerstoff, kann man diese 6-Aminoverbindungen der Formel (XV), 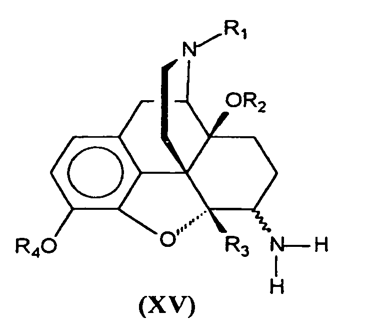 in denen R₁, R₂, R₃ und R₄ wie oben definiert sind, z.B. mit N-Acyl-2-(methylmercapto)-2-imidazolin (das aus kommerziell erhältlichem 2-(Methylmercapto)-2-imidazolin Hydroiiodid leicht darstellbar ist; s. Mundla et al., Tetrahedron Lett., Vol. 41, S. 6563, 2000) oder Ähnlichem in Lösungsmitteln wie Essigsäure, Essigsäure / Ethanol 1:10; Essigsäure /Isopropanol 1 : 10 oder Ähnlichen umsetzen und man erhält die Verbindungen der Formel (XVIII), 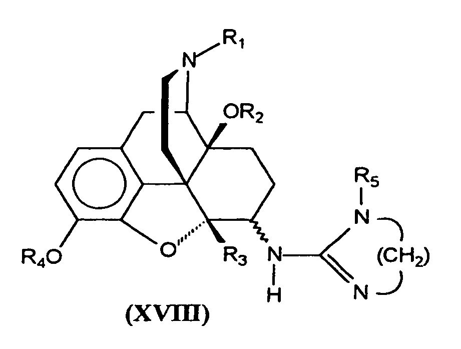 in denen R₁, R₂, R₃ und R₄ wie oben definiert sind; und R₅ ist Wasserstoff; eine Schutzgruppe wie z.B. tert.-Butoxycarbonyl (Boc) oder Benzyloxycarbonyl (Z); C₂-C₇-Alkyloxycarbonyl; C₃-C₈-Alkenyloxycarbonyl; C₃-C₈-Alkinyloxycarbonyl; C₈-C₁₇-Arylalkyloxycarbonyl, worin Aryl C₆-C₁₀-Aryl ist und Alkyloxy C₁-C₆-Alkyloxy-, C₉-C₁₇-Arylalkenyloxycarbonyl, worin Aryl C₆-C₁₀-Aryl ist und Alkenyloxy C₂-C₆-Alkenyloxy ist; C₉-C₁₇-Arylalkinyloxycarbonyl, worin Aryl C₆-C₁₀-Aryl ist und Alkinyloxy C₂-C₆-Alkinyloxy ist; C₂-C₇-Alkanoyl; C₈-C₁₇-Aralkanoyl, worin Aryl C₆-C₁₀-Aryl ist und Alkyl C₂-C₇-Alkyl ist; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl darstellen; n ist eine Zahl zwischen 2 und 4.

Die anschließende Abspaltung der Schutzgruppe (R₅) erfolgt durch Rückflußerhitzen der Verbindungen der Formel (XVIII) in Lösungsmitteln wie Essigsäure / Ethanol 1:10, Essigsäure / Isopropanol 1 : 10, Methanol / Wasser 3 : 1 oder Ähnlichen und man erhält Verbindungen der Formel (XIX), 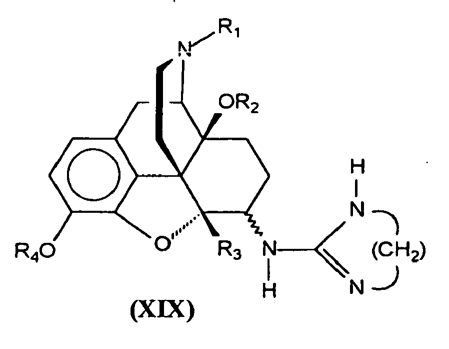 in denen R₁, R₂, R₃ und R₄ wie oben definiert sind; n ist eine Zahl zwischen 2 und 4. Diese Verbindungen entsprechen den erfindungsgemäßen Verbindungen der Formel (I), in denen R₁, R₂, R₃ und R₄ wie oben definiert sind, R₅ entweder 4,5-Dihydro-1*H*-imidazol-2-yl (n = 2), 1,4,5,6-Tetrahydropyrimidin-2-yl (n = 3) oder 4,5,6,7-Tetrahydro-1H-[1,3]diazepin-2-yl (n = 4) ist, R₆ Wasserstoff ist, und X und Y Sauerstoff sind.

Eine alternative Route beginnt mit Verbindungen der Formel (XX), in denen R₁ und R₃ wie oben (s. z.B. Formel IX) definiert sind (s. Weiss et al., J. Amer. Chem. Soc., Vol. 77, S. 5891, 1955; Iijima et al., J. Med. Chem., Vol. 21, S. 398-400, 1978; Coop et al., J. Org. Chem., Vol. 63, S. 4392-4396, 1998; Schmidhammer et al., Helv. Chim. Acta, Vol. 71, S. 1801-1804, 1988; Schmidhammer et al., Helv. Chim. Acta, Vol. 73, S. 1986-1990, 1990).

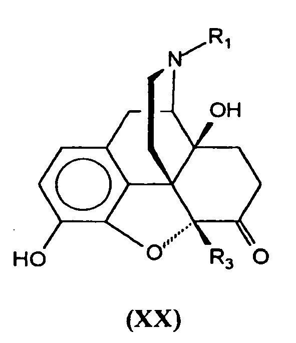

Die Ketone der Formel (XX) werden in Gegenwart einer Säure wie Methansulfonsäure oder Ähnlichem mit Ethylenglykol (als Reagenz und Lösungsmittel) zu Verbindungen der Formel (XXI) umgesetzt, 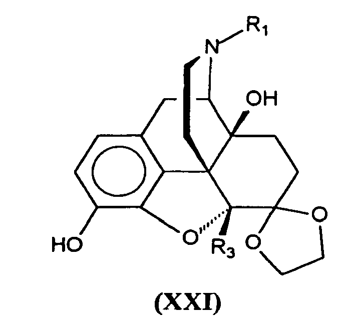 in denen R₁ und R₃ wie oben definiert sind.

Die Einführung einer 3-O-Schutzgruppein Verbindungen der Formel (XXI) erreicht man z.B. mit Benzylhalogeniden oder Trialkylhalogensilanen in Lösungsmitteln wie Dichlormethan, Chloroform, Aceton oder N,N-Dimethylformamid oder Ähnlichem in Gegenwart einer Base wie Natriumhydrogencarbonat, Kaliumcarbonat, Triethylamin oder Ähnlichem und erhält somit Verbindungen der Formel (XXII), 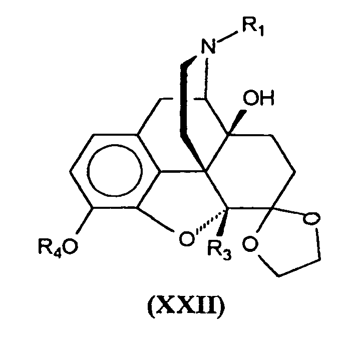 in denen R₁ und R₃ wie oben definiert sind; R₄ ist eine Schutzgruppe wie Benzyl, Tri-(C₁-C₆-Alkyl)silyl oder Tris-(C₇-C₁₆-Arylalkyl)silyl, worin Aryl C₆-C₁₀-Aryl ist und Alkyl C₁-C₆-Alkyl ist.

Diese 14-Hydroxyverbindungen werden in der Folge mit Dialkylsulfaten, Alkylhalogeniden, Alkenylhalogeniden, Alkinylhalogeniden, Arylalkylhalogeniden, Arylalkenylhalogeniden, Arylalkinylhalogeniden oder Chloroformiaten umgesetzt in Lösungsmitteln wie N,N-Dimethylformamid (DMF) oder Tetrahydrofuran (THF) in Gegenwart einer starken Base wie Natriumhydrid, Kaliumhydrid oder Natriumamid um die Verbindungen der Formel (XXIII) zu erhalten, 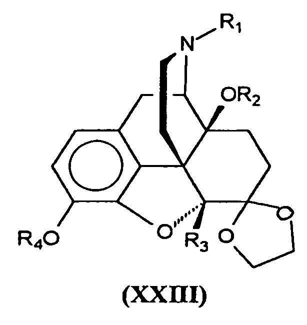 worin R₁, R₂ und R₃ wie oben (s. z.B. Formel (IX)) definiert sind, R₄ ist wie in Formel (XXII) definiert. Wenn R₂ und R₄ Benzyl sind, können Verbindungen der Formel (XXI) direkt mit 2 Äquivalenten Benzylbromid in DMF in Gegenwart von Natriumhydrid umgesetzt werden, zu 3,14-O-Dibenzylderivaten der Formel (XXIII), in der R₂ und R₄ Benzyl sind, R₁ und R₃ wie oben definiert.

Die saure Abspaltung der 3-O-Schutzgruppe und der Ketalfunktion der Verbindungen mit der Formel (XXIII) wird in einem Schritt mit einer Säure wie Salzsäure in Methanol, Tetrafluoroborsäure in Dichlormethan, Trifluoressigsäure durchgeführt, und man erhält Verbindungen der Formel (X) (s. 1. Route), 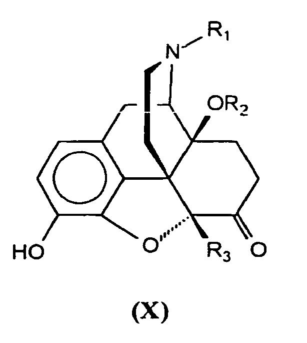 in denen R₁, R₂ und R₃ wie oben definiert sind.
Alternativ dazu kann man, wenn R₄ in Verbindungen der Formel (XXIII) Benzyl ist, durch Hydrogenolyse der 3-O-Benzylbindung mit Wasserstoffgas in Gegenwart eines Katalysators wie Pd/C, PdO, Pd/Al₂O₃, Pt/C, PtO₂, Pt/Al₂O₃, oder Ähnlichem in Lösungsmitteln wie Alkoholen, Alkohol/Wasser-Gemischen, Eisessig oder Ähnlichem, gefolgt von saurer Hydrolyse der Ketalfunktion in Position 6 mit z.B. Methanol und konzentrierter Salzsäure Verbindungen der Formel (X) erhalten.

Die Verbindungen der Formel (X) werden gemäß dem ersten Schema über die Verbindungen der Formeln (XI) bis (XIV) zu den erfindungsgemäßen Verbindungen der Formel (I) umgesetzt.

Die folgenden Beispiele beschreiben die Herstellung die erfindungsgemäßen Verbindungen im Detail.

### Beispiel 1

### Synthese von (4,5α-Epoxy-3-hydroxy-14β-methoxy-17-methylmorphinan-6α-ylamino)-essigsäure-tert.-butylester (Verbindung 1) und (4,5α-Epoxy-3-hydroxy-14β-methoxy-17-methylmorphinan-6β-ylamino)-essigsäure-tert.-butylester (Verbindung 2).

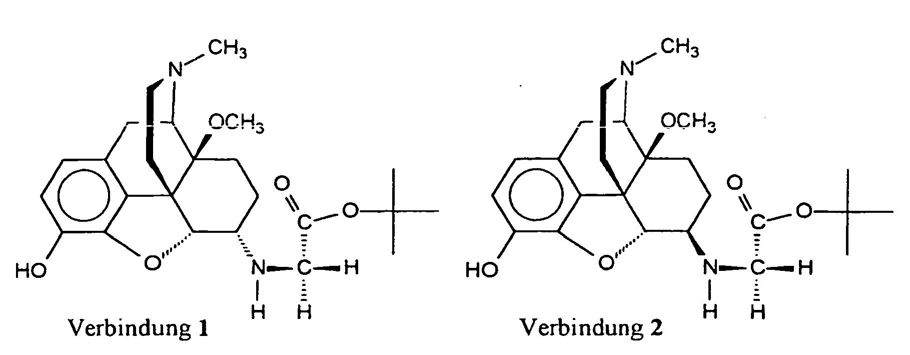

Eine Lösung von 14-O-Methyloxymorphon Hydrobromid (H. Schmidhammer et al. Helv. Chim. Acta 1990, Vol. 71, S. 1779-1783) (2,36 g, 5,96 mmol) und Glycin-tert.-butylester Hydrochlorid (1,11 g, 6,62 mmol) in absolutem MeOH (100 ml) wurde 1 h lang unter N₂ bei Raumtemperatur gerührt. Anschließend wurde eine Lösung von NaCNBH₃ (0,55 g, 8,75 mmol) in MeOH (50 ml) während 20 min. zugetropft und die Lösung weiter unter N₂ bei Raumtemperatur gerührt. Nach 19 h wurde H₂O (20 ml) zugefügt und das Gemisch eingedampft. Der Rückstand wurde mit H₂O (400 ml) versetzt, mit konz. Ammoniak alkalisiert, mit NaCl gesättigt und mit Et₂O (1 x 100 ml, 3 x 50 ml) extrahiert. Die vereinigten organischen Phasen wurden mit H₂O (1 x 200 ml) und gesättigter NaCl-Lösung (1 x 200 ml) gewaschen, getrocknet (Na₂SO₄) und eingedampft. Die wäßrige Phase wurde mit CH₂Cl₂/i-PrOH 4:1 (1 x 100 ml, 3 x 50 ml) extrahiert. Die vereinigten organischen Phasen wurden auf die gleiche Weise behandelt wie die oben beschriebene Etherphase. Aus der 1. Extraktion (Et₂O) wurden 1,05 g eines gelben Öls erhalten, in dem die 2 Produkte (Verbindung **1** und Verbindung **2**) enthalten waren. Aus der 2. Extraktion (CH₂Cl₂/i-PrOH) wurden 0,72 g eines gelben Öls erhalten, in dem neben den 2 Produkten auch die entsprechenden 6-Hydroxyderivate enthalten waren. Die 2 Produkte wurden durch MPLC (p = 5 bar, Kieselgel 60, CH₂Cl₂/MeOH 10:1) getrennt und gereinigt.
Verbindung **1**: Ausbeute: 0,28 g (11%) oranges Schaumharz. IR (KBr): 3407 (OH), 1731 (C=O) cm⁻¹; ¹H-NMR (CDCl₃): δ 6,66 (d, J = 8,1, 1 arom. H); 6,48 (d, J = 8,1, 1 arom. H); 5,05 (s, br, OH-C(3), -NH-C(6)); 4,65 (d, J = 3,6, H-C(5)); 3,42 (s, C(6)-NH-CH₂-); 3,21 (s, CH₃O-C(14)); 2,36 (s, CH₃N); 1,43 (s, -COOC(CH₃)₃); CI-MS: m/z 431 (M⁺ + 1).
Verbindung **2**: Ausbeute: 0,63 g (24%) gelbes Schaumharz. IR (KBr): 3421 (OH), 1729 (C=O) cm⁻¹; ¹H-NMR (CDCl₃): δ 6,68 (d, J = 8,0, 1 arom. H); 6,53 (d, J = 8,0, 1 arom H); 4,71 (s, br, OH-C(3), C(6)-NH-); 4,47 (d, J = 7,0, H-C(5)); 3,48 (d, J = 17,3, 1 H, C(6)-NH-CH₂-); 3,32 (d, J = 17,3, 1 H, C(6)-NH-CH₂-); 3,19 (s, CH₃O-C(14)); 2,42 (s, CH₃N); 1,42 (s, -COOC(CH₃)₃); CI-MS: m/z 431 (M⁺ + 1).

### Beispiel 2

### Synthese von (4,5α-Epoxy-3-hydroxy-14β-methoxy-17-methylmorphinan-6α-ylamino)-essigsäure Sesqui(trifluoracetat) (Verbindung 3-1,5 TFA).

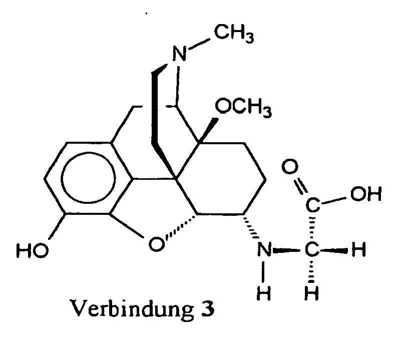

Ein Gemisch von Verbindung **1** (0,18 g, 0,42 mmol) und 30% Trifluoressigsäure (TFA) in CH₂Cl₂ (7 ml) wurde 9 h lang bei Raumtemperatur gerührt und anschließend eingedampft. Der Rückstand (0.26 g oranges Schaumharz) wurde aus i-PrOH/Et₂O/MeOH kristallisiert. Man erhielt dabei nicht das erwartete Bis(trifluoracetat), sondern das Sesqui(trifluoracetat), was durch mehrmalige Elementaranalyse nachgewiesen wurde. Ausbeute 0.13 g (57%) beiges **3**-1,5 TFA: Fp > 190° C (Zers.); IR (KBr): 3428 (OH), 1677 (C=O) cm⁻¹; ¹H-NMR (D₂O): δ 6,90 (d, J = 8,4, 1 arom. H); 6,81 (d, J = 8,4, 1 arom. H); 4,47 (dd, ³J = 3,0, ⁴J = 1,0, H-C(5)); 3,87 (d, J = 1,4, C(6)-NH-CH₂-); 3,35 (s, CH₃O-C(14)); 2,94 (s, CH₃N); ESI-MS: m/z 375 (M⁺ + 1).

### Beispiel 3

### Synthese von (4,5α-Epoxy-3-hydroxy-14β-methoxy-17-methylmorphinan-6β-ylamino)-essigsäure Sesqui(trifluoracetat) (Verbindung 4-1,5 TFA).

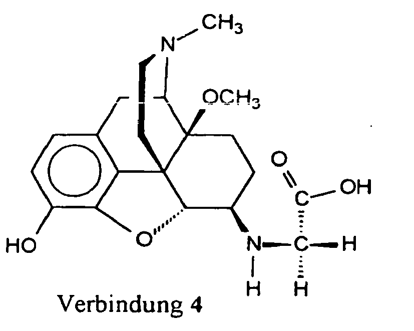

Ein Gemisch von Verbindung **2** (0,30 g, 0,70 mmol) und 30% Trifluoressigsäure (TFA) in CH₂Cl₂ (11 ml) wurde 5 h lang bei Raumtemperatur gerührt und anschließend eingedampft. Der Rückstand (0.43 g gelbes Schaumharz) wurde aus i-PrOH/Et₂O/MeOH kristallisiert. Man erhielt dabei nicht das erwartete Bis(trifluoracetat), sondern das Sesqui(trifluoracetat), was durch mehrmalige Elementaranalyse nachgewiesen wurde. Ausbeute 0.21 g (55%) beiges **4**·1,5 TFA: Fp > 210° C (Zers.); IR (KBr): 3419 (OH), 1677 (C=O) cm⁻¹; ¹H-NMR (D₂O): δ 6,89 (d, J = 8,6, 1 arom. H); 6,83 (d, J = 8,6, 1 arom. H); 4,90 (d, J = 7,8, H-C(5)); 4,04 (s, C(6)-NH-CH₂-); 3,32 (s, CH₃O-C(14)); 2,91 (s, CH₃N); ESI-MS: m/z 375 (M⁺ + 1).

### Beispiel 4

### Synthese von (2'S)-2'-(4,5α-Epoxy-3-hydroxy-14β-methoxy-17-methylmorphinan-6α-ylamino)-propionsäure-tert.-butylester (Verbindung 5) und (2'S)-2'-(4,5α-Epoxy-3-hydroxy-14β-methoxy-17-methylmorphinan-6β-ylamino)-propionsäure-tert.-butylester (Verbindung 6).

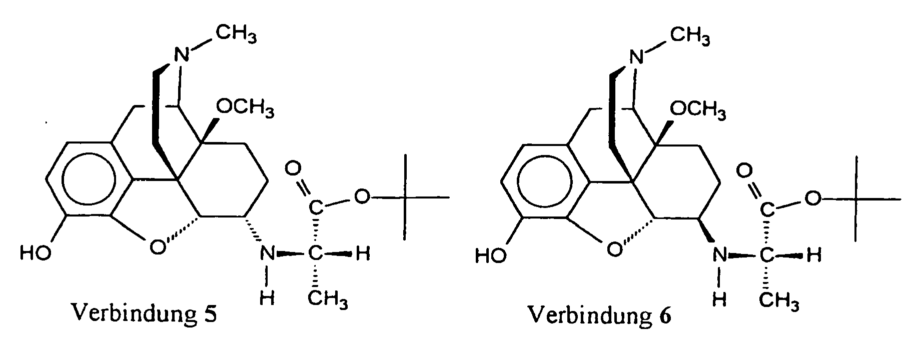

Ein Gemisch von 14-O-Methyloxymorphon Hydrobromid (H. Schmidhammer et al. Helv. Chim. Acta 1990, Vol. 71, S. 1779-1783) (2,54 g, 6,41 mmol), L-Alanin-tert.-butylester Hydrochlorid (1,75 g, 9,63 mmol), absolutem EtOH (150 ml), N-Ethyldiisopropylamin (2,8 ml, 16,07 mmol) und Molekularsieb (2,8 g) wurde 5 h lang unter N₂ bei Raumtemperatur gerührt. Anschließend wurde eine Lösung von NaCNBH₃ (0,51 g, 8,12 mmol) in EtOH (20 ml) während 20 min. zugetropft und die Lösung weiter unter N₂ bei Raumtemperatur gerührt. Nach 2 Tagen wurde H₂O (5 ml) zugefügt und das Gemisch eingedampft. Der Rückstand wurde mit H₂O (200 ml) versetzt und mit Et₂O (2 x 100 ml, 2 x 50 ml) extrahiert. Die vereinigten organischen Phasen wurden mit gesättigter NaCl-Lösung (1 x 200 ml) gewaschen, getrocknet (Na₂SO₄) und eingedampft. Die wäßrige Phase wurde mit CH₂Cl₂ (2 x 100 ml, 2 x 50 ml) extrahiert. Die vereinigten organischen Phasen wurden auf die gleiche Weise behandelt, wie die Etherphase. Aus der 1. Extraktion (Et₂O) wurden 1,34 g eines gelben Öls erhalten, in dem die 2 Produkte (Verbindung **5** und Verbindung **6**) enthalten waren. Aus der 2. Extraktion (CH₂Cl₂) wurden 0,68 g eines gelben Öls erhalten, in dem neben den 2 Produkten auch die entsprechenden 6-Hydroxyderivate enthalten waren. Die 2 Produkte wurden durch MPLC (p = 5 bar, Kieselgel 60, CH₂Cl₂/MeOH 10:1) getrennt und gereinigt. Verbindung **5** wurde aus Methanol kristallisiert, von Verbindung 6 konnte nur eine analytische Menge (50 mg) aus i-PrOH kristallisiert werden, der Rest (0,75 g) wurde als weißes Schaumharz erhalten.
Verbindung **5**: Ausbeute: 0,32 g (11%) farblose Kristalle: Fp 196-200° C; IR (KBr): 3203 (OH), 1729 (C=O) cm⁻¹; ¹H-NMR (CDCl₃): δ 6,69 (d, J = 8,2, 1 arom. H); 6,47 (d, J = 8,2, 1 arom. H); 4,70 (d, J = 3,2, H-C(5)); 3,55 (q, J = 6,8, C(6)-NH-CH(CH₃)-); 3,19 (s, CH₃O-C(14)); 2,35 (s, CH₃N); 1,47 (s, -COOC(CH₃)₃); 1,26 (d, J = 6,8, C(6)-NH-CH(CH₃)-); CI-MS: m/z 445 (M⁺ + 1).
Verbindung **6**: Ausbeute: 0,80 g (24%) farblose Kristalle und weißes Schaumharz: Fp 235-240° C (Zers.); IR (KBr): 3423 (OH), 1722 (C=O) cm⁻¹; ¹H-NMR (CDCl₃): δ 6,69 (d, J = 8,0, 1 arom. H); 6,54 (d, J = 8,2, 1 arom. H); 4,39 (d, J = 7,2, H-C(5)); 3,32 (q, J = 7,0, C(6)-NH-CH(CH₃)-); 3,20 (s, CH₃O-C(14)); 2,39 (s, CH₃N); 1,41 (s, -COOC(CH₃)₃); 1,26 (d, J = 6,8, C(6)-NH-CH(CH₃)-); CI-MS: m/z 445 (M⁺ + 1).

### Beispiel 5

### Synthese von (2'S)-2'-(4,5α-Epoxy-3-hydroxy-14β-methoxy-17-methylmorphinan-6α-ylamino)-propionsäure Bis(tetrafluoroborat) (Verbindung 7-2 HBF₄).

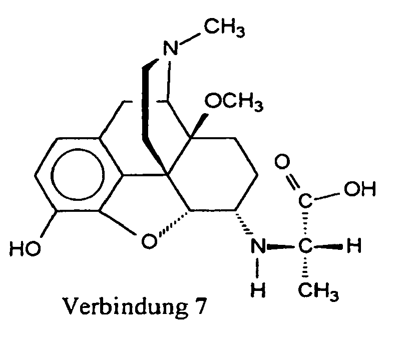

Eine Lösung von Verbindung **5** (0,30 g, 0,70 mmol) in CH₂Cl₂ (3 ml) wurde mit 54% Tetrafluoroborsäure (HBF₄) in Et₂O (0,33 ml, 2,39 mmol) versetzt und das Gemisch 1 h lang bei Raumtemperatur ultrabeschallt. Der dabei entstandene Niederschlag wurde abfiltriert und getrocknet. Ausbeute 0.21 g (79%) weißes 7-2 HBF₄: Fp > 290° C (Zers.); IR (KBr): 3423 (OH), 1741 (C=O), 1064 (B-F) cm⁻¹; ¹H-NMR (D₂O): δ 6,90 (d, J = 8,0, 1 arom H); 6,81 (d, J = 8,0, 1 arom. H); 5,02 (d, J = 2,8, H-C(5)); 4,24 (q, J = 7,0, C(6)-NH-CH(CH₃)-); 3,35 (s, CH₃O-C(14)); 2,94 (s, CH₃N); 1,63 (d, J = 7,0, C(6)-NH-CH(CH₃)-).

### Beispiel 6

### Synthese von (2'S)-2'-(4,5α-Epoxy-3-hydroxy-14β-methoxy-17-methylmorphinan-6β-ylamino)-propionsäure Bis(tetrafluoroborat) (Verbindung 8-2 HBF₄).

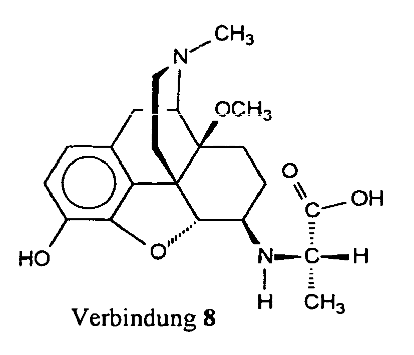

Eine Lösung von Verbindung **6** (0,25 g, 0,56 mmol) in CH₂C1₂ (4 ml) wurde mit 54% Tetrafluoroborsäure (HBF₄) in Et₂O (0,39 ml, 2,85 mmol) versetzt und das Gemisch 1 h lang bei Raumtemperatur ultrabeschallt. Der dabei entstandene Niederschlag wurde abfiltriert und getrocknet. Ausbeute 0.28 g (89%) weißes **8**.2 HBF₄: Fp > 290° C (Zers.); IR (KBr): 3423 (OH), 1720 (C=O), 1083 (B-F) cm⁻¹; ¹H-NMR (D₂O): δ 6,87 (s, 2 arom. H); 4,86 (d, J = 7,6, H-C(5)); 4,31 (q, J = 7,0, C(6)-NH-CH(CH₃)-); 3,33 (s, CH₃O-C(14)); 2,92 (s, CH₃N); 1,58 (d, J = 7,0, C(6)-NH-CH(CH₃)-).

### Beispiel 7

### Synthese von (2'S)-2'-(4,5α-Epoxy-3-hydroxy-14β-methoxy-17-methylmorphinan-6α-ylamino)-3'-phenylpropionsäure-tert.-butylester (Verbindung 9) und (2'S)-2'-(4,5α-Epoxy-3-hydroxy-14β-methoxy-17-methylmorphinan-6β-ylamino)-3'-phenylpropionsäure-tert.-butylester (Verbindung 10).

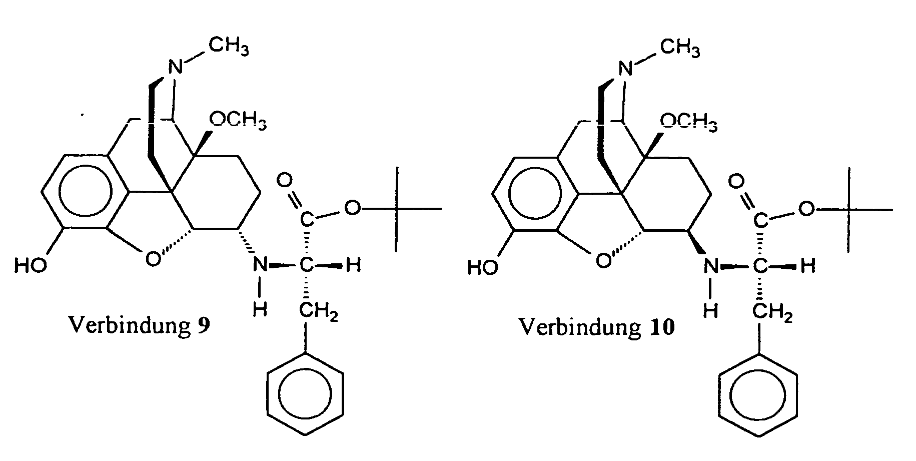

Ein Gemisch von 14-O-Methyloxymorphon Hydrobromid (H. Schmidhammer et al. Helv. Chim. Acta 1990, Vol. 71, S. 1779-1783) (2,70 g, 6,81 mmol), L-Phenylalanin-tert.-butylester Hydrochlorid (2,74 g, 10,63 mmol), absolutem EtOH (150 ml), N-Ethyldiisopropylamin (3,04 ml, 17,49 mmol) und Molekularsieb (3,0 g) wurde 2,5 h lang unter N₂ bei Raumtemperatur gerührt. Anschließend wurde eine Lösung von NaCNBH₃ (0,47 g, 7,48 mmol) in EtOH (20 ml) während 20 min. zugetropft und die Lösung weiter unter N₂ bei Raumtemperatur gerührt. Nach 3 Tagen wurde H₂O (10 ml) zugefügt und das Gemisch eingedampft. Der Rückstand wurde mit H₂O (300 ml) versetzt und mit CH₂Cl₂ (1 x 100 ml, 4 x 50 ml) extrahiert. Die vereinigten organischen Phasen wurden durch Celite filtriert, mit gesättigter NaCl-Lösung (1 x 200 ml) gewaschen, getrocknet (Na₂SO₄) und eingedampft. Dabei erhielt man 3,96 g eines gelben Öls, aus dem die 2 Produkte mittels MPLC (p = 5 bar, Kieselgel 60, CH₂Cl₂/MeOH 10:1) jeweils rein erhalten wurden. Es wurden auch 0,68 g der Ausgangsverbindung (14-O-Methyloxymorphon) als braunes Schaumharz zurückgewonnen.
Verbindung **9**: Ausbeute: 0,34 g (10%) oranges Schaumharz: IR (KBr): 3336 (OH), 1725 (C=O) cm⁻¹; ¹H-NMR (CDCl₃): δ 7,31-7,17 (m, 5 arom. H); 6,71 (d, J = 8,0, 1 arom. H); 6,47 (d, J = 8,0, 1 arom H); 4,71 (d, J = 3,2, H-C(5)); 3,77-3,69 (m, C(6)-NH-CH(CH₂Ph)-); 3,12 (s, CH₃O-C(14)); 2,94-2,90 (m, C(6)-NH-CH(CH₂Ph)-); 2,35 (s, CH₃N); 1,32 (s, -COOC(CH₃)₃); CI-MS: m/z 521 (M⁺ + 1).
Verbindung **10**: Ausbeute: 0,81 g (23%) oranges Schaumharz: IR (KBr): 3409 (OH), 1724 (C=O) cm⁻¹; ¹H-NMR (CDCl₃): δ 7,29-7,17 (m, 5 arom. H); 6,70 (d, J = 8,0, 1 arom. H); 6,54 (*d,* J = 8,0, 1 arom. H); 4,39 (*d,* J = 7,4, H-C(5)); 3,51-3,43 (*m,* C(6)-NH-CH(CH₂Ph)-); 3,20 (*s*, CH₃O-C(14)); 2,98-2,78 (*m*, C(6)-NH-CH(CH₂Ph)-); 2,44 (*s*, CH₃N); 1,28 (*s*, -COOC(CH₃)₃); CI-MS: m/z 521 (M⁺ + 1).

### Beispiel 8

### Synthese von (2'S)-2'-(4,5α-Epoxy-3-hydroxy-14β-methoxy-17-methylmorphinan-6α-ylamino)-3'-phenylpropionsäure Bis(tetrafluoroborat) (Verbindung 11-2 HBF₄).

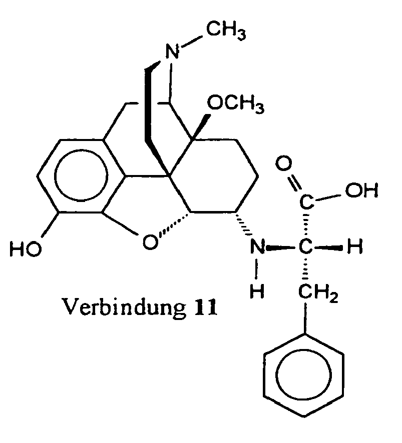

Eine Lösung von Verbindung **9** (0,16 g, 0,31 mmol) in CH₂Cl₂ (3 ml) wurde mit 54% Tetrafluoroborsäure (HBF₄) in Et₂O (0,25 ml, 1,81 mmol) versetzt und das Gemisch 30 min. lang bei Raumtemperatur ultrabeschallt. Anschließend wurde das Gemisch eingedampft, der Rückstand (0,21 g oranges Öl) in H₂O gelöst und gefriergetrocknet. Ausbeute 0.18 g (90%) weißes Lyophilisat: ¹H-NMR (D₂O): δ 7,46-7,35 (m, 5 arom. H); 6,86 (d, J = 8,2, 1 arom. H); 6,77 (d, J = 8,2, 1 arom. H); 4,90 (d, J = 3,4, H-C(5)); 4,46 (t, J = 6,8, C(6)-NH-CH(CH₂Ph)-); 3,35 (d, J = 6,8, C(6)-NH-CH(CH₂Ph)-); 3,25 (s, CH₃O-C(14)); 2,90 (s, CH₃N).

### Beispiel 9

### Synthese von (2'S)-2'-(4,5α-Epoxy-3-hydroxy-14β-methoxy-17-methylmorphinan-6β-ylamino)-3'-phenylpropionsäure Bis(tetrafluoroborat) (Verbindung 12-2 HBF₄).

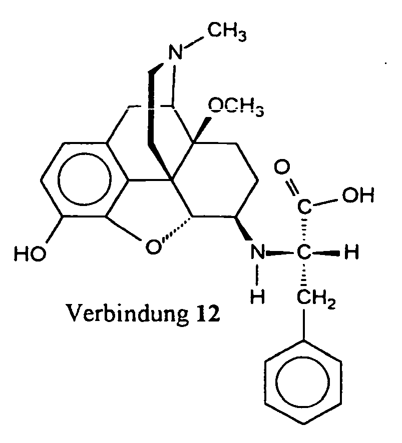

Eine Lösung von Verbindung **10** (0,41 g, 0,79 mmol) in CH₂Cl₂ (5 ml) wurde mit 54% Tetrafluoroborsäure (HBF₄) in Et₂O (0,60 ml, 4,35 mmol) versetzt und das Gemisch 30 min. lang bei Raumtemperatur ultrabeschallt. Anschließend wurde das Gemisch eingedampft, der Rückstand (0,54 g oranges Öl) in H₂O gelöst und gefriergetrocknet. Ausbeute 0.46 g (90%) weißes Lyophilisat: ¹H-NMR (D₂O): δ 7,28 (s, 5 arom. H); 6,88 (d, J = 8,4, 1 arom. H); 6,81 (d, J = 8,4, 1 arom. H); 4,83 (d, J = 7,6, H-C(5)); 4,54 (t, J = 7,0, C(6)-NH-CH(CH₂Ph)-); 3,25 (s, CH₃O-C(14)); 2,86 (s, CH₃N).

### Beispiel 10

### Synthese von 6α-Amino-4,5α-epoxy-14β-methoxy-17-methylmorphinan-3-ol (Verbindung 13).

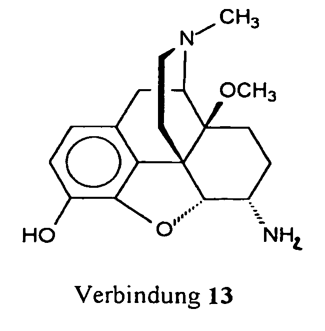

Ein Gemisch von 14-O-Methyloxymorphon Hydrobromid (H. Schmidhammer et al. Helv. Chim Acta 1990, Vol. 71, S. 1779-1783) (6,22 g, 15,70 mmol), Ammoniumacetat (12,00 g, 156 mmol), NaCNBH₃ (0,81 g, 7,64 mmol) und absolutem MeOH (100 ml) wurde 23 h lang unter N₂ bei Raumtemperatur gerührt. Anschließend wurde die Lösung mit konzentrierter HCI angesäuert (beiger Niederschlag) und das Gemisch eingedampft. Der Rückstand wurde in H₂O (550 ml) gelöst, zur Entfernung der wasserunlöslichen Komponenten mit CH₂Cl₂ (1 x 200 ml) extrahiert. Die wäßrige Phase wurde mit konz. Ammoniak alkalisiert, mit NaCl gesättigt und mit CH₂Cl₂/i-PrOH 4:1 (2 x 250 ml, 3 x 125 ml) extrahiert. Die vereinigten organischen Phasen wurden mit gesättigter NaCl-Lösung (1 x 200 ml) gewaschen, getrocknet (Na₂SO₄) und eingedampft. Der Eindampfrückstand (beige Kristalle) wurde aus Methanol umkristallisiert. Ausbeute: 1,95 g (39%) weißes Pulver: Fp > 300° C (Zers.); IR (KBr): 3421 (OH) cm⁻¹; ¹H-NMR (Me₂SO-d₆): δ 6,55 (d, J = 8,0, 1 arom. H); 6,29 (d, J = 8,0, 1 arom. H); 4,33 (dd, ³J = 4,0, ⁴J = 0,8, H-C(5)); 3,38 (s, br, OH-C(3), NH₂-C(6)); 3,13 (s, CH₃O-C(14)); 2,24 (s, CH₃N); CI-MS: m/z 317 (M⁺ + 1).

### Beispiel 11 (Referenz)

### Synthese von 6β-Dibenzylamino-4,5α-epoxy-14β-methoxy-17-methylmorphinan-3-ol (Verbindung 14).

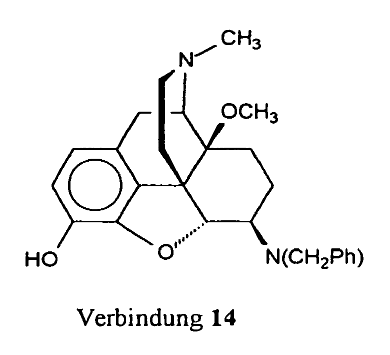

Eine Lösung von 14-O-Methyloxymorphon Hydrobromid (H. Schmidhammer et al. Helv. Chim. Acta 1990, Vol. 71, S. 1779-1783) (2,00 g, 5,05 mmol) in MeOH/H₂O 9:1 (80 ml) wurde mit Silberbenzoat (1,17 g, 5,11 mmol) versetzt und 90 min. lang bei 40° C gerührt. Der dabei entstandene Niederschlag von Silberbromid wurde abfiltriert und das Filtrat eingedampft. Der Rückstand wurde mit EtOH/Toluol 2:3 (50 ml) versetzt und das Lösungsmittel abgezogen Dabei wurden 2,35 g 14-O-Methyloxymorphon Benzoat als gelbes Schaumharz erhalten. Dieses wurde mit Toluol (250 ml), Benzoesäure (0,93 g, 7,62 mmol), Dibenzylamin (1,49 g, 7,54 mmol) und einer Spatelspitze p-Toluolsulfonsäure Monohydrat versetzt und das Gemisch unter Verwendung eines Wasserabscheiders 20 h lang rückflußerhitzt. Anschließend wurde die Lösung auf ein Volumen von 50 ml eingeengt, absolutes EtOH (220 ml), NaCNBH₃ (0,30 g, 4,77 mmol) und Molekularsieb zugesetzt und die Lösung 6 h lang unter N₂ bei Raumtemperatur gerührt. Das Gemisch wurde mit MeOH (100 ml) verdünnt, filtriert und das Filtrat eingedampft. Der Rückstand wurde mit H₂O (550 ml) versetzt, mit konz. Ammoniak alkalisiert, und mit CH₂Cl₂ (1 x 200 ml, 3 x 100 ml) extrahiert. Die vereinigten organischen Phasen wurden mit H₂O (5 x 300 ml) und gesättigter NaCl-Lösung (1 x 200 ml) gewaschen, getrocknet (Na₂SO₄) und eingedampft. Der Eindampfrückstand (2,42 g braunes Öl) wurde aus Methanol kristallisiert. Ausbeute: 1,43 g (57%) beige Kristalle: Fp 124-128° C; IR (KBr): 3178. (OH) cm⁻¹; ¹H-NMR (CDCl₃): δ 7,45-7,20 (m, 10 arom. H); 6,56 (d, J = 8,1, 1 arom. H); 6,44 (d, J = 8,1, 1 arom. H); 4,72 (d, J = 6,8, H-C(5)); 3,87 (d, J = 14,0, 2 H, (PhCH₂)₂N-C(6)); 3,61 (d, J = 14,0, 2 H, (PhCH₂)₂N-C(6)); 3,20 (s, CH₃O-C(14)); 2,34 (s, CH₃N); CI-MS: m/z 497 (M⁺ + 1).

### Beispiel 12

### Synthese von 6β-Amino-4,5α-epoxy-14β-methoxy-17-methylmorphinan-3-ol (Verbindung 15).

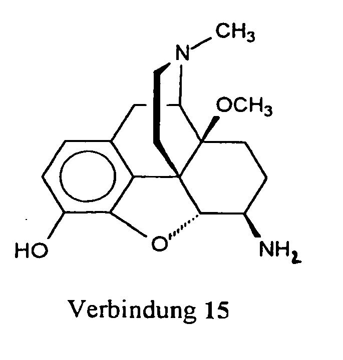

Ein Gemisch von Verbindung **14** (1,02 g, 2,05 mmol), 10% Pd/C-Katalysator (0,52 g), Cyclohexen (30 ml) und absolutem MeOH (30 ml) wurde 16 h lang unter N₂ rückflußerhitzt. Anschließend wurde der Katalysator abfiltriert und das Filtrat eingedampft. Der Rückstand (0,66 g weißes Schaumharz) wurde aus i-PrOH/Et₂O 1:1 (2 ml) kristallisiert. Ausbeute: 0,33 g (42%) beige Kristalle: Fp > 235-239° C; IR (KBr): 3348 (OH) cm⁻¹ ; ¹H-NMR (CDCl₃): 6,62 (*d,* J = 8,0, 1 arom. H); 6,54 (*d,* J = 8,0, 1 arom. H); 4,26 (*d,* J = 7,0, H-C(5)); 3,22 (*s,* CH₃O-C(14)); 2,36 (*s,* CH₃N); CI-MS: m/z 317 (M⁺ + 1).

### Beispiel 13

### Synthese von 4,5α-Epoxy-6β-[N,N'-bis-(tert.-butoxycarbonyl)guanidinyl]-14β-methoxy-17-methylmorphinan-3-ol (Verbindung 16).

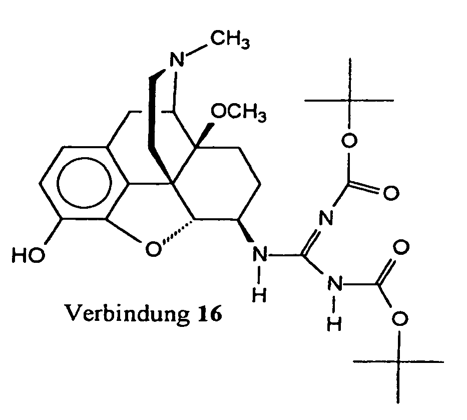

Eine Lösung von Verbindung **15** (0,94 g, 2,97 mmol), N,N'-Bis-(tert.-butoxycarbonyl)-S-methylisothioharnstoff (0,92 g, 3,17 mmol) und N-Ethyldiisopropylamin (0,58 ml, 3,33 mmol) in absolutem N,N-Dimethylformamid (40 ml) wurde mit Silbernitrat (0,54 g, 3,18 mmol) versetzt und das Gemisch 4 h lang gerührt. Anschließend wurde das Silbermercaptan durch Celite abfiltriert und mit CH₂Cl₂ (4 x 50 ml) nachgewaschen. Das Filtrat wurde mit H₂O (10 x 150 ml) und gesättigter NaCl-Lösung (2 x 150 ml) gewaschen, getrocknet (Na₂SO₄) und eingedampft. Dabei wurden 1,46 g eines gelben Schaumharzes erhalten, das durch MPLC (p = 5 bar, Kieselgel 60, CH₂Cl₂/MeOH/konz. Ammoniak 95:4,5:0,5) gereinigt wurde. Ausbeute: 0,77 g (46%) grünliches Schaumharz: ¹H-NMR (CDCl₃): δ 11,49 (s, br, NH-COO(CH₃)₃); 8,59 (d, J = 8,0, C(6)-NH-); 6,71 (d, J = 8,4, 1 arom H); 6,56 (d, J = 8,4, 1 arom. H); 4,41 (d, J = 7,2, H-C(5)); 3,22 (s, CH₃O-C(14)); 2,38 (s, CH₃N); 1,51 (s, C(CH₃)₃), 1,47 (s, C(CH₃)₃).

### Beispiel 14

### Synthese von 4,5α-Epoxy-6β-guanidinyl-14β-methoxy-17-methylmorphinan-3-ol Dihydrochlorid (Verbindung 17-2 HCl).

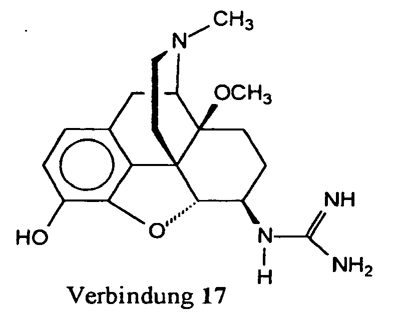

Eine Lösung von Verbindung **16** (50 mg, 0,089 mmol) in Et₂O (3 ml) wurde bis zur deutlich sauren Reaktion mit etherischer HCl sowie mit 4 Tropfen H₂O versetzt. Das Gemisch wurde 1 h lang bei Raumtemperatur ultrabeschallt und anschließend eingedampft. Der Rückstand (40 mg weißes Schaumharz) wurde in H₂O gelöst und gefriergetrocknet. Ausbeute 30 mg (79%) **17**·2 HCl als weißes Lyophilisat: ¹H-NMR (CDCl₃): δ 9,59 (s, OH-C(3)); 9,29 (s, br, NH⁺); 8,53 (d, J = 8,0, C(6)-NH-); 7,29 (s, br, C(6)-NH-C(NH₂)₂⁺), 6,78 (d, J = 8,1, 1 arom. H); 6,69 (d, J = 8,1, 1 arom. H), 4,49 (d, J = 7,2, H-C(5)); 3,26 (s, CH₃O-C(14)); 2,84 (s, CH₃N).

### Beispiel 15

### Synthese von 4,5α-Epoxy-6α-[N,N'-bis(tert.-butoxycarbonyl)guanidinyl]-14β-methoxy-17-methylmorphinan-3-ol (Verbindung 18).

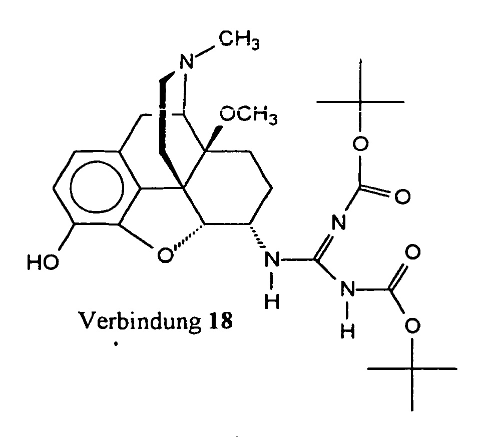

Eine Lösung von Verbindung **13** (1,00 g, 3,16 mmol), N,N'-Bis-(tert.-butoxycarbonyl)-S-methylisothioharnstoff (1,00 g, 3,44 mmol) und N-Ethyldüsopropylamin (0,60 ml, 3,44 mmol) in absolutem N,N-Dimethylformamid (60 ml) wurde mit Silbernitrat (0,55 g, 3,24 mmol) versetzt und das Gemisch 1,5 h lang gerührt. Anschließend wurde das Silbermercaptan durch Celite abfiltriert und mit CH₂Cl₂ (4 x 50 ml) nachgewaschen. Das Filtrat wurde mit H₂O (6 x 200 ml) und gesättigter NaCl-Lösung (1 x 200 ml) gewaschen, getrocknet (Na₂SO₄) und eingedampft. Dabei wurden 1,85 g eines gelben Öls erhalten, das durch MPLC (p = 5 bar, Kieselgel 60, CH₂Cl₂/MeOH 10:1) gereinigt wurde. Ausbeute: 0,67 g (38%) weißes Schaumharz: ¹H-NMR (CDCl₃): δ 11,53 (s, br, NH-COO(CH₃)₃); 8,81 (d, J = 8,0, C(6)-NH-); 6,73 (d, J = 8,2, 1 arom. H); 6,56 (d, J = 8,2, 1 arom. H); 4,66 (dd, ³J = 2,6, ⁴J = 1,6, H-C(5)); 3,25 (s, CH₃O-C(14)); 2,35 (s, CH₃N); 1,50 (s, 2 x C(CH₃)₃).

### Beispiel 16

### Synthese von 4,5α-Epoxy-6α-guanidinyl-14β-methoxy-17-methylmorphinan-3-ol Dihydrochlorid (Verbindung 19-2 HCl).

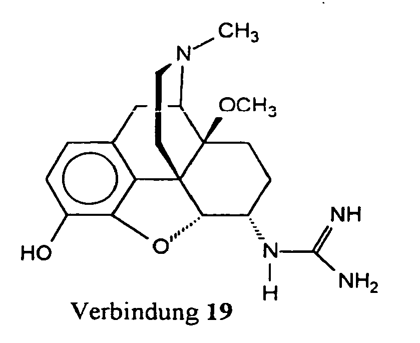

Eine Lösung von Verbindung **18** (50 mg, 0,089 mmol) in Et₂O (3 ml) wurde bis zur deutlich sauren Reaktion mit etherischer HCl sowie mit 4 Tropfen H₂O versetzt. Das Gemisch 1,5 h lang bei Raumtemperatur ultrabeschallt und anschließend eingedampft. Der Rückstand (40 mg weißes Schaumharz) wurde in H₂O gelöst und gefriergetrocknet. Ausbeute 35 mg (92%) **19**·2 HCl als weißes Lyophilisat: ¹H-NMR (CDCl₃): δ 9,29 (s, br, NH⁺); 9,20 (s, OH-C(3)); 7,57 (d, J = 8,8, C(6)-NH-); 7,46 (s, br, C(6)-NH-C(NH₂)₂⁺), 6,76 (d, J = 8,1, 1 arom. H); 6,62 (d, J = 8,1, 1 arom. H); 4,70 (d, J = 4,0, H-C(5)); 3,36 (s, CH₃O-C(14)); 2,88 (s, CH₃N).

### Beispiel 17

### Synthese von 1,3-Bis-(tert.-butoxycarbonyl)-2-{4,5α-Epoxy-6β-[N,N'-bis-(tert.-butoxycarbonyl)guanidinyl]-14β-methoxy-17-methylmorphinan-3-yl}-isoharnstoff (Verbindung 20).

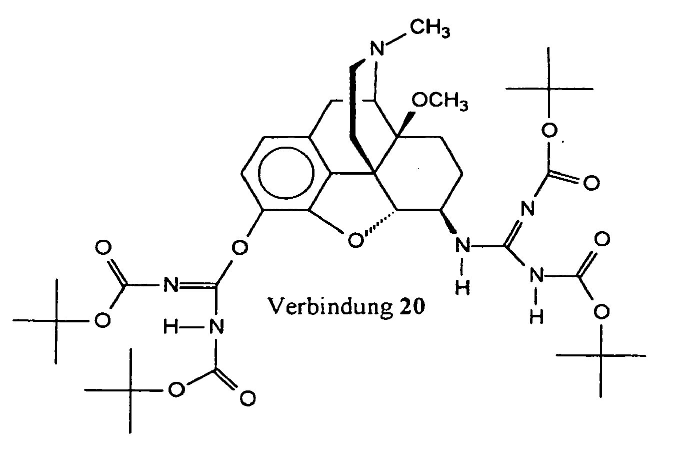

Eine Lösung von Verbindung **15** (0,12 g, 0,38 mmol), N,N'-Bis-(tert.-butoxycarbonyl)-S-methylisothioharnstoff (0,24 g, 0,83 mmol) und Triethylamin (0,12 ml, 0,86 mmol) in absolutem N,N-Dimethylformamid (4 ml) wurde mit Silbernitrat (0,14 g, 0,82 mmol) versetzt und das Gemisch 17 h lang gerührt. Anschließend wurde das Silbermercaptan durch Celite abfiltriert und mit CH₂Cl₂ (4 x 50 ml) nachgewaschen. Das Filtrat wurde mit H₂O (5 x 200 ml) und gesättigter NaCl-Lösung (1 x 200 ml) gewaschen, getrocknet (Na₂SO₄) und eingedampft. Dabei wurden 0,10 g eines gelblichen Schaumharzes erhalten, das durch MPLC (p = 5 bar, Kieselgel 60, CH₂Cl₂/MeOH/konz. Ammoniak 100:5:0,5) gereinigt wurde. Ausbeute: 45 mg (15%) weißes Schaumharz: ¹H-NMR (CDCl₃): δ 11,54 (s, NH-COO(CH₃)₃); 10,44 (s, br, NH-COO(CH₃)₃); 8,59 (d, J = 8,8, C(6)-NH-); 6,91 (d, J = 8,0, 1 arom. H); 6,64 (d, J = 8,0, 1 arom. H); 4,51 (d, J = 4,4, H-C(5)); 3,28 (s, CH₃O-C(14)); 2,37 (s, CH₃N); 1,51 (s, 2 x C(CH₃)₃), 1,47 (s, 2 x C(CH₃)₃). FAB-MS: m/z 801 (M⁺ + 1).

### Beispiel 18

### Synthese von 1,3-Bis-(tert.-butoxycarbonyl)-2-{4,5α-Epoxy-6α-[N,N'-bis-(tert.-butoxycarbonyl)guanidinyl]-14β-methoxy-17-methylmorphinan-3-yl}-isoharnstoff (Verbindung 21).

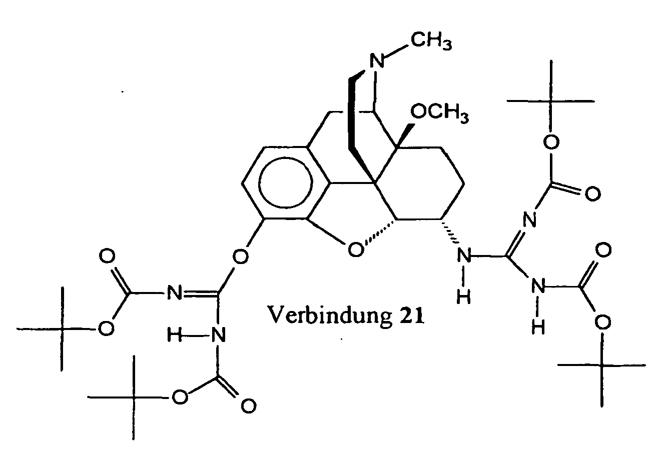

Eine Lösung von Verbindung **13** (0,50 g, 1,58 mmol), N,N'-Bis-(tert.-butoxycarbonyl)-S-methylisothioharnstoff (1,00 g, 3.54 mmol) und Triethylamin (0,5 ml, 4,94 mmol) in absolutem N,N-Dimethylformamid (15 ml) wurde mit Silbernitrat (0,58 g, 3.12 mmol) versetzt und das Gemisch 20 h lang gerührt. Anschließend wurde das Silbermercaptan durch Celite abfiltriert und mit CH₂Cl₂ (5 x 50 ml) nachgewaschen. Das Filtrat wurde mit H₂O (5 x 200 ml) und gesättigter NaCl-Lösung (1 x 200 ml) gewaschen, getrocknet (Na₂SO₄) und eingedampft. Dabei wurden 1,14g eines gelblichen Schaumharzes erhalten, das durch MPLC (p = 5 bar, Kieselgel 60, CH₂Cl₂/MeOH/konz. Ammoniak 100:5:0,5) gereinigt wurde.
Ausbeute: 0,75 g (62%) weißes Schaumharz: ¹H-NMR (CDCl₃): δ 11,56 (s, NH-COO(CH₃)₃); 10,69 (s, br, NH-COO(CH₃)₃); 8,68 (d, J = 8,8, C(6)-NH-); 6,87 (d, J = 8,0, 1 arom. H); 6,66 (d, J = 8,0, 1 arom. H); 4,63 (d, J = 3,6, H-C(5)); 3,27 (s, CH₃O-C(14)); 2,36 (s, CH₃N); 1,51 (s, 4 x C(CH₃)₃). ESI-MS: m/z 801 (M⁺ + 1).

### Beispiel 19

### Synthese von (4,5α-Epoxy-3-hydroxy-14β-methoxy-17-methylmorphinan-6α-ylamino)-essigsäureethylester Diydrochlorid (Verbindung 22-2 HCl) und (4,5α-Epoxy-3-hydroxy-14β-methoxy-17-methylmorphinan-6β-ylamino)-essigsäureethylester Dihydrochlorid (Verbindung 23- 2 HCl).

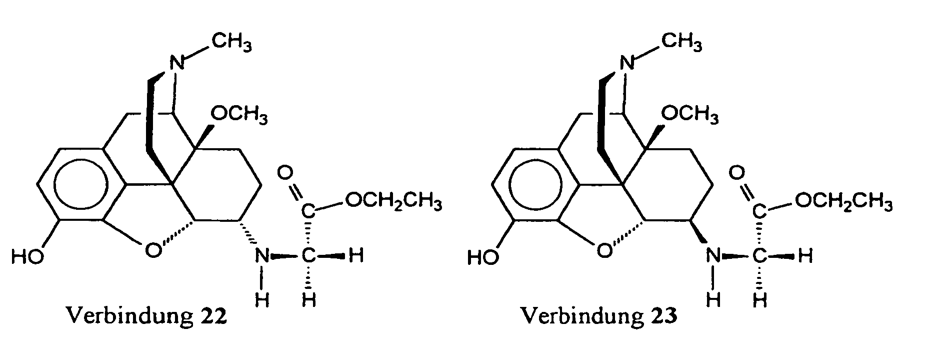

Ein Gemisch von 14-O-Methyloxymorphon Hydrobromid (H. Schmidhammer et al. Helv. Chim. Acta 1990, Vol. 71, S. 1779-1783) (2,00 g, 5,05 mmol), Glycinethylester Hydrochlorid (1,06 g, 7,59 mmol), absolutem EtOH (100 ml), Triethylamin (1,8 ml, 12,91 mmol) und Molekularsieb (2,5 g) wurde 3,5 h lang unter N₂ bei Raumtemperatur gerührt. Anschließend wurde NaCNBH₃ (0,49 g, 7,80 mmol) in mehreren Portionen zugegeben und die Lösung weiter unter N₂ bei Raumtemperatur gerührt. Nach 4 Tagen wurde H₂O (5 ml) zugefügt und das Gemisch eingedampft. Der Rückstand wurde mit H₂O (200 ml) versetzt und mit CH₂Cl₂ (2 x 100 ml, 2 x 50 ml) extrahiert. Die vereinigten organischen Phasen wurden mit gesättigter NaCl-Lösung (2 x 100 ml) gewaschen, getrocknet (Na₂SO₄) und eingedampft, woraus 0,76 g eines braunen Öls resultierten. Die 2 Produkte wurden durch MPLC (p = 4 bar, Kieselgel 60, CH₂Cl₂/MeOH 10:2) getrennt und gereinigt. Anschließend wurden sie in wenig MeOH gelöst und mittels etherischer HCl in die Dihydrochloride übergeführt. Da keine Kristallisation von **22**·2 HCl erfolgte, wurde das Lösungsmittel abgezogen, der Rückstand in H₂O gelöst und gefriergetrocknet. Die Mutterlauge von **23**·2 HCl wurde ebenfalls eingedampft, der Rückstand in H₂O gelöst und gefriergetrocknet.
Verbindung **22**22 HCl: Ausbeute: 0,20 g (8%) gelbliches Lyophilisat: IR (KBr): 3423 (OH), 1743 (C=O) cm⁻¹; ¹H-NMR (DMSO-d₆): δ 9,81, 9,50, 9,34 (3 s, 4 H, OH, NH⁺, NH₂⁺), 6,81 (d, J = 8,1, 1 arom. H); 6,64 (d, J = 8,1, 1 arom. H); 4,50 (d, J = 3,6, H-C(5)); 4,23 (q, J = 6,9, -COOCH₂CH₃); 3,31 (s, CH₃O-C(14)); 2,88 (d, J = 4,4, CH₃N); 1,26 (t, J = 6,9, -COOCH₂CH₃).
Verbindung **23**-2 HCl: Ausbeute: 0,20 g (8%) weiße Kristalle (0,11 g) und gelbliches Lyophilisat (0,09 g): Fp > 200°C (Zers.); IR (KBr): 3413 (OH), 1745 (C=O) cm⁻¹; ¹H-NMR (DMSO-d₆): δ 10,02, 9,61, 9,51 (3 s, 4 H, OH, NH⁺, NH₂⁺), 6,82 (d, J = 8,0, 1 arom. H); 6,70 (d, J = 8,0, 1 arom. H); 4,95 (d, J = 7,4, H-C(5)); 4,22 (q, J = 7,0, -COOCH₂CH₃); 3,26 (s, CH₃O-C(14)); 2,85 (s, CH₃N); 1,25 (t, J = 7,0, -COOCH₂CH₃).

### Beispiel 20

### Synthese von (4,5α-Epoxy-3-hydroxy-14β-ethoxy-17-methylmorphinan-6α-ylamino)-essigsäure-tert.-butylester (Verbindung 24) und (4,5α-Epoxy-3-hydroxy-14β-ethoxy-17-methylmorphinan-6β-ylamino)-essigsäure-tert.-butylester (Verbindung 25).

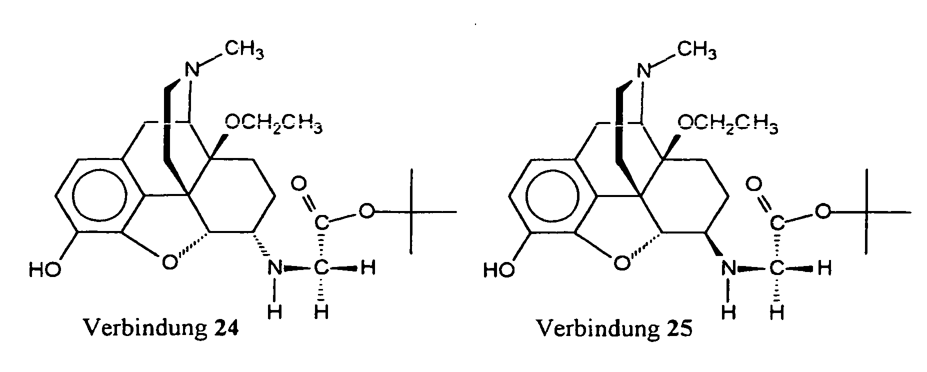

Ein Gemisch von 14-O-Ethyloxymorphon (H. Schmidhammer, R. Krassnig Sci. Pharm. 1990, Vol. 58, S. 255-257) (1,02 g, 3,09 mmol), Glycin-tert.-butylester Hydrochlorid (0,7 g, 4,63 mmol), absolutem EtOH (100 ml), N-Ethyldiisopropylamin (0,9 ml, 5,0 mmol) und Molekularsieb (2 g) wurde 3 h lang unter N₂ bei Raumtemperatur gerührt. Anschließend wurde eine Lösung von NaCNBH₃ (0,25 g, 3,98 mmol) in Ethanol (20 ml) zugetropft und die Lösung weiter unter N₂ bei Raumtemperatur gerührt. Nach 2 Tagen wurde H₂O (5 ml) zugefügt, durch Celite filtriert und das Filtrat eingedampft. Der Rückstand wurde mit H₂O (150 ml) versetzt und mit Et₂O (2 x 100 ml, 1 x 80 ml, 2 x 50 ml) extrahiert. Die vereinigten organischen Phasen wurden mit gesättigter NaCl-Lösung (3 x 100 ml) gewaschen, getrocknet (Na₂SO₄) und eingedampft. Die wäßrige Phase wurde mit CH₂Cl₂/i-PrOH 4:1 (2 x 100 ml) extrahiert. Die vereinigten organischen Phasen wurden auf die gleiche Weise behandelt wie die oben beschriebene Etherphase. Aus der 1. Extraktion (Et₂O) wurden 1,05 g eines gelben Schaumharzes erhalten, aus der 2. Extraktion (CH₂Cl₂/i-PrOH) 0,17 g eines weißen Schaumharzes. Die 2 Produkte wurden durch MPLC (p = 5 bar, Kieselgel 60, CH₂Cl₂/meOH 10:1) getrennt und gereinigt.
Verbindung **24**: Ausbeute: 0,09 g (7%) weißes Schaumharz. IR (KBr): 3425 (OH), 1735 (C=O) cm⁻¹; ¹H-NMR (CDCl₃): δ 6,66 (d, J = 8,0, 1 arom. H); 6,47 (d, J = 8,0, 1 arom. H); 4,68 (d, J = 2,6, H-C(5)); 3,43 (s, C(6)-NH-CH₂-); 2,32 (s, CH₃N); 1,45 (s, -COOC(CH₃)₃); 1,15 (t, J = 7,0, C(14)-OCH₂CH₃); CI-MS: m/z 445 (M⁺ + 1).
Verbindung **25**: Ausbeute: 0,19 g (14%) weißes Schaumharz. IR (KBr): 3440 (OH), 1734 (C=O) cm⁻¹; ¹H-NMR (CDCl₃): δ 6,67 (d, J = 8,0, 1 arom. H); 6,53 (d, J = 8,0, 1 arom. H); 4,48 (d, J = 7,0, H-C(5)); 3,50 (d, J = 17,2, 1 H, C(6)-NH-CH₂-); 3,23 (d, J = 17,2, 1 H, C(6)-NH-CH₂-), 2,33 (s, CH₃N); 1,44 (s, -COOC(CH₃)₃); 1,19 (t, J = 7,0, C(14)-OCH₂CH₃); CI-MS: m/z 445 (M⁺ + 1).

### Beispiel 21

### Synthese von (4,5α-Epoxy-3-hydroxy-14β-ethoxy-17-methylmorphinan-6α-ylamino)-essigsäure Bis(tetrafluoroborat) (Verbindung 26-2 HBF₄)

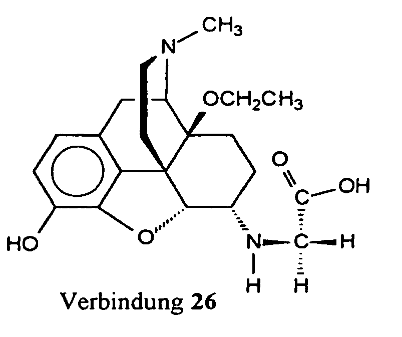

Eine Lösung von Verbindung **24** (0,05 g, 0,11 mmol) in CH₂Cl₂ (3 ml) wurde mit 54% Tetrafluoroborsäure (HBF₄) in Et₂O (0,08 ml) versetzt und das Gemisch 15 min. lang bei Raumtemperatur ultrabeschallt. Der dabei entstandene Niederschlag wurde abfiltriert und getrocknet. Ausbeute 0.03 g (53%) weißes 3.2 HBF₄: Fp > 286°C (Zers.); IR (KBr): 3466 (OH), 1735 (C=O), 1067 (B-F) cm⁻¹; ¹H-NMR (D₂O): δ 6,90 (d, J = 8,0, 1 arom. H); 6,81 (d, J = 8,0, 1 arom. H); 5,07 (d, J = 3,6, H-C(5)); 4,02 (s, C(6)-NH-CH₂-); 2,96 (s, CH₃N); 1,24 (t, J = 7,0, C(14)-OCH₂CH₃); ESI-MS: m/z 389 (M⁺ + 1).

### Beispiel 22

### Synthese von (4,5α-Epoxy-3-hydroxy-14β-ethoxy-17-methylmorphinan-6β-ylamino)-essigsäure Bis(tetrafluoroborat) (Verbindung 27-2 HBF₄)

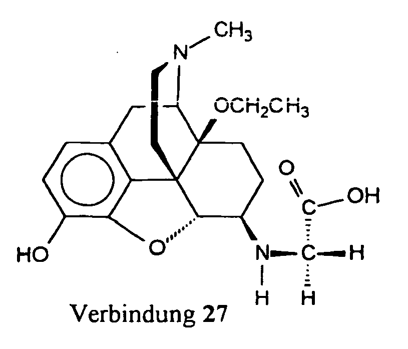

Eine Lösung von Verbindung **25** (0,10 g, 0,22 mmol) in CH₂Cl₂ (6 ml) wurde mit 54% Tetrafluoroborsäure (HBF₄) in Et₂O (0,16 ml) versetzt und das Gemisch 15 min. lang bei Raumtemperatur ultrabeschallt. Der dabei entstandene Niederschlag wurde abfiltriert und getrocknet. Ausbeute 0.09 g (73%) weißes 3.2 HBF₄: Fp > 280°C (Zers.); IR (KBr): 3426 (OH), 1758 (C=O), 1064 (B-F) cm⁻¹; ¹H-NMR (D₂O): δ 6,90 (d, J = 8,0, 1 arom. H); 6,85 (d, J = 8,0, 1 arom. H); 4,92 (d, J = 7,6, H-C(5)); 4,03 (s, C(6)-NH-CH₂-); 2,94 (s, CH₃N); 1,29 (t, J = 6,8, C(14)-OCH₂CH₃); ESI-MS: m/z 389 (M⁺ + 1).

### Beispiel 23

### Synthese von (2'S)-2'-(17-Cyclopropylmethyl-4,5α-epoxy-3,14β-dihydroxymorphinan-6β-ylamino)-3-phenylpropionsäure-tert.-butylester (Verbindung 28).

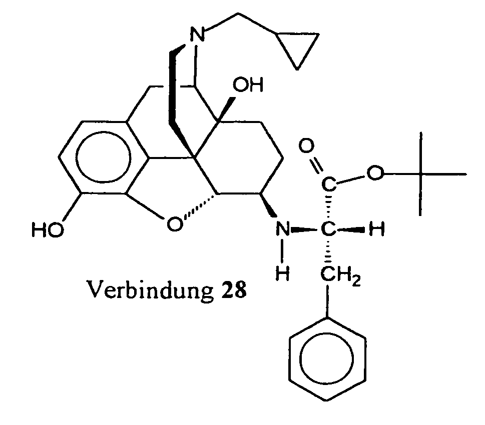

Ein Gemisch von Naltrexon Hydrochlorid (Brit. Patent GB 1119270, 1968) (5,46 g, 13,23 mmol), L-Phenylalanin-tert.-butylester Hydrochlorid (5,46 g, 21,18 mmol), absolutem EtOH (250 ml), N-Ethyldüsopropylamin (6 ml, 43,4 mmol) und Molekularsieb (5 g) wurde 6 h lang unter N₂ bei Raumtemperatur gerührt. Anschließend wurde NaCNBH₃ (0,91 g, 14,48 mmol) zugegeben und die Lösung weiter unter N₂ bei Raumtemperatur gerührt. Nach 6 Tagen wurde H₂O (20 ml) zugefügt, filtriert und das Filtrat eingedampft. Der Rückstand wurde mit H₂O (300 ml) versetzt, mit konz. Ammoniak alkalisiert und mit CH₂Cl₂ (1 x 100 ml, 4 x 50 ml) extrahiert. Die vereinigten organischen Phasen wurden mit H₂O (2 x 200 ml) gewaschen, getrocknet (Na₂SO₄) und eingedampft. Vom Eindampfrückstand (8,44 g) wurden 2 g mittels Zirkularchromatographie (Kieselgel 60, CH₂Cl₂/MeOH/konz. Ammoniak von 250:1:0,1 bis 150:2,5:0,2) gereinigt. Ausbeute 0,33 g (19% bezogen auf das gesamte Rohprodukt) reines 28 als weißes Schaumharz. ¹H-NMR (DMSO-d₆): δ 8,98 (s, OH-C(3)), 7,22 (m, 5 arom. H), 6,56 (d, J = 8,0, 1 arom. H); 6,46 (d, J = 8,0, 1 arom. H); 4,78 (s, OH-C(14)), 4,14 (d, J = 6,0, H-C(5)); 1,20 (s, -COOC(CH₃)₃); 0,84 (m, CH (cyclopropyl)); 0,47 (m, CH₂ (cyclopropyl)); 0,09 (m, CH₂ (cyclopropyl)); CI-MS: m/z 547 (M⁺+ 1).

### Beispiel 24

### Synthese von (2'S)-2'-(17-Cyclopropylmethyl-4,5α-epoxy-3,14β-dihydroxymorphinan-6β-ylamino)-3-phenylpropionsäure Bis(tetrafluoroborat) (Verbindung 29-2 HBF₄).

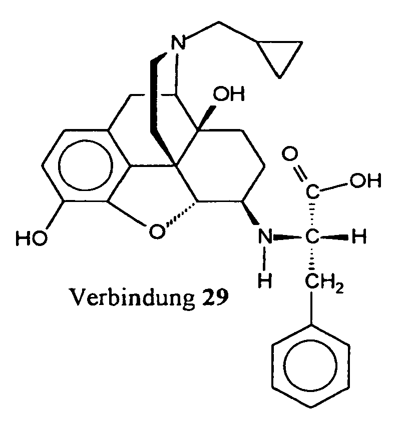

Eine Lösung von Verbindung **28** (0,18 g, 0,33 mmol) in CH₂Cl₂ (5 ml) wurde mit 54% Tetrafluoroborsäure (HBF₄) in Et₂O (0,22 ml) versetzt und das Gemisch 1 h lang bei 0°C gerührt. Der dabei entstandene Niederschlag wurde abfiltriert und getrocknet. Das Rohprodukt wurde durch Zirkularchromatographie (Kieselgel 60, CH₂Cl₂/MeOH von 7:3 bis 3:7, dann MeOH alleine) gereinigt. Ausbeute 0,06 g (27%) reines 29.2 HBF₄ als gelbes Schaumharz. ¹H-NMR (DMSO-d₆): δ 7,17 (m, 5 arom. H), 6,45 (d, J = 8,2, 1 arom. H); 6,35 (d, J = 8,2, 1 arom. H); 4,73 (s, OH-C(14)), 4,08 (d, J = 7,4, H-C(5)); 0,80 (m, CH (cyclopropyl)); 0,44 (m, CH₂ (cyclopropyl)); 0,09 (m, CH₂ (cyclopropyl)); HR-FAB-MS: m/z berechnet für C₂₉H₃₅N₂O₅ (M⁺ + 1): 491,2536, gefunden: 491,2540.

### Beispiel 25

### Synthese von 3-Benzyloxy-17-cyclopropylmethyl-4,5α-epoxy-14β-hydroxy-morphinan-6-spiro-2'-1,3-dioxolan (Verbindung 30).

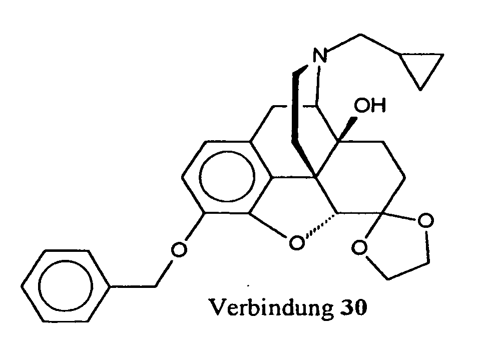

Ein Gemisch, von 17-Cyclopropyl-4,5α-epoxy-3,14β-dihydroxymorphinan-6-spiro-2'-dioxolan (H. Schmidhammer et al. Heterocycles 1998, Vol. 49, S. 489-497) (6,90 g, 17,90 mmol), K₂CO₃ (6,70 g, 48,48 mmol), Benzylbromid (2,34 ml, 19,66 mmol) und absolutem DMF (70 ml), wurde 21 h lang unter N₂ bei Raumtemperatur gerührt. Das anorganische Material wurde abfiltriert, mit CH₂Cl₂ gespült und das Filtrat eingedampft. Der Rückstand (gelblich gefärbte Kristalle) wurde aus MeOH umkristallisiert. Ausbeute 7,37 g (87%) reines 30 als farblose Kristalle. Fp 130-131°C; IR (KBr): 3352 (OH) cm⁻¹; ¹H-NMR (CDCl₃): δ 7,42-7,27 (m, 5 arom. H); 6,75 (d, J = 8,3, 1 arom. H); 6,54 (d, J = 8,3, 1 arom. H); 5,17 (d, J = 11,7, OCH₂Ph), 5,10 (d, J = 11,7, OCH₂Ph), 4,58 (s, H-C(5)); 4,19-3,73 (m, C(6)-O-CH₂-CH₂-O-C(6)); CI-MS: m/z 476 (M⁺ + 1).

### Beispiel 26

### Synthese von 3-Benzyloxy-17-cyclopropylmethyl-4,5α-epoxy-14β-{[(E)-3-phenylprop-2-enyl]oxy}morphinan-6-spiro-2'-1,3-dioxolan Hydrochlorid (Verbindung 31-HCl).

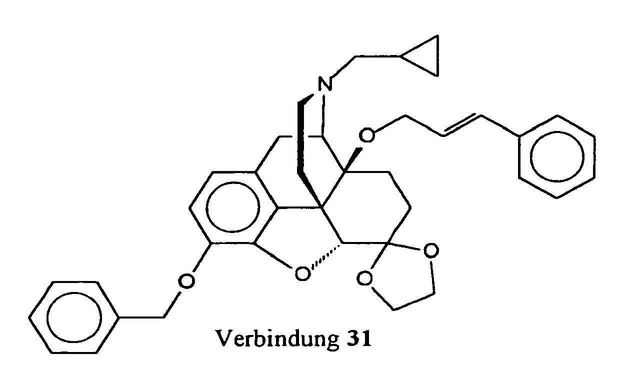

Ein Gemisch von Verbindung **30** (4,00 g, 8,41 mmol), absolutem DMF (50 ml), NaH (0,60 g, 25,23 mmol, aus 1,00 g 60%-iger NaH-Dispersion durch mehrmaliges Waschen mit Petrolether) wurde 20 min. unter N₂ bei 0 °C gerührt. Anschließend wurde eine Lösung von Cinnamylbromid (2,15 g, 10,93 mmol) in DMF (20 ml) zugetropft und das Gemisch 3 h lang bei Raumtemperatur unter N₂ weitergerührt. Nach der Zerstörung von überschüssigem NaH durch vorsichtige Zugabe kleiner Eisstückchen wurde die Mischung auf 400 ml H₂O gegossen und mit CH₂Cl₂ (4 x 75 ml) extrahiert. Die vereinigten organischen Phasen wurden mit H₂O (5 x 300 ml) und gesättigter NaCl-Lösung (1 x 100 ml) gewaschen, getrocknet (Na₂SO₄) und eingedampft. Der Eindampfrückstand (5,25 g oranges Öl) wurde säulenchromatographisch gereinigt (Kieselgel 60, CH₂Cl₂/MeOH/konz. Ammoniak 250:2:0,5). Ausbeute 1,86 g (37%) reines **31**. Für analytische Zwecke wurden 0,2 g in Ether gelöst und **31**·HCl durch Zugabe von etherischer HCl als als ockerfarbenes Pulver gefällt. Fp 133-136°C; ¹H-NMR (DMSO-d₆): δ 8,33 (br s, NH⁺); 7,53-7,24 (m, 10 arom. H); 6,93 (d, J = 8,4, 1 arom. H); 6,72-6,68 (m, 1 arom. H, 2 olef. H); 5,15 (s, OCH₂Ph); 4,65 (s, H-C(5)); 4,31-4,21 (m, C(6)-O-CH₂- CH₂-O-C(6)); 1,09 (m, CH (cyclopropyl)); 0,72-0,44 (m, 2 x CH₂ (cyclopropyl)); CI-MS: m/z 592 (M⁺+1).

### Beispiel 27

### Synthese von 17-Cyclopropylmethyl-4,5α-epoxy-3-hydroxy-14β-[3-(phenylpropyl)oxy]morphinan-6-spiro-2'-1,3-dioxolan Hydrochlorid (Verbindung 32-HCl).

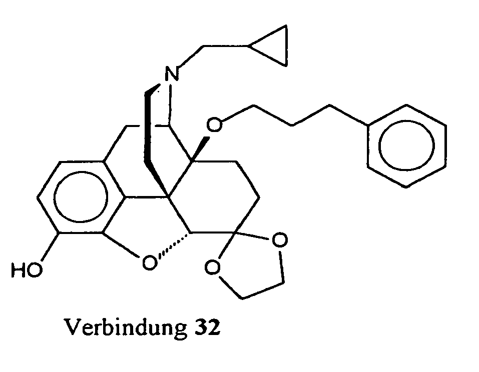

Ein Gemisch von **31** (0,89 g, 1,51 mmol), MeOH (50 ml), THF (15 ml) und 10% Pd/C (90 mg) wurde bei Raumtemperatur und 30 psi 2 h lang hydriert. Anschließend wurde der Katalysator abfiltriert und das Filtrat eingedampft. Der Eindampfrückstand (1,0 g gelbliches Öl) wurde säulenchromatographisch gereinigt (Kieselgel 60, CH₂Cl₂/MeOH/konz. Ammoniak 250:5:0,5). Ausbeute 0,41 g (53%) reines **32**. Für analytische Zwecke wurden 70 mg in Ether gelöst und **32**·HCl durch Zugabe von etherischer HCl als als weißes Pulver gefällt. Fp 158-162°C; ¹H-NMR (DMSO-d₆): δ 9,24 (s, OH), 7,79 (br s, NH⁺); 7,35-7,19 (m, 5 arom. H); 6,68 (d, J = 8,0, 1 arom. H); 6,57 (d, J = 8,0, 1 arom. H); 4,51 (s, H-C(5)); 4,31-4,21 (m, C(6)-O-CH₂- CH₂-O-C(6)); CI-MS: m/z 504 (M⁺ + 1).

### Beispiel 28

### Synthese von 17-Cyclopropylmethyl-4,5α-epoxy-3-hydroxy-14β-[3-(phenylpropyl)oxy]morphinan-6-on Hydrochlorid (Verbindung 33-HCl).

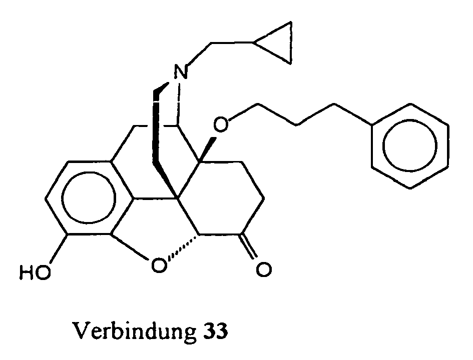

Eine Lösung von **32** (4,00 g, 7,94 mmol) in 28 ml MeOH und 12 ml konz. HCl wurde 1,5 h lang rückflusserhitzt, anschließend auf 100 ml Eis/Wasser gegossen und mit konz. Ammoniak alkalisiert. Das Gemisch wurde mit CH₂Cl₂ (4 x 100 ml) extrahiert, die vereinigten organischen Phasen mit Wasser (2 x 100 ml) und gesättigter NaCl-Lösung (2 x 100 ml) gewaschen, getrocknet (Na₂SO₄) und eingedampft. Der Eindampfrückstand (3,98 g braunes Öl) wurde säulenchromatographisch gereinigt (Kieselgel 60, CH₂Cl₂/MeOH/konz. Ammoniak 250:3:0,5). Ausbeute 3,05 g (83%) reines **33**. Für analytische Zwecke wurden 90 mg in Ether gelöst und **33**·HCl durch Zugabe von etherischer HCl als als farblose Kristalle gefällt. Fp 220-230°C; ¹H-NMR (DMSO-d₆): δ 9,52 (s, OH), 8,20 (s, NH⁺); 7,30-7,18 (m, 5 arom. H); 6,71 (d, J = 8,0, 1 arom. H); 6,64 (d, J = 8,0, 1 arom. H); 4,89 (s, H-C(5)); CI-MS: m/z 460 (M⁺ + 1).

### Beispiel 29

### Synthese von {17-Cyclopropylmethyl-4,5α-epoxy-3-hydroxy-14β-[(3-phenylpropyl)oxy]-morphinan-6α-ylamino}-essigsäure-tert.-butylester (Verbindung 34) und {17-Cydopropylmethyl-4,5α-epoxy-3-hydroxy- 14β-[(3-phenylpropyl)oxy]-morphinan-6β-ylamino}-essigsäure-tert.-butylester (Verbindung 35).

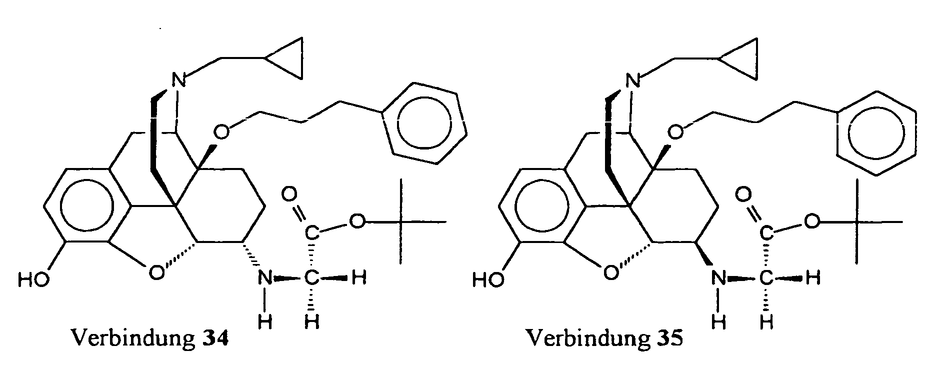

Ein Gemisch von Verbindung **33** (0,7 g, 1,41 mmol), Glycin-tert.-butylester Hydrochlorid (0,26 g, 1,55 mmol), absolutem EtOH (20 ml), Triethylamin (0,49 ml, 3,53 mmol) und Molekularsieb (0,7 g) wurde 23 h lang unter N₂ bei Raumtemperatur gerührt. Anschließend wurde NaCNBH₃ (0,13 g, 2,07 mmol) zugegeben und die Lösung weiter unter N₂ bei Raumtemperatur gerührt. Nach 3 Tagen wurde H₂O (5 ml) zugefügt, filtriert und das Filtrat eingedampft. Der Rückstand wurde mit H₂O (20 ml) versetzt, mit konz. Ammoniak alkalisiert und mit CH₂Cl₂ (1 x 50 ml, 3 x 30 ml) extrahiert. Die vereinigten organischen Phasen wurden mit gesättigter NaCl-Lösung (3 x 200 ml) gewaschen, getrocknet (Na₂SO₄)und eingedampft. Der Eindampfrückstand (0,62 g braunes Öl) wurde säulenchromatographisch getrennt und gereinigt (Kieselgel 60, CH₂Cl₂/MeOH/konz Ammoniak 250:2:0,5).
Verbindung **34**: Ausbeute 70 mg (9%). ¹H-NMR (CDCl₃): δ 7,28-7,16 (m, 5 arom. H), 6,67 (d, J = 8,1, 1 arom. H); 6,45 (d, J = 8,1, 1 arom. H); 4,70 (d, J = 3,4, H-C(5)); 1,44 (s, -COOC(CH₃)₃); CI-MS: m/z 575 (M⁺ + 1).
Verbindung **35**: Ausbeute 40 mg (5%). ¹H-NMR (DMSO-d₆): δ 8,96 (s, OH); 7,31-7,16 (m, 5 arom. H), 6,54 (d, J = 8,3, 1 arom. H); 6,45 (d, J = 8,3, 1 arom. H); 4,25 (d, J = 6,6, H-C(5)); 1,39 (s, -COOC(CH₃)₃); CI-MS: m/z 575 (M⁺ + 1).

### Beispiel 30

### Synthese von (2'S)-2'-(17-Cyclopropylmethyl-4,5α-epoxy-3-hydroxy-14β-[(3-phenylpropyl)oxy]-morphinan-6α-ylamino)-3-phenylpropionsäure-tert.-butylester (Verbindung 36) und (2'S)-2'-(17-Cyclopropylmethyl-4,5α-epoxy-3-hydroxy-14β-[(3-phenylpropyl)oxy]-morphinan-6β-ylamino)-3-phenylpropionsäure-tert.-butylester (Verbindung 37).

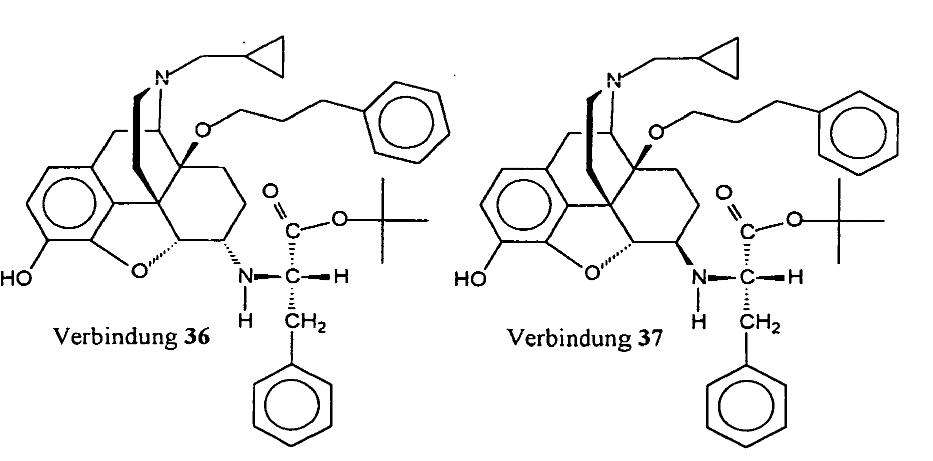

Ein Gemisch von Verbindung **33** (0,7 g, 1,41 mmol), L-Phenylalanin-tert.-butylester Hydrochlorid (0,55 g, 2,13 mmol), absolutem EtOH (20 ml), Triethylamin (0,49 ml, 3,53 mmol) und Molekularsieb (0,7 g) wurde 23 h lang unter N₂ bei Raumtemperatur gerührt. Anschließend wurde NaCNBH₃ (0,13 g, 2,07 mmol) zugegeben und die Lösung weiter unter N₂ bei Raumtemperatur gerührt. Nach 4 Tagen wurde H₂O (5 ml) zugefügt, filtriert und das Filtrat eingedampft. Der Rückstand wurde mit H₂O (20 ml) versetzt, mit konz. Ammoniak alkalisiert und mit CH₂Cl₂ (1 x 50 ml, 3 x 30 ml) extrahiert. Die vereinigten organischen Phasen wurden mit gesättigter NaCl-Lösung (3 x 100 ml) gewaschen, getrocknet (Na₂SO₄) und eingedampft. Der Eindampfrückstand (braunes Öl) wurde säulenchromatographisch getrennt und gereinigt (Kieselgel 60, CH₂Cl₂/MeOH/konz. Ammoniak 250:2:0,5). Verbindung **36**: Ausbeute 70 mg (7%). ¹H-NMR (DMSO-d₆): δ 8,90 (s, OH); 7,31-7,12 (m, 5 arom. H), 6,56 (d, J = 8,2, 1 arom. H); 6,40 (d, J = 8,2, 1 arom. H); 4,52 (d, J = 3,6, H-C(5)); 1,22 (s, -COOC(CH₃)₃); CI-MS: m/z 665 (M⁺ + 1).
Verbindung **37**: Ausbeute 0,33 g (35%). ¹H-NMR (DMSO-d₆): δ 8,98 (s, OH); 7,27-7,13 (m, 5 arom. H), 6,54 (d, J = 8,0, 1 arom. H); 6,44 (d, J = 8,0, 1 arom. H); 4,21 (d, J = 7,0, H-C(5)); 1,20 (s, -COOC(CH₃)₃); CI-MS: m/z 665 (M⁺ + 1).

### Beispiel 31

### Synthese von {17-Cyclopropylmethyl-4,5α-epoxy-3-hydroxy-14β-[(3-phenylpropyl)oxy]-morphinan-6β-ylamino}-essigsäure Dihydrochlorid (Verbindung 38-2 HCl).

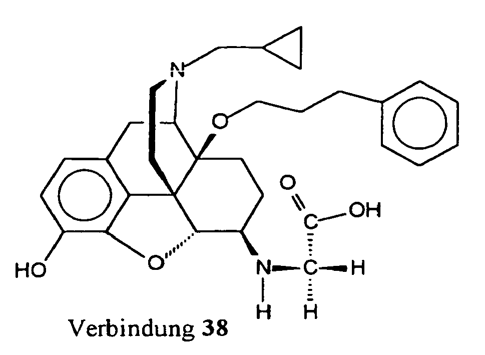

Ein Gemisch von Verbindung **35** (40 mg, 0,07 mmol) und 4 M HCl in Dioxan (2 ml) wurde 6 h lang rückflusserhitzt. Der Niederschlag wurde abfiltriert und getrocknet. Ausbeute 10 mg (24%) weißes 38.2 HCl: ¹H-NMR (D₂O): δ 7,38-7,26 (m, 5 arom. H), 6,89 (d, J = 8,0, 1 arom. H); 6,83 (d, J = 8,0, 1 arom. H); 4,91 (d, J = 7,4, H-C(5)); 4,00 (s, C(6)-NH-CH₂-).

### Beispiel 32

### Opioid-Rezeptorbindungsstudien

Opioid-Rezeptorbindungsstudien wurden an Rattenhirn-Homogenisaten unter Verwendung von [³H]DAMGO (µ-Rezeptor-Agonist) als Radioligand und unter strikter Einhaltung einer früher publizierten Vorschrift durchgeführt (M. Spetea et al. Neurochemical Research 1998, Vol. 23, S. 1213-1218).

Die Verbindung **1-4, 7, 8, 11, 16, 17, 22, 23 und 38** zeigen sehr hohe Affinität zu µ-Opioidrezeptoren, welche hauptsächlich verantwortlich sind für Analgesie (Tabelle 1). Morphin zeigt im Vergleich dazu deutlich geringere Affinität zu µ-Opioidrezeptoren.

**Tabelle 1. Opioid-Rezeptorbindungsstudien der Verbindungen 1-4, 7, 8, 11, 16, 17, 22, 23, 38 und Morphin**

| Verbindung | Kᵢ (nM) (µ-Rezeptoren) |
|---|---|
| **1** | 0,48 |
| **2** | 1,30 |
| **3** | 0,90 |
| **4** | 0,83 |
| **7** | 0,77 |
| **8** | 1,90 |
| **11** | 0,95 |
| **16** | 0,61 |
| **17** | 0,25 |
| 22 | 0,21 |
| 23 | 0,80 |
| 38 | 0,24 |
| Morphin | 6,55 |

### Beispiel 33

### Analgesietest

Es wurde ein Test an Ratten ("rat tail flick test") verwendet. Dieser Test wurde wie früher beschrieben durchgeführt (Zs. Fürst et al. Eur. J. Pharmacol. 1993, Vol. 236, S. 209-215).

Die Verbindung **3, 4, 7** und **8** zeigen sehr hohe analgetische Aktivität. So ist Verbindung **4** 68mal aktiver als Morphin nach subcutaner Applikation (sc) und 238mal aktiver als Morphin nach intracerebroventrikulärer Applikation (icv) (Tabelle 2). Die hohen sc/icv-Verhältniszahlen der Verbindungen **3, 4, 7 und 8** zeigen, dass diese Verbindungen im Vergleich zu Morphin bevorzugt in die Peripherie verteilt werden und auch bevorzugt in der Peripherie ihre analgetische Wirkung entfalten. Das heißt, aus diesen Zahlen geht hervor, dass Verbindung **3** nur sehr begrenzt die Blut-Hirn-Schranke überwinden kann und seine Wirksamkeit daher in erster Linie in der Peripherie (außerhalb des Zentralnervensystems) zeigt. Das geht einher mit einer sehr verringerten Nebenwirkungsrate, was zentrale Nebenwirkungen wie z.B. Übelkeit, Erbrechen, Sedation, Benommenheit, Verwirrtheit, Atemdepression und Sucht betrifft.

Die Verbindungen **3** und **4** zeigen eine wesentlich längere analgetische Wirkungsdauer als Morphin. Während die Verbindungen **3** (0,5 µg/Ratte) und **4** (0,25 µg/Ratte) noch nach 120 Minuten 100% analgetische Aktivität zeigen, ist die Aktivität von Morphin (50 µg/Ratte) nach 120 Minuten auf 20% abgesunken (Tabelle 3). Die Verbindungen **3** und **4** haben nach 180 Minuten noch 90% der analgetischen Aktivität.

**Tabelle 2. "Rat tail flick test" der Verbindungen 3, 4, 7, 8 und Morphin**

| Verbindung | ED₅₀ (µg/kg, sc^{a}) | ED₅₀ (µg/kg, icv^{b}) | sc/icv |
|---|---|---|---|
| **3** | 86 | 0,49 | 176 |
| **4** | 28 | 0,42 | 67 |
| **7** | 100 | 0,75 | 133 |
| **8** | 500 | 0,67 | 746 |
| Morphin | 1900 | 100 | 19 |

| | | | |
|---|---|---|---|
| ^{a} sc = subcutane Applikation. ^{b} icv = intracerebroventrikuläre Applikation. | | | |

**Tabelle 3. Analgetische Wirkung (in Prozent) der Verbindungen 3, 4 und Morphin nach 10, 20, 30, 60, 120 und 180 Minuten im "Rat tail flick test" nach intracerebroventrikulärer Applikation**

| Verbindung (µg/Ratte^{a}) | 10 | 20 | 30 | 60 | 120 | 180 Minuten |
|---|---|---|---|---|---|---|
| **3**(0,5) | 46 | 62 | 59 | 100 | 100 | 90 |
| **4**(0,25) | 61 | 74 | 76 | 100 | 100 | 90 |
| Morphin (50) | 57 | 91 | 74 | 59 | 20 | NB^{b} |

| | | | | | | |
|---|---|---|---|---|---|---|
| ^{a} Das Gewicht der verwendeten Ratten war jeweils 120 g. ^{b} NB = nicht bestimmt. | | | | | | |

### Beispiel 34

### Randall-Selitto-Test

Der Randall-Selitto-Test (L. O. Randall und J. J. Selitto, Arch. Int. Pharmacodyn 1957, Vol. 111, S. 409-419) wurde verwendet, um den analgetischen Effekt auf Carrageen-induzierte Hyperalgesie an der rechten Hinterpfote von Ratten zu studieren. Es wird dabei die "hindpaw withdrawal latency" (HWL; Latenzzeit des Zurückziehens der rechten Hinterpfote) bei mechanischer Stimulation gemessen (z.B. I. Bileviciute-Ljungar und M. Spetea, Br. J. Pharmacol. 2001, Vol. 132, S. 252-258).

Die Verbindungen **4** und die zentral wirksame Referenzsubstanz 14-Methoxymetopon (Zs. Fürst et al. Eur. J. Pharmacol. 1993, Vol. 236, S. 209-215) zeigen beide signifikante analgetische Aktivität bei einer Dosis von je 20 µg/kg nach subcutaner Applikation (als signifikante analgetische Aktivität wird ein mindestens 100%iger Anstieg der Latenzzeit des Zurückziehens der Hinterpfote (HWL) bezeichnet). Während Verbindung **4** einen lang anhaltenden Effekt (ca. 14 Stunden) zeigt, ist der Effekt von 14-Methoxymetopon mit 2 Stunden wesentlich kürzer.

Der ausschließlich periphere analgetische Effekt der Verbindung **4** wurde durch folgenden Versuch bewiesen. Der analgetische Effekt der Verbindung **4** (20 µg/kg) wurde durch subcutane Applikation des selektiv peripher wirkenden Opioidantagonisten Naloxonmethiodid (ein Äquivalent) vollständig aufgehoben, während der analgetische Effekt des 14-Methoxymetopons (20 µg/kg) durch subcutane Applikation des selektiv peripher wirkenden Opioidantagonisten Naloxonmethiodid (ein Äquivalent) nicht beeinflusst wurde. Das ist der Beweis, dass der analgetische Effekt der Verbindung **4** über periphere Opioidrezeptoren vermittelt wird, während der analgetische Effekt des 14-Methoxymetopons über Opioidrezeptoren des Zentralnervensystems vermittelt wird. Das lässt den Schluss zu, dass Verbindung **4** die Blut-Hirn-Schranke nicht überwinden kann und daher auch keine über das Zentralnervensystem vermittelte Nebenwirkungen (z.B. Atemdepression, Benommenheit, Verwirrtheit, Sedation, Schläfrigkeit, Sucht) zeigen kann.

### Beispiel 35

### Atemdepressions-Test in Ratten

In diesem Test wird das Atemvolumen und die Frequenz der Atmung von anästhesierten Ratten gemessen.

Die Verbindungen **3** und **4** wurden auf ihre Eigenschaft, eine Atemdepression hervorrufen zu können, untersucht (s. Tabelle 4). Bei subcutaner Applikation (sc) der Verbindungen **3** und **4** sind sehr hohe Dosen notwendig, um eine Atemdepression hervorzurufen. Da bei intracerebroventrikulärer Applikation (icv) eine Atemdepression in wesentlich niedrigerer Dosierung hervorgerufen wird, resultieren für Verbindungen **3** und **4** sehr hohe sc/icv-Verhältniszahlen (1205 bzw. 1190). Die sc/icv-Verhältniszahlen für Morphin und Fentanyl sind deutlich niedriger (34 bzw. 0,2).

Während die analgetischen ED₅₀-Werte ("Rat tail flick test") den ED₅₀-Werten des Atemdepressions-Tests nach intracerebroventrikulärer Applikation sehr ähnlich sind, sind diese Werte nach subcutaner Applikation sehr verschieden. Vergleicht man die hohen ED₅₀-Werte des Atemdepressions-Tests nach subcutaner Applikation von **3** und **4** mit den niedrigen ED₅₀-Werten des "Rat tail flick tests" nach subkutaner Applikation, so ist zu erkennen, dass für Verbindung **3** eine 12-fach höhere Dosis als die analgetische Dosis notwendig ist, um eine Atemdepression auszulösen. Im Falle der Verbindung **4** ist eine 18-fach höhere Dosis notwendig. Analgetische Dosen der Verbindungen **3** und **4** sind also nicht in der Lage eine Atemdepression auszulösen.

**Tabelle 4. "Atemdepressions-Test in Ratten" der Verbindungen 3, 4, Morphin und Fentanyl**

| Verbindung | ED₅₀ (µg/kg, sc^{a}) | ED₅₀ (µg/kg, icv^{b}) | sc/icv |
|---|---|---|---|
| **3** | 1000 | 0,83 | 1205 |
| **4** | 500 | 0,42 | 1190 |
| Fentanyl | 25 | 125 | 0,2 |
| Morphin | 2800 | 83 | 34 |

| | | | |
|---|---|---|---|
| ^{a} sc = subcutane Applikation. ^{b} icv = intracerebroventrikuläre Applikation. | | | |

### Beispiel 36

### Bestimmung des antiallodynischen Effekts

Es wurden zwei Tests an Ratten durchgeführt:
a) Von Frey Test (mechanische Allodynie)
b) Kalt-Wasser Allodynie Test (thermale Allodynie)
Bei diesen Tests handelt es sich um Modelle, um den Effekt einer Substanz auf neuropathische Schmerzen zu prüfen.
Für beide Tests wurden Ligationen um den Ischiasnerv der Tiere angebracht (G. J. Bennett und Y. K. Xie, Pain 1988, Vol. 33, 87-107).

### a) Von Frey Test (mechanische Allodynie)

Auf die Haut der Hinterpfote der Ratten (200-350 g) wurde mit von Frey Haaren ein leichter Druck (2 bis 60 g) ausgeübt und in verschiedenen Zeitabständen (5, 15, 30 und 60 min nach Applikation der Substanz) die Latenzzeit des Zurückziehens der Hinterpfote studiert. Die zu untersuchende Substanz wurde intraplantar (ipl) appliziert.

Die Verbindungen **4** zeigt signifikante antiallodynische Aktivität bei einer Dosis von 100 □g/Ratte nach subcutaner Applikation (als signifikante antiallodynische Aktivität wird ein mindestens 100%iger Anstieg der Latenzzeit des Zurückziehens der Hinterpfote bezeichnet). Der peripher wirkende Opioidantagonist Naloxonmethiodid (ipl) war in der Lage den antiallodynischen Effekt von Verbindung **4** vollständig aufzuheben.

### b) Kalt-Wasser Allodynie Test (thermale Allodynie)

Thermale Stimulierung (kaltes Wasser) wurde verwendet, um ein Zurückziehen der Hinterpfote der Ratten (200-300 g) auszulösen. Nach 5, 15 und 30 min (nach Applikation der Substanz) wurde die Latenzzeit des Zurückziehens der Hinterpfote studiert (J. C. Hunter et al., Eur. J. Pharmacol., 1997, Vol. 324, 153-160). Die zu untersuchenden Substanzen wurden intraplantar (ipl) appliziert.

Die Verbindungen **4** zeigt signifikante antiallodynische Aktivität bei einer Dosis von 100 µg/Ratte nach subcutaner Applikation (als signifikante antiallodynische Aktivität wird ein mindestens 100%iger Anstieg der Latenzzeit des Zurückziehens der Hinterpfote bezeichnet). Der peripher wirkende Opioidantagonist Naloxonmethiodid (ipl) war in der Lage den antiallodynischen Effekt von Verbindung **4** vollständig aufzuheben.

## Patentansprüche

1. Verbindungen der Formel (I), 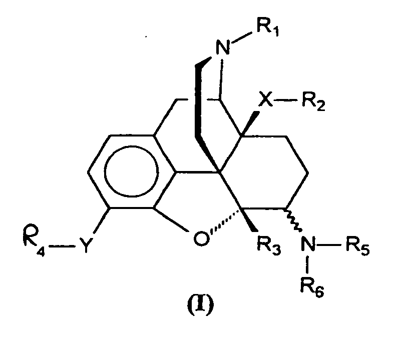 in der die Substituenten die folgende Bedeutung haben:
R₁ ist Wasserstoff; C₁-C₆-Alkyl; C₂-C₆-Alkenyl; C₂-C₆-Alkinyl; C₁-C₆-Monohydroxyalkyl; C₂-C₆-Dihydroxyalkyl; C₃-C₆-Trihydroxyalkyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl;
der mit R₁ verbundene Stickstoff kann zusätzlich auch quarternisiert sein durch zwei Substituenten R₁, die gleich oder verschieden sein können und die wie zuvor dargestellt definiert sind und wobei der zweite, quaternisierende Substituent zusätzlich die Bedeutung Hydroxyl, Oxyl (N-Oxid) sowie Alkoxyl haben kann;
R₂ ist C₁-C₆-Alkyl; C₁-C₆-Monohydroxyalkyl; C₂-C₆-Dihydroxyalkyl; C₃-C₆-Trihydroxyalkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₂-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist;
R₃ ist Wasserstoff; C₁-C₆-Alkyl; C₂-C₆-Alkenyl; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; Alkoxyalkyl, worin Alkoxy C₁-C₆-Alkoxy und Alkyl C₁-C₆-Alkyl ist; CO₂(C₁-C₆-Alkyl); CO₂H, CH₂OH.
R₄, vorbehaltlich der Definition für Y, ist Wasserstoff; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; C₂-C₆-Alkanoyl; C₃-C₆-Alkenoyl; C₃-C₆-Alkinoyl; C₇-C₁₆-Arylalkanoyl, worin Aryl C₆-C₁₀-Aryl und Alkanoyl C₁-C₆-Alkanoyl; C₉-C₁₆-Arylalkenoyl, worin Aryl C₆-C₁₀-Aryl ist und Alkenoyl C₃-C₆-Alkenoyl ist; C₉-C₁₆-Arylalkinoyl, worin Aryl C₆-C₁₀-Aryl ist und Alkinoyl C₃-C₆-Alkinoyl ist; Iminomethyl, Formamidinyl, C₁-C₆-N-Alkyl- und N,N'-Dialkylformamidinyl; C₂-C₆-N-Alkenyl- und N,N'-Dialkenylformamidinyl; C₂-C₆-N-Alkinyl- und N,N'-Dialkinylformamidinyl; C₄-C₁₆-N-Cycloalkylalkyl- und N,N'-Dicycloalkylalkylformamidinyl, worin Cycloalkyl C₃-C₁₀-Cycloalkyl ist und Alkyl C₁-C₆-Alkyl ist; C₅-C₁₆-N-Cycloalkylalkenyl- und N,N'-Dicycloalkylalkenylformamidinyl, worin Cycloalkyl C₃-C₁₀-Cycloalkyl ist und Alkenyl C₂-C₆-Alkenyl ist; C₅-C₁₆-N-Cycloalkylalkinyl- und N,N'-Dicycloalkylalkinylformamidinyl, worin Cycloalkyl C₃-C₁₀-Cycloalkyl ist und Alkinyl C₂-C₆-Alkinyl ist; C₇-C₁₆-N-Arytalkyl- und N,N'-Diarylalkylformamidinyl, worin Aryl C₆-C₁₀-Aryl ist und Alkyl C₁-C₆-Alkyl ist..
R₅ und R₆, die gleich oder verschieden sein können, sind Wasserstoff; weiterhin CH(A)CO₂B, worin A Wasserstoff; Hydroxyl; C₁-C₆-Alkyl; C₂-C₆-Alkenyl; C₂-C₆-Alkinyl; C₄-C₁₆-Cycloalkylalkyl, worin Cycloalkyl C₃-C₁₀-Cycloalkyl ist und Alkyl C₁-C₆-Alkylist; C₅-C₁₆-Cycloalkylalkenyl, worin Cycloalkyl C₃-C₁₀-Cycloalkyl ist und Alkenyl C₂-C₆-Alkenylist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl; Amino; C1-C6-Alkylamino; Guanidino; C₁-C₆-Alkyl-CO₂B ist; und worin B Wasserstoff; C₁-C₃₀-, bevorzugt C₁-C₆-Alkyl; C₂-C₃₀-, bevorzugt C₂-C₆-Alkenyl; C₂-C₃₀-, bevorzugt C₂-C₆-Alkinyl; C₄-C₁₆-Cycloalkylalkyl, worin Cycloalkyl C₃-C₁₀-Cycloalkyl ist und Alkyl C₁-C₆-Alkyl ist; C₅-C₁₆-Cycloalkylalkenyl, worin Cycloalkyl C₃-C₁₀-Cycloalkyl ist und Alkenyl C₂-C₆-Alkenyl ist; C₅-C₁₆-Cycloalkylalkinyl, worin Cycloalkyl C₃-C₁₀-Cycloalkyl ist und Alkinyl C₂-C₆-Alkinyl ist; C₇-C₁₆-Arylalkyl, worin Aryl C₆-C₁₀-Aryl ist und Alkyl C₁-C₆-Alkyl ist; C₈-C₁₆-Arylalkenyl, worin Aryl C₆-C₁₀-Aryl und Alkenyl C₂-C₆-Alkenyl ist; C₈-C₁₆-Arylalkinyl, worin Aryl C₆-C₁₀-Aryl ist und Alkinyl C₂-C₆-Alkinyl ist; Phenyl; substituiertes Phenyl; CH₂OCO-C₁-C₆-Alkyl; CH(C₁-C₆-Alkyl)OCO-C₁-C₆-Alkyl; CH₂OCOO-C₁-C₆-Alkyl; CH(C₁-C₆-Alkyl)OCOO-C₁-C₆-Alkyl; CH₂CON(C₁-C₆-Alkyl)₂; CH(C₁-C₆-Alkyl)CON(C₁-C₆-Alkyl)₂, Phthalidyl, (5-Methyl-2-oxo-1,3-dioxol-4-yl)methyl; und weiterhin Iminomethyl, Formamidinyl, C₁-C₆-N-Alkyl- und N,N'-Dialkylformamidinyl; C₂-C₆-N-Alkenyl- und N,N'-Dialkenylformamidinyl; C₂-C₆-N-Alkinyl- und N,N'-Dialkinylformamidinyl; C₄-C₁₆-N-Cycloalkylalkyl- und N,N'-Dicycloalkylalkylformamidinyl, worin Cycloalkyl C₃-C₁₀-Cycloalkyl ist und Alkyl C₁-C₆-Alkyl ist; C₅-C₁₆-N-Cycloalkylalkenyl- und N,N'-Dicycloalkylalkenylformamidinyl, worin Cycloalkyl C₃-C₁₀-Cycloalkyl ist und Alkenyl C₂-C₆-Alkenyl ist; C₅-C₁₆-N-Cycloalkylalkinyl- und N,N'-Dicycloalkylalkinylformamidinyl, worin Cycloalkyl C₃-C₁₀-Cycloalkyl ist und Alkinyl C₂-C₆-Alkinyl ist; C₇-C₁₆-N-Arylalkyl- und N,N'-Diarylalkylformamidinyl, worin Aryl C₆-C₁₀-Aryl ist und Alkyl C₁-C₆-Alkyl ist; C₈-C₁₆-N-Arylalkenyl- und N,N'-Diarylalkenylformamidinyl, worin Aryl C₆-C₁₀-Aryl und Alkenyl C₂-C₆-Alkenyl ist; C₈-C₁₆-N-Arylalkinyl- und N,N'-Diarylalkinylformamidinyl, worin Aryl C₆-C₁₀-Aryl ist und Alkinyl C₂-C₆-Alkinyl; C₂-C₇-N-Alkyloxycarbonyl- und N,N'-Bis(alkyloxycarbonyl)formamidinyl; C₃-C₈-N-Alkenyloxycarbonyl- und N,N'-Bis(alkenyloxycarbonyl)formamidinyl; C₃-C₈-N-Alkinyloxycarbonyl- und N,N'-Bis(alkinyloxycarbonyl)formamidinyl; C₈-C₁₇-N-Arylalkyloxycarbonyl- und N,N'-Bis(arylalkyloxycarbonyl)formamidinyl, worin Aryl C₆-C₁₀-Aryl ist und Alkyloxy C₁-C₆-Alkyloxy; C₉-C₁₇-N-Arylalkenyloxycarbonyl- und N,N'-Bis(arylalkenyloxycarbonyl)formamidinyl, worin Aryl C₆-C₁₀-Aryl ist und Alkenyloxy C₂-C₆-Alkenyloxy ist; C₉-C₁₇-N-Arylalkinyloxycarbonyl- und N,N'-Bis(arylalkinyloxycarbonyl)formamidinyl, worin Aryl C₆-C₁₀-Aryl ist und Alkinyloxy C₂-C₆-Alkinyloxy ist; C₁-C₆-N-Alkanoyl- und N,N'-Dialkanoylformamidinyl; C₃-C₆-N-Alkenoyl- und N,N'-Dialkenoylformamidinyl; C₃-C₆-N-Alkinoyl- und N,N'-Dialkinoylformamidinyl; C₇-C₁₆-N-Arylalkanoyl- und N,N'-Diarylalkanoylformamidinyl, worin Aryl C₆-C₁₀-Aryl und Alkanoyl C₁-C₆-Alkyl; C₉-C₁₆-N-Arylalkenoyl- und N,N'-Diarylalkenoylformamidinyl, worin Aryl C₆-C₁₀-Aryl ist und Alkenoyl C₃-C₆-Alkenoyl ist; C₉-C₁₆-N-Arylalkinoyl- und N,N'-Diarylalkinoylformamidinyl, worin Aryl C₆-C₁₀-Aryl ist und Alkinoyl C₃-C₆-Alkinoyl ist; 4,5-Dihydro-1*H*-imidazol-2-yl, 1,4,5,6-Tetrahydropyrimidin-2-yl, 4,5,6,7-Tetrahydro-1H-[1,3]diazepin-2-yl;
X ist Sauerstoff, und
Y ist Sauerstoff oder die Gruppierung (Y-R₄) ist H;
sowie pharmazeutisch akzeptable Säureadditionssalze und leicht zugängliche Derivate (z.B. Ester oder Amide der Aminosäurederivate) davon.

2. Verbindungen des Anspruches 1, in denen R₁ C₁-C₆ Alkyl ist; R₂ C₁-C₆ Alkyl oder C₇-C₁₆-Arylalkyl ist, worin Aryl C₆-C₁₀-Aryl ist und Alkyl C₁-C₆-Alkyl ist; R₃, R₄ und R₆ Wasserstoff sind; R₅ ist CH(A)CO₂B, worin A Wasserstoff ist; Hydroxyl; C₁-C₆-Alkyl; C₇-C₁₆-Arylalkyl, worin Aryl C₆-C₁₀-Aryl ist und Alkyl C₁-C₆-Alkyl ist; Amino; oder Guanidino; B Wasserstoff oder C₁-C₆-Alkyl ist; R₅ kann weiterhin Formamidinyl; C₂-C₇-(Alkyloxycarbonyl)formamidinyl; C₃-C₈-(Alkenyloxycarbonyl)formamidinyl; C₃-C₈-(Alkinyloxycarbonyl)formamidinyl; C₈-C₁₇-(Arylalkyloxycarbonyl)formamidinyl, worin Aryl C₆-C₁₀-Aryl ist und Alkyloxy C₁-C₆-Alkyloxy; C₉-C₁₇-(Arylalkenyloxycarbonyl)formamidinyl, worin Aryl C₆-C₁₀-Aryl ist und Alkenyloxy C₂-C₆-Alkenyloxy ist; C₉-C₁₇-(Arylalkinyloxycarbonyl)formamidinyl, worin Aryl C₆-C₁₀-Aryl ist und Alkinyloxy C₂-C₆-Alkinyloxy ist, sein.

3. Verbindungen des Anspruches 1, ausgewählt unter:
(4,5α-Epoxy-3-hydroxy-14β-methoxy-17-methylmorphinan-6α-ylamino)-essigsäure-tert.-butylester, (4,5α-Epoxy-3-hydroxy-14β-methoxy-17-methylmorphinan-6β-ylamino)-essigsäure-tert.-butylester, (4,5α-Epoxy-3-hydroxy-14β-methoxy-17-methylmorphinan-6α-ylamino)-essigsäure, (4,5α-Epoxy-3-hydroxy-14β-methoxy-17-methylmorphinan-6β-ylamino)-essigsäure, (2'S)-2'-(4,5α-Epoxy-3-hydroxy-14β-methoxy-17-methylmorphinan-6α-ylamino)-propionsäure-tert.-butylester, (2'S)-2'-(4,5α-Epoxy-3-hydroxy-14β-methoxy-17-methylmorphinan-6β-ylamino)-propionsäure-tert.-butylester, (2'S)-2'-(4,5α-Epoxy-3-hydroxy-14β-methoxy-17-methylmorphinan-6α-ylamino)-propionsäure, (2'S)-2'-(4,5α-Epoxy-3-hydroxy-14β-methoxy-17-methylmorphinan-6β-ylamino)-propionsäure, (2'S)-2'-(4,5α-Epoxy-3-hydroxy-14β-methoxy-17-methylmorphinan-6α-ylamino)-3'-phenylpropionsäure-tert.-butylester, (2'S)-2'-(4,5α-Epoxy-3-hydroxy-14β-methoxy-17-methylmorphinan-6β-ylamino)-3'-phenylpropionsäure-tert.-butylester, (2'S)-2'-(4,5α-Epoxy-3-hydroxy-14β-methoxy-17-methylmorphinan-6α-ylamino)-3'-phenylpropionsäure, (2'S)-2'-(4,5α-Epoxy-3-hydroxy-14β-methoxy-17-methylmorphinan-6β-ylamino)-3'-phenylpropionsäure, 6α-Amino-4,5α-epoxy- 14β-methoxy- 17-methylmorphinan-3-ol, 6β-Dibenzylamino-4,5α-epoxy-14β-methoxy-17-methylmorphinan-3-ol, 6β-Amino-4,5α-epoxy-14β-methoxy-17-methylmorphinan-3-ol, 4,5α-Epoxy-6β-[N,N'-bis-(tert.-butoxycarbonyl)guanidinyl]-14β-methoxy-17-methylmorphinan-3-ol, 4,5α-Epoxy-6β-guanidinyl-14β-methoxy-17-methylmorphinan-3-ol, 4,5α-Epoxy-6α-[N,N'-bis-(tert.-butoxycarbonyl)guanidinyl]-14β-methoxy-17-methylmorphinan-3-ol, 4,5α-Epoxy-6α-guanidinyl-14β-methoxy-17-methylmorphinan-3-ol, 1,3-Bis-(tert.-butoxycarbonyl)-2-{4,5α-Epoxy-6β-[N,N'-bis-(tert.-butoxycarbonyl)guanidinyl]-14β-methoxy-17-methylmorphinan-3-yl}-isoharnstoff, 1,3-Bis-(tert.-butoxycarbonyl)-2-{4,5α-Epoxy-6α-[N,N'-bis-(tert.-butoxycarbonyl)guanidinyl]-14β-methoxy-17-methylmorphinan-3-yl}-isoharnstoff, (4,5α-Epoxy-3-hydroxy-14β-methoxy-17-methylmorphinan-6α-ylamino)-essigsäureethylester Diydrochlorid, (4,5α-Epoxy-3-hydroxy-14β-methoxy-17-methylmorphinan-6β-ylamino)-essigsäureethylester Dihydrochlorid, (4,5α-Epoxy-3-hydroxy-14β-ethoxy-17-methylmorphinan-6α-ylamino)-essigsäure-tert.-butylester, (4,5α-Epoxy-3-hydroxy-14β-ethoxy-17-methylmorphinan-6β-ylamino)-essigsäure-tert.-butylester, (4,5α-Epoxy-3-hydroxy-14β-ethoxy-17-methylmorphinan-6α-ylamino)-essigsäure Bis(tetrafluoroborat), (4,5α-Epoxy-3-hydroxy-14β-ethoxy-17-methylmorphinan-6β-ylamino)-essigsäure Bis(tetrafluoroborat), (2'S)-2'-(17-Cyclopropylmethyl-4,5α-epoxy-3,14β-dihydroxymorphinan-6β-ylamino)-3-phenylpropionsäure-tert.-butylester, (2'S)-2'-(17-Cyclopropylmethyl-4,5α-epoxy-3,14β-dihydroxymorphinan-6β-ylamino)-3-phenylpropionsäure Bis(tetrafluoroborat), {17-Cyclopropylmethyl-4,5α-epoxy-3-hydroxy-14β-[(3-phenylpropyl)oxy]-morphinan-6α-ylamino}-essigsäure-tert.-butylester, {17-Cyclopropylmethyl-4,5α-epoxy-3-hydroxy-14β-[(3-phenylpropyl)oxy]-morphinan-6α-ylamino}-essigsäure-tert.-butylester, (2'S)-2'-(17-Cyclopropylmethyl-4,5α-epoxy-3-hydroxy-14β-[(3-phenylpropyl)oxy]-morphinan-6α-ylamino)-3-phenylpropionsäure-tert.-butylester, {17-Cyclopropylmethyl-4,5α-epoxy-3-hydroxy-14β-[(3-phenylpropyl)oxy]-morphinan-6β-ylamino}-essigsäure Dihydrochlorid; oder irgendein pharmazeutisch akzeptables Salz bzw. ein leicht zugängliches Derivat davon.

4. Zusammensetzung, umfassend eine Verbindung der Ansprüche 1 bis 3 und/oder ein pharmazeutisch akzeptables Säureadditionssalz davon, zusammen mit einem pharmazeutisch akzeptablen Trägerstoff.

5. Verbindung nach irgendeinem der Ansprüche 1 bis 3 als Medikament.

6. Verwendung einer Verbindung der Ansprüche 1 bis 3 für die Herstellung eines Medikaments für die Behandlung von Schmerzen.

7. Verwendung einer Verbindung der Ansprüche 1 bis 3 für die Herstellung eines Medikaments für die Behandlung von Intestinalerkrankungen, insbesondere chronische Entzündungen des Dünn- und Dickdarms (Reizdarmsyndrom - Colon irritabile, Colitis ulcerosa, Morbus Crohn) sowie Diarrhoe oder Obstipation.

8. Verwendung einer Verbindung der Ansprüche 1 bis 3 für die Herstellung eines Medikaments für die Behandlung von rheumatischen Erkrankungen, einschließlich rheumatoider Arthritis, Osteoarthritis, Arthrosen, Spondylosen, Lumbago, Lupus erythematodes und Spondylarthropathien.

9. Verwendung einer Verbindung der Ansprüche 1 bis 3 für die Herstellung eines Medikaments für die Behandlung von Tumoren und Krebs sowie für die Behandlung von Fettsucht und Übergewicht, als auch zur Unterdrückung der Abstoßung von Transplantaten nach Transplantationen und zur Prävention und Behandlung von Darmverschluss (Ileus).

10. Verwendung einer Verbindung der Ansprüche 1 bis 3 für die Herstellung eines Medikaments für die Suchtentwöhnung von beispielsweise Opiaten, Kokain oder Alkohol und zur Behandlung von psychischen Erkrankungen.

## Claims

1. Compounds of formula (I), 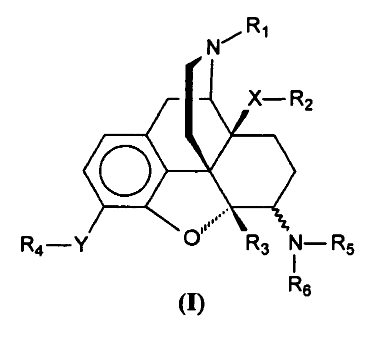 in which the substituents have the following meaning:
R₁ is hydrogen; C₁-C₆-alkyl; C₂-C₆-alkenyl; C₂-C₆-alkinyl; C₁-C₆-monohydroxyalkyl; C₂-C₆-dihydroxyalkyl; C₃-C₆-trihydroxyalkyl; C₄-C₁₆-cycloalkylalkyl, where cycloalkyl is C₃-C₁₀-cycloalkyl and alkyl is C₁-C₆-alkyl; C₅-C₁₆-cycloalkylalkenyl, where cycloalkyl is C₃-C₁₀-cycloalkyl and alkenyl is C₂-C₆-alkenyl; C₅-C₁₆-cycloalkylalkinyl, where cycloalkyl is C₃-C₁₀-cycloalkyl and alkinyl is C₂-C₆-alkinyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl;
the nitrogen joined with R₁ can also be quartemised by two substituents R₁, which can be the same or different and which are defined as previously shown, and wherein the second, quartemised substituent can additionally have the meaning hydroxyl, oxyl (N oxide) as well as alkoxyl;
R₂ is C₁-C₆-alkyl; C₁-C₆-monohydroxyalkyl; C₂-C₆-dihydroxyalkyl; C₃-C₆-trihydroxyalkyl; C₂-C₆-alkenyl; C₂-C₆-alkinyl; C₄-C₁₆-cycloalkylalkyl, where cycloalkyl is C₃-C₁₀-cycloalkyl and alkyl is C₁-C₆-alkyl; C₅-C₁₆-cycloalkylalkenyl, where cycloalkyl is C₃-C₁₀-cycloalkyl and alkenyl is C₂-C₆-alkenyl; C₅-C₁₆-cycloalkylalkinyl, where cycloalkyl is C₃-C₁₀-cycloalkyl and alkinyl is C₂-C₆-alkinyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl; C₂-C₆-alkanoyl; C₃-C₆-alkenoyl; C₃-C₆-alkinoyl; C₇-C₁₆-arylalkanoyl, where aryl is C₆-C₁₀-aryl and alkanoyl is C₁-C₆-alkanoyl; C₉-C₁₆-arylalkenoyl, where aryl is C₆-C₁₀-aryl and alkenoyl is C₃-C₆-alkenoyl; C₉-C₁₆-arylalkinoyl, where aryl is C₆-C₁₀-aryl and alkinoyl is C₃-C₆-alkinoyl;
R₃ is hydrogen; C₁-C₆-alkyl; C₂-C₆-alkenyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; alkoxyalkyl, where alkoxy is C₁-C₆-alkoxy and alkyl is C₁-C₆-alkyl; CO₂(C₁-C₆-alkyl); CO₂H; CH₂OH;
R₄, subject to the definition of Y, is hydrogen; C₁-C₆-alkyl; C₂-C₆-alkenyl; C₂-C₆-alkinyl; C₄-C₁₆-cycloalkylalkyl, where cycloalkyl is C₃-C₁₀-cycloalkyl and alkyl is C₁-C₆-alkyl; C₅-C₁₆-cycloalkylalkenyl, where cycloalkyl is C₃-C₁₀-cycloalkyl and alkenyl is C₂-C₆-alkenyl; C₅-C₁₆-cycloalkylalkinyl, where cycloalkyl is C₃-C₁₀-cycloalkyl and alkinyl is C₂-C₆-alkinyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl; C₂-C₆-alkanoyl; C₃-C₆-alkenoyl; C₃-C₆-alkinoyl; C₇-C₁₆-arylalkanoyl, where aryl is C₆-C₁₀-aryl and alkanoyl is C₁-C₆-alkanoyl; C₉-C₁₆-arylalkenoyl, where aryl is C₆-C₁₀-aryl and alkenoyl is C₃-C₆-alkenoyl; C₉-C₁₆-arylalkinoyl, where aryl is C₆-C₁₀-aryl and alkinoyl is C₃-C₆-alkinoyl; iminomethyl, formamidinyl, C₁-C₆-N-alkyl- and N,N'-dialkylformamidinyl; C₂-C₆-N-alkenyl- and N,N'-dialkenylformamidinyl; C₂-C₆-N-alkinyl- and N,N'-dialkinylformamidinyl; C₄-C₁₆-N-cycloalkylalkyl- and N,N'-dicycloalkylalkylformamidinyl, where cycloalkyl is C₃-C₁₀-cycloalkyl and alkyl is C₁-C₆-alkyl; C₅-C₁₆-N-cylcoalkylalkenyl- and N,N'-dicycloalkylalkenylformamidinyl, where cycloalkyl is C₃-C₁₀-cycloalkyl and alkenyl is C₂-C₆-alkenyl; C₅-C₁₆-N-cycloalkylalkinyl- and N,N'-dicycloalkylalkinylformamidinyl, where cycloalkyl is C₃-C₁₀-cycloalkyl and alkinyl is C₂-C₆-alkinyl; C₇-C₁₆-N-arylalkyl- and N,N'-diarylalkylformamidinyl, where aryl is C₆-C₁₀-aryl and alkyl is C₁-C₆-alkyl;
R₅ and R₆, which can be the same or different, are hydrogen; furthermore, CH(A)CO₂B, where A is hydrogen; hydroxyl; C₁-C₆-alkyl; C₂-C₆-alkenyl; C₂-C₆-alkinyl; C₄-C₁₆-cycloalkylalkyl, where cycloalkyl is C₃-C₁₀-cycloalkyl and alkyl is C₁-C₆-alkyl; C₅-C₁₆-cycloalkylalkenyl, where cycloalkyl is C₃-C₁₀-cycloalkyl and alkenyl is C₂-C₆-alkenyl; C₅-C₁₆-cycloalkylalkinyl, where cycloalkyl is C₃-C₁₀-cycloalkyl and alkinyl is C₂-C₆-alkinyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl; amino; C₁-C₆-alkylamino; guanidino; C₁-C₆-alkyl-CO₂B; and where B is hydrogen; C₁-C₃₀-, preferably C₁-C₆-alkyl; C₂-C₃₀-, preferably C₂-C₆-alkenyl; C₂-C₃₀-, preferably C₂-C₆-alkinyl; C₄-C₁₆-cycloalkylalkyl, where cycloalkyl is C₃-C₁₀-cycloalkyl and alkyl is C₁-C₆-alkyl; C₅-C₁₆-cycloalkylalkenyl, where cycloalkyl is C₃-C₁₀-cycloalkyl and alkenyl is C₂-C₆-alkenyl; C₅-C₁₆-cycloalkylalkinyl, where cycloalkyl is C₃-C₁₀-cycloalkyl and alkinyl is C₂-C₆-alkinyl; C₇-C₁₆-arylalkyl, where aryl is C₆-C₁₀-aryl and alkyl is C₁-C₆-alkyl; C₈-C₁₆-arylalkenyl, where aryl is C₆-C₁₀-aryl and alkenyl is C₂-C₆-alkenyl; C₈-C₁₆-arylalkinyl, where aryl is C₆-C₁₀-aryl and alkinyl is C₂-C₆-alkinyl; phenyl; substituted phenyl; CH₂OCO-C₁-C₆-alkyl; CH(C₁-C₆-alkyl)OCO-C₁-C₆-alkyl; CH₂OCOO-C₁-C₆-alkyl; CH(C₁-C₆-alkyl)OCOO-C₁-C₆-alkyl; CH₂CON(C₁-C₆-alkyl)₂; CH(C₁-C₆-alkyl)CON(C₁-C₆-alkyl)₂; phthalidyl, (5-methyl-2-oxo-1,3-dioxol-4-yl)methyl; and also iminomethyl, formamidinyl, C₁-C₆-N-alkyl- and N,N'-dialkylformamidinyl; C₂-C₆-N-alkenyl- and N,N'-dialkenylformamidinyl; C₂-C₆-N-alkinyl- and N,N'-dialkinylformamidinyl; C₄-C₁₆-N-cycloalkylalkyl- and N,N'-dicycloalkylalkylformamidinyl, where cycloalkyl is C₃-C₁₀-cycloalkyl and alkyl is C₁-C₆-alkyl; C₅-C₁₆-N-cylcoalkylalkenyl- and N,N'-dicycloalkylalkenylformamidinyl, where cycloalkyl is C₃-C₁₀-cycloalkyl and alkenyl is C₂-C₆-alkenyl; C₅-C₁₆-N-cycloalkylalkinyl- and N,N'-dicycloalkylalkinylformamidinyl, where cycloalkyl is C₃-C₁₀-cycloalkyl and alkinyl is C₂-C₆-alkinyl; C₇-C₁₆-N-arylalkyl- and N,N'-diarylalkylformamidinyl, where aryl is C₆-C₁₀-aryl and alkyl is C₁-C₆-alkyl; C₈-C₁₆-N-arylalkenyl- and N,N'-diarylalkenylformamidinyl, where aryl is C₆-C₁₀-aryl and alkenyl is C₂-C₆-alkenyl; C₈-C₁₆-N-arylalkinyl- and N,N'-diarylalkinylformamidinyl, where aryl is C₆-C₁₀-aryl and alkinyl is C₂-C₆-alkinyl; C₂-C₇-N-alkyloxycarbonyl- and N,N'-bis(alkyloxycarbonyl)formamidinyl; C₃-C₈-N-alkenyloxycarbonyl- and N,N'-bis(alkenyloxycarbonyl)formamidinyl; C₃-C₈-N-alkinyloxycarbonyl- and N,N'-bis(alkinyloxycarbonyl)formamidinyl; C₈-C₁₇-N-arylalkyloxycarbonyl- and N,N'-bis(arylalkyloxycarbonyl)formamidinyl, where aryl is C₆-C₁₀-aryl and alkyloxy is C₁-C₆-alkyloxy; C₉-C₁₇-N-arylalkenyloxycarbonyl- and N,N'-bis(arylalkenyloxycarbonyl)formamidinyl, where aryl is C₆-C₁₀-aryl and alkenyloxy is C₂-C₆-alkenyloxy; C₉-C₁₇-N-arylalkinyloxycarbonyl- and N,N'-bis(arylalkinyloxycarbonyl)formamidinyl, where aryl is C₆-C₁₀-aryl and alkinyloxy is C₂-C₆-alkinyloxy; C₁-C₆-N-alkanoyl- and N,N'-dialkanoylformamidinyl; C₃-C₆-N-alkenoyl- and N,N'-dialkenoylformamidinyl; C₃-C₆-N-alkinoyl- and N,N'-dialkinoylformamidinyl; C₇-C₁₆-N-arylalkanoyl- and N,N'-diarylalkanoylformamidinyl, where aryl is C₆-C₁₀-aryl and alkanoyl is C₁-C₆-alkyl; C₉-C₁₆-N-arylalkenoyl- and N,N'-diarylalkenoylformamidinyl, where aryl is C₆-C₁₀-aryl and alkenoyl is C₃-C₆-alkenoyl; C₉-C₁₆-N-arylalkinoyl- and N,N'-diarylalkinoylformamidinyl, where aryl is C₆-C₁₀-aryl and alkinoyl is C₃-C₆-alkinoyl; 4,5-dihydro-1*H*-imidazol-2-yl, 1,4,5,6-tetrahydropyrimidin-2-yl, 4, 5, 6, 7-tetrahydro- 1H-[1,3]diazepin-2-yl;
X is oxygen, and
Y is oxygen or the group (Y-R₄) is H;
and pharmaceutically acceptable acid addition salts and easily accessible derivatives (e.g. esters or amides of amino acid derivatives).

2. Compounds of Claim 1 in which R₁ is C₁-C₆-alkyl; R₂ is C₁-C₆-alkyl or C₇-C₁₆-arylalkyl, where aryl is C₆-C₁₀-aryl and alkyl is C₁-C₆-alkyl; R₃, R₄ and R₆ are hydrogen; R₅ is CH(A)CO₂B where A is hydrogen; hydroxyl; C₁-C₆-alkyl; C₇-C₁₆-arylalkyl, where aryl is C₆-C₁₀-aryl and alkyl is C₁-C₆-alkyl; amino; or guanidino; B is hydrogen or C₁-C₆-alkyl; R₅ can furthermore be formamidinyl; C₂-C₇-(alkyloxycarbonyl)formamidinyl; C₃-C₈-(alkenyloxycarbonyl)formamidinyl; C₃-C₈-(alkinyloxycarbonyl)formamidinyl; C₈-C₁₇-(arylalkyloxycarbonyl)formamidinyl, where aryl is C₆-C₁₀-aryl and alkyloxy is C₁-C₆-alkyloxy; C₉-C₁₇-(arylalkenyloxycarbonyl)formamidinyl, where aryl is C₆-C₁₀-aryl and alkenyloxy is C₂-C₆-alkenyloxy; C₉-C₁₇-(arylalkinyloxycarbonyl)formamidinyl, where aryl is C₆-C₁₀-aryl and alkinyloxy is C₂-C₆-alkinyloxy.

3. Compounds of Claim 1, selected from:
(4,5α-epoxy-3-hydroxy-14β-methoxy-17-methylmorphinan-6α-ylamino)-acetic acid-tert.-butylester, (4,5α-epoxy-3-hydroxy-14β-methoxy-17-methylmorphinan-6β-ylamino)-acetic acid-tert.-butylester, (4,5α-epoxy-3-hydroxy-14β-methoxy-17-methylmorphinan-6α-ylamino)-acetic acid, (4,5α-epoxy-3-hydroxy-14β-methoxy-17-methy)morphinan-6β-ylamino)-acetic acid, (2'S)-2'-(4,5α-epoxy-3-hydroxy-14β-methoxy-17-methylmorphinan-6α-ylamino)-propionic acid-tert.-butylester, (2'S)-2'-(4,5α-epoxy-3-hydroxy-14β-methoxy-17-methylmorphinan-6β-ylamino)-propionic acid-tert.-butylester, (2'S)-2'-(4,5α-epoxy-3-hydroxy-14β-methoxy-17-methylmorphinan-6α-ylamino)-propionic acid, (2'S)-2'-(4,5α-epoxy-3-hydroxy-14β-methoxy-17-methylmorphinan-6β-ylamino)-propionic acid, (2'S)-2'-(4,5α-epoxy-3-hydroxy-14β-methoxy-17-methylmorphinan-6α-ylamino)-3'-phenylpropionic acid-tert.-butylester, (2'S)-2'-(4,5α-epoxy-3-hydroxy-14β-methoxy-17-methylmorphinan-6β-ylamino)-3'-phenylpropionic acid-tert.-butylester, (2'S)-2'-(4,5α-epoxy-3-hydroxy-14β-methoxy-17-methylmorphinan-6α-ylamino)-3'-phenylpropionic acid, (2'S)-2'-(4,5α-epoxy-3-hydroxy-14β-methoxy-17-methylmorphinan-6β-ylamino)-3'-phenylpropionic acid, 6α-amino-4,5α-epoxy-14β-methoxy-17-methylmorphinan-3-ol, 6β-dibenzylamino-4,5α-epoxy-14β-methoxy-17-methylmorphinan-3-ol, 6β-amino-4,5α-epoxy-14β-methoxy-17-methylmorphinan-3-ol, 4,5α-epoxy-6β-[N,N'-bis-(tert.-butoxycarbonyl)guanidinyl]-14β-methoxy-17-methylmorphinan-3-ol, 4,5α-epoxy-6β-guanidinyl-14β-methoxy-17-methylmorphinan-3-ol, 4,5α-epoxy-6α-[N,N'-bis-(tert.-butoxycarbonyl)guanidinyl]-14β-methoxy-17-methylmorphinan-3-ol, 4,5α-epoxy-6α-guanidinyl-14β-methoxy-17-methylmorphinan-3-ol, 1,3-bis-(tert.-butoxycarbonyl)-2-{4,5α-epoxy-6β-[N,N'-bis-(tert.-butoxycarbonyl)guanidinyl]-14β-methoxy-17-methylmorphinan-3-yl}-isourea, 1,3-bis-(tert.-butoxycarbonyl)-2-{4,5α-epoxy-6α-[N,N'-bis-(tert.-butoxycarbonyl)guanidinyl]-14β-methoxy-17-methylmorphinan-3-yl}-isourea, (4,5α-epoxy-3-hydroxy-14β-methoxy-17-methylmorphinan-6α-ylamino)-acetic acid-ethylester dihydrochloride, (4,5α-epoxy-3-hydroxy-14β-methoxy-17-methylmorphinan-6β-ylamino)-acetic acid-ethylester dihydrochloride, (4,5α-epoxy-3-hydroxy-14β-ethoxy-17-methylmorphinan-6α-ylamino)-acetic acid-tert.-butylester, (4,5α-epoxy-3-hydroxy-14β-ethoxy-17-methylmorphinan-6β-ylamino)-acetic acid-tert.-butylester, (4,5α-epoxy-3-hydroxy-14β-ethoxy-17-methylmorphinan-6α-ylamino)-acetic acid bis(tetrafluoroborate), (4,5α-epoxy-3-hydroxy-14β-ethoxy-17-methylmorphinan-6α-ylamino)-acetic acid bis(tetrafluoroborate), (2'S)-2'-(17-cyclopropylmethyl-4,5α-epoxy-3,14β-dihydroxymorphinan-6β-ylamino)-3-phenylpropionic acid-tert.-butylester, (2'S)-2'-(17-cyclopropylmethyl-4,5α-epoxy-3,14β-dihydroxymorphinan-6β-ylamino)-3-phenylpropionic acid bis(tetrafluoroborate), {17-cyclopropylmethyl-4,5α-epoxy-3-hydroxy-14β-[(3-phenylpropyl)oxy]-morphinan-6α-ylamino}-acetic acid-tert.-butylester, {17-cyclopropylmethyl-4,5α-epoxy-3-hydroxy-14β-[(3-phenylpropyl)oxy]-morphinan-6α-ylamino}-acetic acid-tert.-butylester, (2'S)-2'-(17-cyclopropylmethyl-4,5α-epoxy-3-hydroxy-14β-[(3-phenylpropyl)oxy]-morphinan-6α-ylamino)-3-phenylpropionic acid-tert.-butylester, {17-cyclopropylmethyl-4,5α-epoxy-3-hydroxy-14β-[(3-phenylpropyl)oxy]-morphinan-6β-ylamino}-acetic acid dihydrochloride; or any pharmaceutically acceptable salt or an easily accessible derivative of them.

4. A composition, comprising a compound of Claims 1 to 3 and / or a pharmaceutically acceptable acid addition salt of it, together with a pharmaceutically acceptable carrier substance.

5. A compound according to any of Claims 1 to 3 as medicament.

6. Use of a compound of Claims 1 to 3 for the preparation of a medicament for the treatment of pain.

7. Use of a compound of Claims 1 to 3 for the preparation of a medicament for the treatment of intestinal diseases, in particular chronic inflammation of the small and large intestines (irritable colon syndrome - colon irritabile, colitis ulcerosa, morbus Crohn), diarrhoea or obstipation.

8. Use of a compound of Claims 1 to 3 for the preparation of a medicament for the treatment of rheumatic diseases, including rheumatoid arthritis, osteoarthritis, arthrosis, spondylosis, lumbago, lupus erythematosus, spondylarthropathy.

9. Use of a compound of Claims 1 to 3 for the preparation of a medicament for the treatment of tumours and cancer as well as for the treatment of obesity and overweight, and also for the suppression of rejection of transplants after transplantations and for the prevention and treatment of intestinal obstruction (ileus).

10. Use of a compound of Claims 1 to 3 for the preparation of a medicament for the withdrawal from drug addiction, for example, to opiates, cocaine or alcohol and for the treatment of psychic diseases.

## Revendications

1. Composé de formule (I), 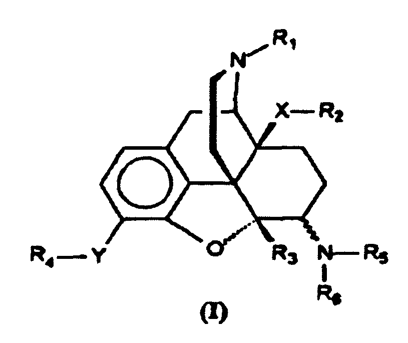 dans laquelle les substituants ont la signification suivante :
R₁ est un hydrogène; alkyle en C₁ à C₆ ; alcényle en C₂ à C₆ ; alcynyle en C₂ à C₆ ; monohydroxyalkyle en C₁ à C₆ ; dihydroxyalkyle en C₂ à C₆; trihydroxyalkyle en C₃ à C₆; cycloalkylalkyle en C₄ à C₁₆, dans lequel le cycloalkyle est un cycloalkyle en C₃ à C₁₀ et l'alkyle est un alkyle en C₁ à C₆; cycloalkylalcényle en C₅ à C₁₆, dans lequel le cycloalkyle est un cycloalkyle en C₃ à C₁₀ et l'alcényle est un alcényle en C₂ à C₆ ; cycloalkylalcynyle en C₅ à C₁₆, dans lequel le cycloalkyle est un cycloalkyle en C₃ à C₁₀ et l'alcynyle est un alcynyle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; arylalcynyle en C₈ à C₁₆, dans lequel l'aryle est un aryle en C₆ à C₁₀ et l'alcynyle est un alcynyle en C₂ à C₆ ;
qui peut être additionnellement aussi quaternisé avec un azote lié à R₁ au moyen de deux substituants R₁, qui peuvent être identiques ou différents et qui sont définis comme présenté précédemment et dans lesquels le deuxième substituant réalisant la quaternisation peut avoir de plus la signification d'un hydroxyle, oxyle (N-oxyde) ainsi qu'alcoxyle ;
R₂ est un alkyle en C₁ à C₆ ; monohydroxyalkyle en C₁ à C₆ ; dihydroxyalkyle en C₂ à C₆ ; trihydroxyalkyle en C₃ à C₆ ; alcényle en C₂ à C₆ ; alcynyle en C₂ à C₆ ; cycloalkylalkyle en C₄ à C₁₆, dans lequel le cycloalkyle est un cycloalkyle en C₃ à C₁₀ et l'alkyle est un alkyle en C₁ à C₆ ; cycloalkylalcényle en C₅ à C₁₆, dans lequel le cycloalkyle est un cycloalkyle en C₃ à C₁₀ et l'alcényle est un alcényle en C₂ à C₆ ; cycloalkylalcynyle en C₅ à C₁₆, dans lequel le cycloalkyle est un cycloalkyle en C₃ à C₁₀ et l'alcynyle est un alcynyle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; arylalcynyle en C₈ à C₁₆, dans lequel l'aryle est un aryle en C₆ à C₁₀ et l'alcynyle est un alcynyle en C₂ à C₆ ; alcanoyle en C₂ à C₆ ; alcénoyle en C₃ à C₆ ; alcynoyle en C₃ à C₆ ; arylalcanoyle en C₇ à C₁₆, dans lequel l'aryle est un aryle en C₆ à C₁₀ et l'alcanoyle est un alcanoyle en C₁ à C₆ ; arylalcénoyle en C₉ à C₁₆, dans lequel l'aryle est un aryle en C₆ à C₁₀ et l'alcénoyle est un alcénoyle en C₃ à C₆ ; arylalcynoyle en C₉ à C₁₆, dans lequel l'aryle est un aryle en C₆ à C₁₀ et l'alcynoyle est un alcynoyle en C₃ à C₆ ;
R₃ est un hydrogène ; alkyle en C₁ à C₆ ; alcényle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; alcoxyalkyle, dans lequel l'alcoxy est un alcoxy en C₁ à C₆ et l'alkyle est un alkyle en C₁ à C₆ ; CO₂ (alkyle en C₁ à C₆) ; CO₂H ; CH₂OH.
R₄, sous réserve de la définition de Y, est un hydrogène ; alkyle en C₁ à C₆ ; alcényle en C₂ à C₆ ; alcynyle en C₂ à C₆ ; cycloalkylalkyle en C₄ à C₁₆, dans lequel le cycloalkyle est un cycloalkyle en C₃ à C₁₀ et l'alkyle est un alkyle en C₁ à C₆ ; cycloalkylalcényle en C₅ à C₁₆, dans lequel le cycloalkyle est un cycloalkyle en C₃ à C₁₀ et l'alcényle est un alcényle en C₂ à C₆ ; cycloalkylalcynyle en C₅ à C₁₆, dans lequel le cycloalkyle est un cycloalkyle en C₃ à C₁₀ et l'alcynyle est un alcynyle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; arylalcynyle en C₈ à C₁₆, dans lequel l'aryle est un aryle en C₆ à C₁₀ et l'alcynyle est un alcynyle en C₂ à C₆ ; alcanoyle en C₂ à C₆ ; alcénoyle en C₃ à C₆ ; alcynoyle en C₃ à C₆ ; arylalcanoyle en C₇ à C₁₆, dans lequel l'aryle est un aryle en C₆ à C₁₀ et l'alcanoyle est un alcanoyle en C₁ à C₆ ; arylalcénoyle en C₉ à C₁₆, dans lequel l'aryle est un aryle en C₆ à C₁₀ et l'alcénoyle est un alcénoyle en C₃ à C₆ ; arylalcynoyle en C₉ à C₁₆, dans lequel l'aryle est un aryle en C₆ à C₁₀ et l'alcynoyle est un alcynoyle en C₃ à C₆ ; iminométhyle, formamidinyle, N-(alkyle en C₁ à C₆)- et N,N'-dialkylformamidinyle ; N-(alcényle en C₂ à C₆)- et N,N'-dialcénylformamidinyle ; N-(alcynyle en C₂ à C₆) - et N,N'-dialcynylformamidinyle ; N-(cycloalkylalkyle en C₄ à C₁₆) - et N,N'-dicycloalkylalkylformamidinyle, dans lesquels le cycloalkyle est un cycloalkyle en C₃ à C₁₀ et l'alkyle est un alkyle en C₁ à C₆ ; N-(cycloalkylalcényle en C₅ à C₁₆) - et N,N'-dicycloalkylalcénylformamidinyle, dans lesquels le cycloalkyle est un cycloalkyle en C₃ à C₁₀ et l'alcényle est un alcényle en C₂ à C₆ ; N-(cycloalkylalcynyle en C₅ à C₁₆) - et N,N'-dicycloalkylalcynylformamidinyle, dans lesquels le cycloalkyle est un cycloalkyle en C₃ à C₁₀ et l'alcynyle est un alcynyle en C₂ à C₆ ; N-(arylalkyle en C₇ à C₁₆) - et N,N'-diarylalkylformamidinyle, dans lesquels l'aryle est un aryle en C₆ à C₁₀ et l' alkyle est un alkyle en C₁ à C₆.
R₅ et R₆, qui peuvent être identiques ou différents, sont un hydrogène, de plus CH(A)CO₂B, dans lequel A représente un hydrogène ; hydroxyle ; alkyle en C₁ à C₆; alcényle en C₂ à C₆ ; alcynyle en C₂ à C₆ ; cycloalkylalkyle en C₄ à C₁₆, dans lequel le cycloalkyle est un cycloalkyle en C₃ à C₁₀ et l'alkyle est un alkyle en C₁ à C₆ ; cycloalkylalcényle en C₅ à C₁₆, dans lequel le cycloalkyle est un cycloalkyle en C₃ à C₁₀ et l'alcényle est un alcényle en C₂ à C₆ ; cycloalkylalcynyle en C₅ à C₁₆, dans lequel le cycloalkyle est un cycloalkyle en C₃ à C₁₀ et l'alcynyle est un alcynyle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; arylalcynyle en C₈ à C₁₆, dans lequel l'aryle est un aryle en C₆ à C₁₀ et l'alcynyle est un alcynyle en C₂ à C₆ ; amino ; alkylamino en C₁ à C₆ ; guanidino ; (alkyle en C₁ à C₆)-CO₂B ; et dans lequel B représente un hydrogène ; alkyle en C₁ à C₃₀, de préférence en C₁ à C₆ ; alcényle en C₂ à C₃₀, de préférence en C₂ à C₆ ; alcynyle en C₂ à C₃₀, de préférence en C₂ à C₆ ; cycloalkylalkyle en C₄ à C₁₆, dans lequel le cycloalkyle est un cycloalkyle en C₃ à C₁₀ et l'alkyle est un alkyle en C₁ à C₆ ; cycloalkylalcényle en C₅ à C₁₆, dans lequel le cycloalkyle est un cycloalkyle en C₃ à C₁₀ et l'alcényle est un alcényle en C₂ à C₆ ; cycloalkylalcynyle en C₅ à C₁₆, dans lequel le cycloalkyle est un cycloalkyle en C₃ à C₁₀ et l'alcynyle est un alcynyle en C₂ à C₆ ; arylalkyle en C₇ à C₁₆, dans lequel l'aryle est un aryle en C₆ à C₁₀ et l'alkyle est un alkyle en C₁ à C₆ ; arylalcényle en C₈ à C₁₆, dans lequel l'aryle est un aryle en C₆ à C₁₀ et l'alcényle est un alcényle en C₂ à C₆ ; arylalcynyle en C₈ à C₁₆, dans lequel l'aryle est un aryle en C₆ à C₁₀ et l'alcynyle est un alcynyle en C₂ à C₆ ; phényle ; phényle substitué ; CH₂OCO-alkyle en C₁ à C₆ ; CH(alkyle en C₁ à C₆) OCO-alkyle en C₁ à C₆ ; CH₂OCOO-(alkyle en C₁ à C₆) ; CH(alkyle en C₁ à C₆) OCOO-alkyle en C₁ à C₆ ; CH₂CON(alkyle en C₁ à C₆)₂ ; CH(alkyle en C₁ à C₆) CON (alkyle en C₁ à C₆)₂ ; phtalidyle, (5-méthyl-2-iodo-1,3-dioxol-4-yl)méthyle ; et de plus un iminométhyle, formamidinyle, N-(alkyle en C₁ à C₆)- et N,N'-dialkylformamidinyle ; N-(alcényle en C₂ à C₆)-et N,N'-dialcénylformamidinyle ; N-(alcynyle en C₂ à C₆)- et N,N'-dialcynylformamidinyle ; N-(cycloalkylalkyle en C₄ à C₁₆) - et N,N'-dicycloalkylalkylformamidinyle, dans lesquels le cycloalkyle est un cycloalkyle en C₃ à C₁₀ et l'alkyle est un alkyle en C₁ à C₆ ; N-(cycloalkylalcényle en C₅ à C₁₆) - et N,N'-dicycloalkyl-alcénylformamidinyle, dans lesquels le cycloalkyle est un cycloalkyle en C₃ à C₁₀ et l'alcényle est un alcényle en C₂ à C₆ ; N-(cycloalkylalcynyle en C₅ à C₁₆)- et N,N'-dicycloalkylalcynylformamidinyle, dans lesquels le cycloalkyle est un cycloalkyle en C₃ à C₁₀ et l'alcynyle est un alcynyle en C₂ à C₆ ; N-(arylalkyle en C₇ à C₁₆)- et N,N'-diarylalkylformamidinyle, dans lesquels l'aryle est un aryle en C₆ à C₁₀ et l'alkyle est un alkyle en C₁ à C₆ ; N-(arylalcényle en C₈ à C₁₆) - et N,N'-diarylalcényl-formamidinyle, dans lesquels l'aryle est un aryle en C₆ à C₁₀ et l'alcényle est un alcényle en C₂ à C₆ ; N-(arylalcynyle en C₈ à C₁₆)- et N,N'-diarylalcynyl-formamidinyle, dans lesquels l'aryle est un aryle en C₆ à C₁₀ et l'alcynyle est un alcynyle en C₂ à C₆ ; N-(alkyloxycarbonyle en C₂ à C₇) - et N,N'-bis(alkyloxy-carbonyl)formamidinyle ; N-(alcényloxy-carbonyle en C₃ à C₈)- et N,N'-bis(alcényloxycarbonyl)-formamidinyle ; N-(alcynyloxycarbonyle en C₃ à C₈) - et N,N'-bis-(alcynyloxycarbonyl)formamidinyle ; N-(arylalkyloxy-carbonyle en C₈ à C₁₇)- et N,N'-bis(arylalkyloxy-carbonyl)formamidinyle, dans lesquels l'aryle est un aryle en C₆ à C₁₀ et l'alkyloxy un alkyloxy en C₁ à C₆ ; N-(arylalcényloxycarbonyle en C₉ à C₁₇)- en N,N'-bis(arylalcényloxycarbonyl)-formamidinyle, dans lesquels l'aryle est un aryle en C₆ à C₁₀ et l'alcényloxy un alcényloxy en C₂ à C₆ ; N-(arylalcynyloxycarbonyle en C₉ à C₁₇)- et N,N'-bis(arylalcynyloxycarbonyl)formamidinyle, dans lesquels l'aryle est un aryle en C₆ à C₁₀ et l'alcynyloxy un alcynyloxy en C₂ à C₆ ; N-(alcanoyle en C₁ à C₆) - et N,N'-dialcanoylformamidinyle ; N-(alcénoyle en C₃ à C₆)- et N,N'-dialcénoyl-formamidinyle ; N-(alcynoyle en C₃ à C₆) - et N,N'-dialcynoylformamidinyle ; N-(arylalcanoyle en C₇ à C₁₆)- et N,N'-diarylalcanoylformamidinyle, dans lesquels l'aryle est un aryle en C₆ à C₁₀ et l'alcanoyle un alkyle en C₁ à C₆ ; N-(arylalcénoyle en C₉ à C₁₆) - et N,N'-diarylalcénoylformamidinyle, dans lesquels l'aryle est un aryle en C₆ à C₁₀ et l'alcénoyle un alcénoyle en C₃ à C₆ ; N-(arylalcynoyle en C₉ à C₁₆)- et N,N'-diarylalcynoylformamidinyle, dans lesquels l'aryle est un aryle en C₆ à C₁₀ et l'alcynoyle un alcynoyle en C₃ à C₆ ; 4,5-dihydro-1*H*-imidazol-2-yle, 1,4,5,6-tétrahydropyrimidin-2-yle, 4,5,6,7-tétrahydro-1H-[1,3]diazépin-2-yle ;
X est un oxygène, et
Y est un oxygène ou le groupement (Y-R₄) est H ;
ainsi que les sels d'addition avec un acide pharmaceutiquement acceptables et les dérivés facilement accessibles (par exemple les esters ou amides des dérivés d'amino-acides).

2. Composé selon la revendication 1, dans lequel R₁ est un alkyle en C₁ à C₆ ; R₂ est un alkyle en C₁ à C₆ ou arylalkyle en C₇ à C₁₆, dans lequel l'aryle est un aryle en C₆ à C₁₀ et l'alkyle est un alkyle en C₁ à C₆ ; R₃, R₄ et R₆ sont un hydrogène ; R₅ est CH(A)CO₂B, dans lequel A est un hydrogène ; hydroxyle ; alkyle en C₁ à C₆ ; arylalkyle en C₇ à C₁₆, dans lequel l'aryle est un aryle en C₆ à C₁₀ et l'alkyle est un alkyle en C₁ à C₆ ; amino ; ou guanidino ; B est un hydrogène ou alkyle en C₁ à C₆ ; R₅ peut en outre être un formamidinyle ; (alkyloxycarbonyle en C₂ à C₇)formamidinyle ; (alcényloxycarbonyle en C₃ à C₈)formamidinyle ; (alcynyloxycarbonyle en C₃ à C₈)formamidinyle ; (arylalkyloxycarbonyle en C₈ à C₁₇) formamidinyle, dans lequel l'aryle est un aryle en C₆ à C₁₀ et l'alkyloxy est un alkyloxy en C₁ à C₆ ; (arylalcényloxycarbonyle en C₉ à C₁₇)formamidinyle, dans lequel l'aryle est un aryle en C₆ à C₁₀ et l'alcényloxy est un alcényloxy en C₂ à C₆ ; (arylalcynyloxycarbonyle en C₉ à C₁₇) formamidinyle, dans lequel l'aryle est un aryle en C₆ à C₁₀ et l'alcynyloxy est un alcynyloxy en C₂ à C₆.

3. Composé selon la revendication 1, choisi parmi : ester tert.-butylique d'acide (4,5α-époxy-3-hydroxy-14β-méthoxy-17-méthylmorphinan-6α-ylamino)-acétique, ester tert.-butylique d'acide (4,5α-époxy-3-hydroxy-14β-méthoxy-17-méthylmorphinan-6β-ylamino)-acétique, acide (4,5α-époxy-3-hydroxy-14β-méthoxy-17-méthylmorphinan-6α-ylamino)-acétique, acide (4,5α-époxy-3-hydroxy-14β-méthoxy-17-méthylmorphinan-6β-ylamino)-acétique, ester tert.-butylique d'acide (2'S)-2'-(4,5α-époxy-3-hydroxy-14β-méthoxy-17-méthyl-morphinan-6α-ylamino)-propionique, ester tert.-butylique d'acide (2'S)-2'-(4,5α-époxy-3-hydroxy-14β-méthoxy-17-méthylmorphinan-6β-ylamino)-propionique, acide (2'S)-2'-(4,5α-époxy-3-hydroxy-14β-méthoxy-17-méthylmorphinan-6α-ylamino)-propionique, acide (2'S)-2'-(4,5α-époxy-3-hydroxy-14β-méthoxy-17-méthylmorphinan-6β-ylamino)-propionique, ester tert.-butylique d'acide (2'S)-2'-(4,5α-époxy-3-hydroxy-14β-méthoxy-17-méthyl-morphinan-6α-ylamino)-3'-phénylpropionique, ester tert.-butylique d'acide (2'S)-2'-(4,5α-époxy-3-hydroxy-14β-méthoxy-17-méthylmorphinan-6β-ylamino)-3'-phénylpropionique, acide (2'S)-2'-(4,5α-époxy-3-hydroxy-14β-méthoxy-17-méthylmorphinan-6α-ylamino)-3'-phénylpropionique, acide (2'S)-2'-(4,5α-époxy-3-hydroxy-14β-méthoxy-17-méthylmorphinan-6β-ylamino)-3'-phénylpropionique, 6α-amino-4,5α-époxy-14β-méthoxy-17-méthylmorphinan-3-ol, 6β-dibenzylamino-4,5α-époxy-14β-méthoxy-17-méthylmorphinan-3-ol, 6β-amino-4,5α-époxy-14β-méthoxy-17-méthylmorphinan-3-ol, 4,5α-époxy-6β-[N,N'-bis(tert.-butoxycarbonyl)guanidinyl]-14β-méthoxy-17-méthylmorphinan-3-ol, 4,5α-époxy-6β-guanidinyl-14β-méthoxy-17-méthylmorphinan-3-ol, 4,5α-époxy-6α-[N,N'-bis(tert.-butoxycarbonyl)guanidinyl]-14β-méthoxy-17-méthylmorphinan-3-ol, 4,5α-époxy-6α-guanidinyl-14β-méthoxy-17-méthylmorphinan-3-ol, 1,3-bis-(tert.-butoxycarbonyl)-2-{4,5α-époxy-6β-[N,N'-bis-(tert.-butoxycarbonyl)guanidinyl]-14β-méthoxy-17-méthylmorphinan-3-yl}-isourée, 1,3-bis-(tert.-butoxy-carbonyl)-2-{4,5α-époxy-6α-[N,N'-bis-(tert.-butoxy-carbonyl)guanidinyl]-14β-méthoxy-17-méthylmorphinan-3-yl]-isourée, ester éthylique d'acide (4,5α-époxy-3-hydroxy-14β-méthoxy-17-méthylmorphinan-6α-ylamino)-acétique dichlorhydrate, ester éthylique d'acide (4,5α-époxy-3-hydroxy-14β-méthoxy-17-méthylmorphinan-6β-ylamino)-acétique dichlorhydrate, ester tert.-butylique d'acide (4,5α-époxy-3-hydroxy-14β-éthoxy-17-méthylmorphinan-6α-ylamino)-acétique, ester tert.-butylique d'acide (4,5α-époxy-3-hydroxy-14β-éthoxy-17-méthylmorphinan-6β-ylamino)-acétique, acide (4,5α-époxy-3-hydroxy-14β-éthoxy-17-méthylmorphinan-6α-ylamino)-acétique bis(tétrafluoroborate), acide (4,5α-époxy-3-hydroxy-14β-éthoxy-17-méthylmorphinan-6β-ylamino)-acétique bis(tétrafluoroborate), ester tert.-butylique d'acide (2'S)-2'-(17-cyclopropylméthyl-4,5α-époxy-3,14β-dihydroxymorphinan-6β-ylamino)-3-phénylpropionique, acide (2'S)-2'-(17-cyclopropyl-méthyl-4,5α-époxy-3,14β-dihydroxymorphinan-6β-ylamino)-3-phénylpropionique bis(tétrafluoroborate), ester tert.-butylique d'acide {17-cyclopropylméthyl-4,5α-époxy-3-hydroxy-14β-[(3-phénylpropyl)oxy]-morphinan-6α-ylamino}-acétique, ester tert.-butylique d'acide {17-cyclopropylméthyl-4,5α-époxy-3-hydroxy-14β-[(3-phénylpropyl)oxy]-morphinan-6α-ylamino}-acétique, ester tert.-butylique d'acide (2'S)-2'-(17-cyclopropylméthyl-4,5α-époxy-3-hydroxy-14β-[(3-phényl-propyl)oxy]-morphinan-6α-ylamino)-3-phénylpropionique, acide {17-cyclopropylméthyl-4,5α-époxy-3-hydroxy-14β-[(3-phénylpropyl)oxy]-morphinan-6β-ylamino}-acétique dichlorhydrate, ou un sel quelconque pharmaceutiquement acceptable ou un dérivé facilement accessible de ceux-ci.

4. Composition, comprenant un composé selon les revendications 1 à 3 et/ou un sel d'addition avec un acide pharmaceutiquement acceptable de celui-ci, en même temps qu'un support pharmaceutiquement acceptable.

5. Composé selon l'une quelconque des revendications 1 à 3 comme médicament.

6. Utilisation d'un composé selon les revendications 1 à 3 pour la préparation d'un médicament pour le traitement de douleurs.

7. Utilisation d'un composé selon les revendications 1 à 3 pour la préparation d'un médicament pour le traitement de maladies intestinales, en particulier d'inflammations chroniques de l'intestin grêle ou du gros intestin (syndrome de l'intestin irritable - côlon irritable, colite ulcéreuse, Morbus Crohn) ainsi que la diarrhée ou la constipation.

8. Utilisation d'un composé selon les revendications 1 à 3 pour la préparation d'un médicament pour le traitement de maladies rhumatoïdes, y compris l'arthrite rhumatoïde, l'ostéoarthrite, les arthroses, les spondyloses, le lumbago, le lupus érythémateux et les spondylarthropathies.

9. Utilisation d'un composé selon les revendications 1 à 3 pour la préparation d'un médicament pour le traitement de tumeurs et de cancers ainsi que pour le traitement de l'adipose et du surpoids, ainsi que pour diminuer le rejet des transplants après transplantation et pour la prévention et le traitement de l'occlusion intestinale (Ileus).

10. Utilisation d'un composé selon les revendications 1 à 3 pour la préparation d'un médicament pour la désintoxication par exemple des opiacées, de la cocaïne ou de l'alcool et pour le traitement de maladies psychiques.
